(19) Europäisches Patentamt
European Patent Office
Office européen des brevets

(11) EP 3 674 299 B1

(12) **EUROPEAN PATENT SPECIFICATION**

(45) Date of publication and mention of the grant of the patent:
**24.11.2021 Bulletin 2021/47**

(51) Int Cl.:
***C07D 403/14*** *(2006.01)* ***H01L 51/50*** *(2006.01)*
***H01L 51/00*** *(2006.01)*

(21) Application number: **18001019.1**

(22) Date of filing: **28.12.2018**

(54) **ORGANIC MOLECULES FOR OPTOELECTRONIC DEVICES**

ORGANISCHE MOLEKÜLE FÜR OPTOELEKTRONISCHE VORRICHTUNGEN

MOLÉCULES ORGANIQUES POUR DISPOSITIFS OPTOÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.07.2020 Bulletin 2020/27**

(73) Proprietor: **cynora GmbH**
**76646 Bruchsal (DE)**

(72) Inventors:
- **RACZYNSKA, Dagmara**
**69214 Eppelheim (DE)**
- **SZAFRANOWSKA, Barbara**
**76646 Bruchsal (DE)**

(74) Representative: **Hoppe, Georg Johannes**
**Darani Anwaltskanzlei**
**Beuckestrasse 20**
**14163 Berlin (DE)**

(56) References cited:
**EP-A1- 2 272 828** **US-A1- 2007 196 692**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 674 299 B1

Printed by Jouve, 75001 PARIS (FR)

## Description

The invention relates to light-emitting organic molecules and their use in organic light-emitting diodes (OLEDs) and in other optoelectronic devices. EP 2272828 A1 describes a compound for an organic electroluminescent device and US 2007/0196692 A1 an organic electroluminescent device.

## Description

The object of the present invention is to provide molecules which are suitable for use in organic optoelectronic devices.

This object is achieved by the invention which provides a new class of organic molecules.

The organic molecules of the invention are purely organic molecules, i.e. they do not contain any metal ions in contrast to metal complexes known for use in organic optoelectronic devices.

According to the present invention, the organic molecules exhibit emission maxima in the blue, sky-blue or green spectral range. The organic molecules exhibit in particular emission maxima between 420 nm and 520 nm, preferably between 440 nm and 495 nm, more preferably between 450 nm and 470 nm. The photoluminescence quantum yields of the organic molecules according to the invention are, in particular, 50 % or more. Furthermore, the organic molecules according to the invention exhibit in particular a small full width at half maximum (FWHM), in particular below 0.4 eV. The molecules according to the invention exhibit in particular thermally activated delayed fluorescence (TADF). The use of the molecules according to the invention in an optoelectronic device, for example an organic light-emitting diode (OLED), leads to higher efficiencies of the device. Corresponding OLEDs have a higher stability than OLEDs with known emitter materials and comparable color.

The organic light-emitting molecules of the invention comprise or consist of one first chemical moiety comprising or consisting of a structure of formula I

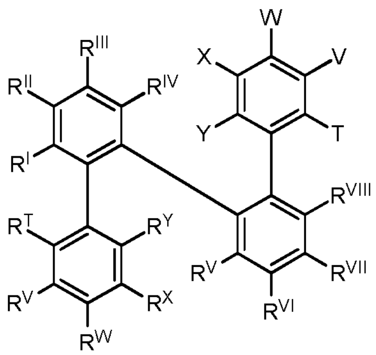

Formula I

and

- two second chemical moieties, each independently from another comprising or consisting of a structure of formula II,

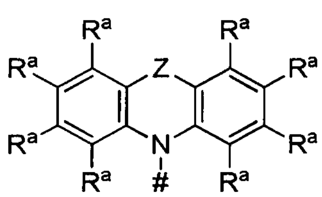

Formula II

wherein the first chemical moiety is linked to each of the two second chemical moieties via a single bond.

$R^T$, $R^V$, $R^W$, $R^X$, and $R^Y$ is the binding site of a single bond linking the first chemical moiety to one of the two second chemical moieties or is selected from the group consisting of $R^1$ and $R^A$;

$R^A$ comprises or consists of a structure of formula A:

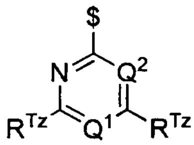

Formula A

wherein $Q^1$ and $Q^2$ are selected from the group consisting of N and C-$R^{Py}$; and $ represents the binding site of a single bond linking $R^A$ to the first chemical moiety;

T, V, W, X, and Y is the binding site of a single bond linking the first chemical moiety to one of the two second chemical moieties or is selected from the group consisting of $R^2$ and $R^B$;

$R^B$ comprises or consists of a structure of formula B:

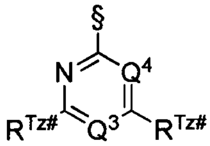

Formula B

wherein

$Q^3$ and $Q^4$ are selected from the group consisting of N and C-$R^{Py\#}$; and
§ represents the binding site of a single bond linking $R^B$ to the first chemical moiety;
# represents the binding site of a single bond linking each of the second chemical moiety to the first chemical moiety;
Z is at each occurrence independently from another selected from the group consisting of a direct bond, $CR^3R^4$, $C=CR^3R^4$, C=O, $C=NR^3$, $NR^3$, O, $SiR^3R^4$, S, S(O) and $S(O)_2$;
$R^1$, $R^2$ is at each occurrence independently from another selected from the group consisting of:

hydrogen, deuterium,
F, Cl, Br, I,
CN, and
$CF_3$,
$C_1$-$C_5$-alkyl, wherein one or more hydrogen atoms are optionally substituted by deuterium;
$C_2$-$C_8$-alkenyl, wherein one or more hydrogen atoms are optionally substituted by deuterium;
$C_2$-$C_8$-alkynyl, wherein one or more hydrogen atoms are optionally substituted by deuterium; and
$C_6$-$C_{18}$-aryl, which is optionally substituted with one or more substituents $R^6$;

$R^I$, $R^{II}$, $R^{III}$, $R^{IV}$, $R^V$, $R^{VI}$, $R^{VII}$, and $R^{VIII}$ is independently from each other at each occurrence independently from another selected from the group consisting of:

hydrogen, deuterium,
F, Cl, Br, I,
CN, and
$CF_3$
$C_1$-$C_5$-alkyl,

wherein one or more hydrogen atoms are optionally substituted by deuterium; $C_2$-$C_8$-alkenyl,

wherein one or more hydrogen atoms are optionally substituted by deuterium; $C_2$-$C_8$-alkynyl, wherein one or more hydrogen atoms are optionally substituted by deuterium; and $C_6$-$C_{18}$-aryl, which is optionally substituted with one or more substituents $R^6$;

$R^{Tz}$ is at each occurrence independently from another selected from the group consisting of: hydrogen, deuterium,
$C_1$-$C_{10}$-alkyl,

wherein one or more hydrogen atoms are optionally substituted by deuterium; $C_6$-$C_{18}$-aryl, which is optionally substituted with one or more substituents $R^5$, and $C_3$-$C_{17}$-heteroaryl, which is optionally substituted with one or more substituents $R^5$;

$R^{Tz\#}$ is at each occurrence independently from another selected from the group consisting of: hydrogen, deuterium,
$C_1$-$C_{10}$-alkyl,

wherein one or more hydrogen atoms are optionally substituted by deuterium; $C_6$-$C_{18}$-aryl, which is optionally substituted with one or more substituents $R^5$, and $C_3$-$C_{17}$-heteroaryl, which is optionally substituted with one or more substituents $R^5$;

$R^{Py}$ is at each occurrence independently from another selected from the group consisting of: hydrogen, deuterium,
$C_1$-$C_{10}$-alkyl,

wherein one or more hydrogen atoms are optionally substituted by deuterium; $C_6$-$C_{18}$-aryl, which is optionally substituted with one or more substituents $R^5$, and $C_3$-$C_{17}$-heteroaryl, which is optionally substituted with one or more substituents $R^5$;

$R^{Py\#}$ is at each occurrence independently from another selected from the group consisting of: hydrogen, deuterium,
$C_1$-$C_{10}$-alkyl,

wherein one or more hydrogen atoms are optionally substituted by deuterium; $C_6$-$C_{18}$-aryl, which is optionally substituted with one or more substituents $R^5$, and $C_3$-$C_{17}$-heteroaryl, which is optionally substituted with one or more substituents $R^5$;

$R^a$, $R^3$ and $R^4$ is at each occurrence independently from another selected from the group consisting of: hydrogen, deuterium, $N(R^5)_2$, $OR^5$, $SR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$;
$C_3$-$C_{57}$-heteroaryl,
which is optionally substituted with one or more substituents $R^5$; and
a mono- or polycyclic, aliphatic, aromatic and/or benzo-fused ring system formed by ringclosure with one or more of the other substituents selected from the group consisting of $R^a$, $R^3$, $R^4$ and $R^5$;
$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and
wherein one or more non-adjacent $CH_2$-groups are optionally substituted by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and
wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and
wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and
wherein one or more non-adjacent $CH_2$-groups are optionally substituted by $R^6C=CR^6$, C=C, $Si(R^6)_2$, $Ge(R^6)_2$, $Sn(R^5)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and
wherein one or more non-adjacent $CH_2$-groups are optionally substituted by $R^6C=CR^6$, $C\equiv C$, $Si(R^6)_2$, $Ge(R^6)_2$, $Sn(R^5)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$;
$C_3$-$C_{57}$-heteroaryl,
which is optionally substituted with one or more substituents $R^6$; and
a mono- or polycyclic, aliphatic, aromatic and/or benzo-fused ring system formed by ringclosure with one or more of the other substituents selected from the group consisting of $R^a$, $R^3$, $R^4$ and $R^5$;
$R^6$ is at each occurrence independently from another selected from the group consisting of: hydrogen, deuterium, OPh, $CF_3$, CN, F,
$C_1$-$C_5$-alkyl,
wherein one or more hydrogen atoms are optionally, independently from each other substituted by deuterium,

$CN$, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
C2-C5-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,

which is optionally substituted with one or more $C_1$-$C_5$-alkyl substituents; $C_3$-$C_{17}$-heteroaryl,
which is optionally substituted with one or more $C_1$-$C_5$-alkyl substituents; N($C_6$-$C_{18}$-aryl)$_2$;

N($C_3$-$C_{17}$-heteroaryl)$_2$; and
N($C_3$-$C_{17}$-heteroaryl)($C_6$-$C_{18}$-aryl);

In particular embodiments, $R^a$, $R^3$ and/or $R^4$ form the mono- or polycyclic, aliphatic, aromatic and/or benzo-fused ring system with a substituent $R^a$, $R^3$, $R^4$ or $R^5$ that is located directly adjacent on the ring.

Further, in certain embodiments, $R^a$, $R^3$ and $R^4$ form the mono- or polycyclic, aliphatic, aromatic and/or benzo-fused ring system including at least one or more rings with a ring size of 5 to 8 atoms.

Moreover, the mono- or polycyclic, aliphatic, aromatic and/or benzo-fused ring system described above includes in certain embodiments one or more aromatic rings.

In particular, the mono- or polycyclic, aliphatic, aromatic and/or benzo-fused ring system may itself have one or more substituents $R^5$.

In certain embodiments, $R^5$ forms a mono- or polycyclic, aliphatic, aromatic and/or benzo-fused ring system together with one or more of the other $R^a$, $R^3$, $R^4$ or $R^5$ within the organic molecule, and $R^5$ forms the mono- or polycyclic, aliphatic, aromatic and/or benzo-fused ring system with a substituent $R^a$, $R^3$, $R^4$ or $R^5$ that is located directly adjacent on the ring.

Further, in certain embodiments, $R^5$ forms the mono- or polycyclic, aliphatic, aromatic and/or benzo-fused ring system including at least one or more rings with a ring size of 5 to 8 atoms.

Moreover, the mono- or polycyclic, aliphatic, aromatic and/or benzo-fused ring system includes in certain embodiments one or more aromatic rings.

In particular, the mono- or polycyclic, aliphatic, aromatic and/or benzo-fused ring system may itself have one or more substituents $R^6$.

The substituents $R^a$, $R^3$, $R^4$ or $R^5$ independently from each other optionally form a mono- or polycyclic, aliphatic, aromatic and/or benzo-fused ring system together with one or more of the other substituents $R^a$, $R^3$, $R^4$ or $R^5$.

According to the invention,

exactly one (one and only one) substituent selected from the group consisting of $R^T$, $R^V$, $R^W$, $R^X$, and $R^Y$ is $R^A$,
exactly one substituent selected from the group consisting of T, V, W, X, and Y is $R^B$,
at least one variable selected from the group consisting of $Q^1$ and $Q^2$ is N (which means that $Q^1$ is N and $Q^2$ is $CR^{Py}$; or $Q^1$ is $CR^{Py}$ and $Q^2$ is N; or, preferably, both $Q^1$ and $Q^2$ are N) i.e., $R^A$ comprises or consists of a structure of formula A-a, of formula A-b or of formula A-c:

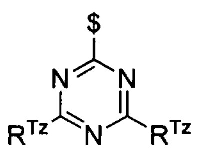
Formula A-a

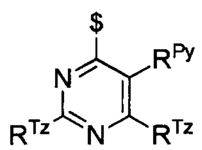
Formula A-b

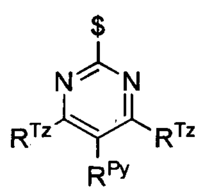
Formula A-c;

at least one variable selected from the group consisting of $Q^3$ and $Q^4$ is N (which means that $Q^3$ is N and $Q^4$ is $CR^{Py\#}$; or $Q^3$ is $CR^{Py\#}$ and $Q^4$ is N; or, preferably, both $Q^3$ and $Q^4$ are N), i.e. $R^B$ comprises or consists of a structure of formula B-a, of formula B-b or of formula B-c:

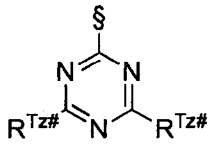

Formula B-a

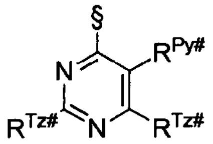

Formula B-b

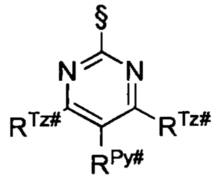

Formula B-c;

exactly one substituent selected from the group consisting of $R^T$, $R^V$, $R^W$, $R^X$, and $R^Y$ represents the binding site of a single bond linking the first chemical moiety and one of the two second chemical moieties, and
exactly one substituent selected from the group consisting of T, V, W, X and Y represents the binding site of a single bond linking the first chemical moiety and one of the two second chemical moieties.

In one embodiment of the invention, $R^{Tz}$ and $R^{Tz\#}$ is independently from each other selected from the group consisting of H, methyl and phenyl, which is optionally substituted with one or more substituents $R^6$.

In one embodiment of the invention, $R^{Py}$ and $R^{Py\#}$ are both hydrogen.

In one embodiment of the invention, $R^{Tz}$ and $R^{Tz\#}$ is independently from each other selected from the group consisting of H, methyl, and phenyl, which is optionally substituted with one or more substituents $R^6$, and
$R^{Py}$ and $R^{Py\#}$ are both hydrogen.

In one embodiment of the invention, $R^{Tz}$ and $R^{Tz\#}$ is phenyl, which is optionally substituted with one or more substituents $R^6$.

In one embodiment of the invention, $R^{Tz}$ and $R^{Tz\#}$ is phenyl.

In one embodiment of the invention, $R^{Tz}$ and $R^{Tz\#}$ is phenyl and $R^{Py}$ and $R^{Py\#}$ is hydrogen.

In one embodiment of the invention, $R^1$ and $R^2$ is independently from each other at each occurrence independently from another selected from the group consisting of: hydrogen, deuterium, CN, $CF_3$, methyl, and phenyl.

In one embodiment of the invention, $R^I$, $R^{II}$, $R^{III}$, $R^{IV}$, $R^V$, $R^{VI}$, $R^{VII}$ and $R^{VIII}$ is independently from each other at each occurrence independently from another selected from the group consisting of
hydrogen, deuterium, CN, $CF_3$, methyl, and phenyl.

In one embodiment of the invention, $R^1$, $R^2$, $R^I$, $R^{II}$, $R^{III}$, $R^{IV}$, $R^V$, $R^{VI}$, $R^{VII}$, and $R^{VIII}$ is independently from each other at each occurrence independently selected from another selected from the group consisting of
hydrogen, deuterium, and CN.

In one embodiment of the invention, $R^1$, $R^2$, $R^I$, $R^{II}$, $R^{III}$, $R^{IV}$, $R^V$, $R^{VI}$, $R^{VII}$, and $R^{VIII}$ is independently from each other at each occurrence independently selected from another selected from the group consisting of
hydrogen and CN.

In one embodiment of the invention, $R^1$, $R^2$, $R^I$, $R^{II}$, $R^{III}$, $R^{IV}$, $R^V$, $R^{VI}$, $R^{VII}$, and $R^{VIII}$ is independently from each other at each occurrence independently selected from another selected from the group consisting of

hydrogen and CN,
wherein not more than two (i.e. one or two) substituents selected from the group consisting of $R^1$, $R^2$, $R^I$, $R^{II}$, $R^{III}$, $R^{IV}$, $R^V$, $R^{VI}$, $R^{VII}$, and $R^{VIII}$ are CN.

In one embodiment of the invention, $R^1$, $R^2$, $R^I$, $R^{II}$, $R^{III}$, $R^{IV}$, $R^V$, $R^{VI}$, $R^{VII}$, and $R^{VIII}$ is independently from each other at each occurrence independently selected from another selected from the group consisting of

hydrogen and CN,
wherein exactly two substituents selected from the group consisting of $R^1$, $R^2$, $R^I$, $R^{II}$, $R^{III}$, $R^{IV}$, $R^V$, $R^{VI}$, $R^{VII}$, and $R^{VIII}$ are CN.

In one embodiment of the invention, $R^1$, $R^2$, $R^I$, $R^{II}$, $R^{III}$, $R^{IV}$, $R^V$, $R^{VI}$, $R^{VII}$, and $R^{VIII}$ is independently from each other at each occurrence independently selected from another selected from the group consisting of

hydrogen and CN,
wherein exactly one substituent selected from $R^1$ is CN and exactly one substituent selected from $R^2$ is CN.

In one embodiment of the invention, $R^{III}$ and $R^{VI}$ is CN and $R^1$, $R^2$, $R^I$, $R^{II}$, $R^{IV}$, $R^V$, $R^{VII}$, and $R^{VIII}$ is hydrogen at each occurrence.

In one embodiment of the invention, $R^{II}$ and $R^{VII}$ is CN and $R^1$, $R^2$, $R^I$, $R^{III}$, $R^{IV}$, $R^V$, $R^{VI}$, and $R^{VIII}$ is hydrogen at each occurrence.

In one embodiment of the invention, $R^{II}$ and $R^{VI}$ is CN and $R^1$, $R^2$, $R^I$, $R^{III}$, $R^{IV}$, $R^V$, $R^{VII}$, and $R^{VIII}$ is hydrogen at each occurrence.

In one embodiment of the organic molecules of the invention, $R^1$, $R^2$, $R^I$, $R^{II}$, $R^{III}$, $R^{IV}$, $R^V$, $R^{VI}$, $R^{VII}$, and $R^{VIII}$ is hydrogen at each occurrence.

In one embodiment of the invention, $R^1$, $R^2$, $R^I$, $R^{II}$, $R^{III}$, $R^{IV}$, $R^V$, $R^{VI}$, $R^{VII}$, and $R^{VIII}$ is independently from each other at each occurrence independently selected from another selected from the group consisting of

hydrogen and CN,
wherein exactly one substituent selected from the group consisting of $R^1$, $R^2$, $R^I$, $R^{II}$, $R^{III}$, $R^{IV}$, $R^V$, $R^{VI}$, $R^{III}$, and $R^{VIII}$ is CN.

In one embodiment of the invention, $R^1$, $R^2$, $R^I$, $R^{II}$, $R^{III}$, $R^{IV}$, $R^V$, $R^{VI}$, $R^{VII}$, and $R^{VIII}$ is hydrogen at each occurrence.

In one embodiment of the invention, $Q^1$, $Q^2$, $Q^3$, and $Q^4$ are all N.

In one embodiment, exactly one substituent selected from the group consisting of $R^T$, $R^V$, $R^W$, $R^X$, and $R^Y$ is $R^A$, and T is the same as $R^T$, V is the same as $R^V$, W is the same as $R^W$, X is the same as $R^X$ and Y is the same as $R^Y$.

In certain embodiments of the invention, the first chemical moiety comprises or consists of a structure of formula Ia:

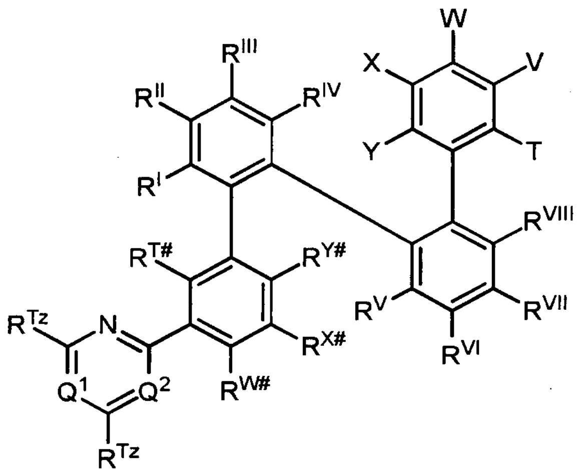

Formula Ia

wherein T, V, W, X, Y, $R^{Tz}$ and $R^I$ are defined as above;
$R^{T\#}$, $R^{W\#}$, $R^{X\#}$, and $R^{Y\#}$ is selected from group consisting of $R^1$ and the binding site of a single bond linking the first chemical moiety and one of the two second chemical moieties;
exactly one substituent selected from the group consisting of $R^{T\#}$, $R^{W\#}$, $R^{X\#}$, and $R^{Y\#}$ represents the binding site of a single bond linking the first chemical moiety and one of the two second chemical moieties.

In one embodiment of the invention, the first chemical moiety comprises or consists of a structure of formula Ia, wherein $Q^1$ and $Q^2$ are both N.

In one embodiment of the invention, the first chemical moiety comprises or consists of a structure of formula Ia,

wherein $R^1$, $R^2$, $R^I$, $R^{II}$, $R^{III}$, $R^{IV}$, $R^V$, $R^{VI}$, $R^{VII}$ and $R^{VIII}$ are all H,
and $R^{Tz}$ is phenyl at each occurrence, which is optionally substituted with one or more substituents $R^6$.

In certain embodiments of the invention, the first chemical moiety comprises or consists of a structure of formula Iaa:

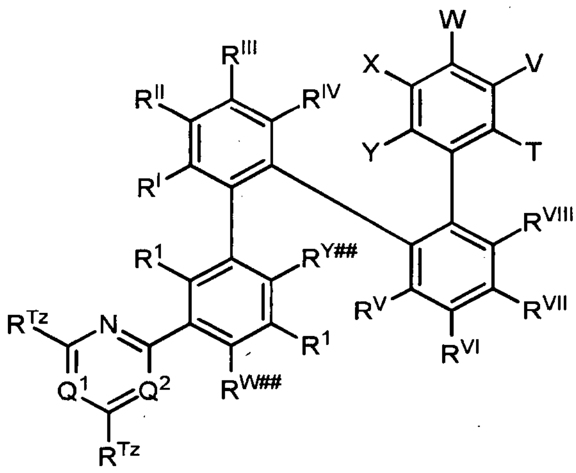

Formula Iaa

wherein T, V, W, X, Y, $R^{Tz}$, $R^1$ and $R^I$ are defined as above;
$R^{W\#\#}$ and $R^{Y\#\#}$ is selected from group consisting of $R^1$ and the binding site of a single bond linking the first chemical moiety and one of the two second chemical moieties;
exactly one substituent selected from the group consisting of $R^{W\#\#}$ and $R^{Y\#\#}$ represents the binding site of a single bond linking the first chemical moiety and one of the two second chemical moieties.

In one embodiment of the invention, the first chemical moiety comprises or consists of a structure of formula Iaa,

wherein $R^1$, $R^2$, $R^I$, $R^{II}$, $R^{III}$, $R^{IV}$, $R^V$, $R^{VI}$, $R^{VII}$ and $R^{VIII}$ are all H,
and $R^{Tz}$ is phenyl at each occurrence, which is optionally substituted with one or more substituents $R^6$.

In one embodiment of the invention, the first chemical moiety comprises or consists of a structure of formula Iaa,

wherein $R^1$, $R^2$, $R^I$, $R^{II}$, $R^{III}$, $R^{IV}$, $R^V$, $R^{VI}$, $R^{VII}$ and $R^{VIII}$ are all H,
and $R^{Tz}$ is phenyl at each occurrence.

In certain embodiments of the invention, the first chemical moiety comprises or consists of a structure of formula Iaaa:

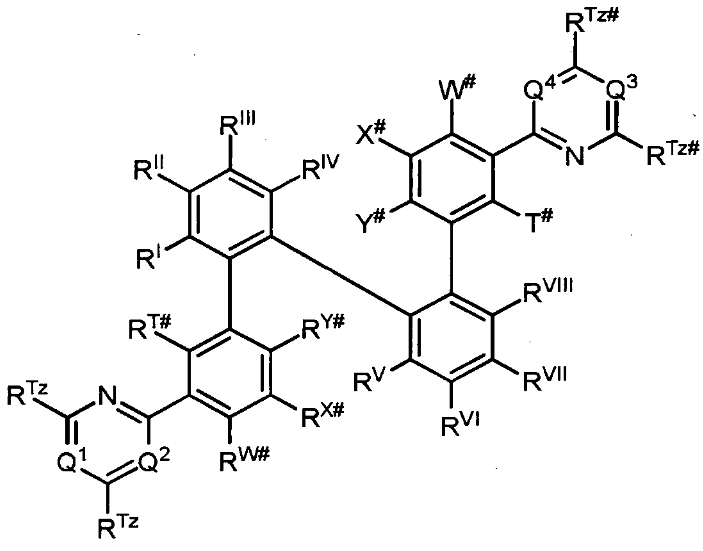

Formula Iaaa

wherein $R^{Tz}$ and $R^I$ are defined as above;

$R^{T\#}$, $R^{W\#}$, $R^{X\#}$, and $R^{Y\#}$ is selected from group consisting of $R^1$ and the binding site of a single bond linking the first chemical moiety and one of the two second chemical moieties;
$T^{\#}$, $W^{\#}$, $X^{\#}$ and $Y^{\#}$ is selected from group consisting of $R^2$ and the binding site of a single bond linking the first chemical moiety and one of the two second chemical moieties;
exactly one substituent selected from the group consisting of $R^{T\#}$, $R^{W\#}$, $R^{X\#}$, and $R^{Y\#}$ represents the binding site of a single bond linking the first chemical moiety and one of the two second chemical moieties, and
exactly one substituent selected from the group consisting of $T^{\#}$, $W^{\#}$, $X^{\#}$ and $Y^{\#}$ represents the binding site of a single bond linking the first chemical moiety to one of the two second chemical moieties.

In one embodiment of the invention, the first chemical moiety comprises or consists of a structure of formula Iaaa,

wherein $R^1$, $R^2$, $R^I$, $R^{II}$, $R^{III}$, $R^{IV}$, $R^V$, $R^{VI}$, $R^{VII}$ and $R^{VIII}$ are all H,
and $R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment of the invention, the first chemical moiety comprises or consists of a structure of formula Iaaa,

wherein $R^1$, $R^2$, $R^I$, $R^{II}$, $R^{III}$, $R^{IV}$, $R^V$, $R^{VI}$, $R^{VII}$ and $R^{VIII}$ are all H,
$R^{Tz}$ is phenyl at each occurrence, and
$R^{Tz\#}$ is phenyl at each occurrence.

In certain embodiments of the invention, the first chemical moiety comprises or consists of a structure of formula Iaaaa:

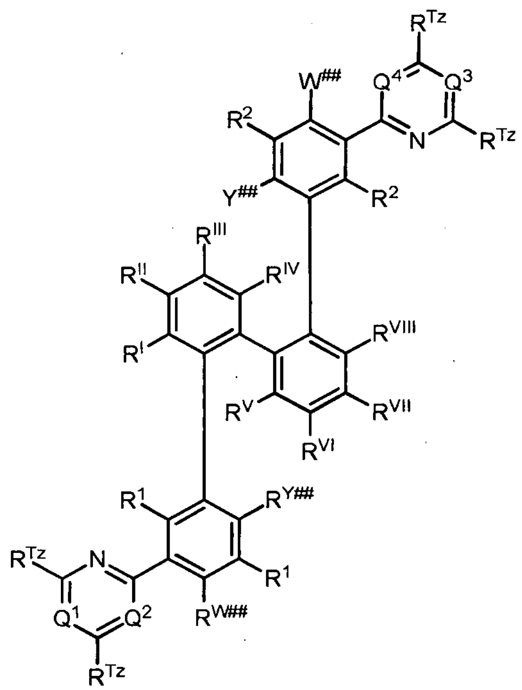

Formula Iaaaa

wherein $R^1$, $R^2$, $R^{Tz}$ and $R^I$ are defined as above;
$R^{W\#\#}$ and $R^{Y\#\#}$ is selected from group consisting of $R^1$ and the binding site of a single bond linking the first chemical moiety and one of the two second chemical moieties;
$W^{\#\#}$ and $Y^{\#\#}$ is selected from group consisting of $R^2$ and the binding site of a single bond linking the first chemical moiety and one of the two second chemical moieties;
exactly one substituent selected from the group consisting of $R^{W\#\#}$ and $R^{Y\#\#}$ represents the binding site of a single bond linking the first chemical moiety and one of the two second chemical moieties, and
exactly one substituent selected from the group consisting of $W^{\#\#}$ and $Y^{\#\#}$ represents the binding site of a single bond linking the first chemical moiety and one of the two second chemical moieties.

In one embodiment of the invention, the first chemical moiety comprises or consists of a structure of formula Iaaaa,

wherein $R^1$, $R^2$, $R^I$, $R^{II}$, $R^{III}$, $R^{IV}$, $R^V$, $R^{VI}$, $R^{VII}$ and $R^{VIII}$ is H or CN,
$R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment of the invention, the first chemical moiety comprises or consists of a structure of formula Iaaaa,

wherein $R^1$, $R^2$, $R^I$, $R^{II}$, $R^{III}$, $R^{IV}$, $R^V$, $R^{VI}$, $R^{VII}$ and $R^{VIII}$ is H or CN,
wherein a maximum of two substituents (i.e. one or two substituents) selected form the group consisting of $R^1$, $R^2$, $R^I$, $R^{II}$, $R^{III}$, $R^{IV}$, $R^V$, $R^{VI}$, $R^{VII}$ and $R^{VIII}$ are CN,
$R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment of the invention, the first chemical moiety comprises or consists of a structure of formula Iaaaa,

wherein $R^1$, $R^2$, $R^I$, $R^{II}$, $R^{III}$, $R^{IV}$, $R^V$, $R^{VI}$, $R^{VII}$ and $R^{VIII}$ are all H,
$R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment of the invention, the first chemical moiety comprises or consists of a structure of formula Iaaaa,

wherein $R^1$, $R^2$, $R^I$, $R^{II}$, $R^{III}$, $R^{IV}$, $R^V$, $R^{VI}$, $R^{VII}$ and $R^{VIII}$ are all H, and
$R^{Tz}$ and $R^{Tz\#}$ are both phenyl.

In one embodiment of the invention, the first chemical moiety comprises or consists of a structure of formula Iaaaa,

wherein $R^1$, $R^2$, $R^I$, $R^{II}$, $R^{III}$, $R^{IV}$, $R^V$, $R^{VI}$, $R^{VII}$ and $R^{VIII}$ is H or CN,
wherein exactly one substituents $R^1$ is CN,
and exactly one substituent $R^2$ is CN,
$R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment of the invention, the first chemical moiety comprises or consists of a structure of formula Iaaaa,

wherein $R^1$, $R^2$, $R^I$, $R^{II}$, $R^{III}$, $R^{IV}$, $R^V$, $R^{VI}$, $R^{VII}$ and $R^{VIII}$ is H or CN,
wherein exactly two substituents selected form the group consisting of $R^I$, $R^{II}$, $R^{III}$, $R^{IV}$, $R^V$, $R^{VI}$, $R^{VII}$ and $R^{VIII}$ are CN,
$R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment of the invention, the first chemical moiety comprises or consists of a structure of formula Iaaaa,

wherein $R^1$, $R^2$, $R^I$, $R^{III}$, $R^{IV}$, $R^V$, $R^{VI}$, and $R^{VIII}$ are all H,
$R^{II}$ is CN,
$R^{VII}$ is CN,
$R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment of the invention, the first chemical moiety comprises or consists of a structure of formula Iaaaa,

wherein $R^1$, $R^2$, $R^I$, $R^{II}$, $R^{IV}$, $R^V$, $R^{VII}$, and $R^{VIII}$ are all H,
$R^{III}$ is CN,
$R^{VI}$ is CN,
$R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment of the invention, the first chemical moiety comprises or consists of a structure of formula Iaaaa,

wherein $R^1$, $R^2$, $R^I$, $R^{III}$, $R^{IV}$, $R^V$, $R^{VII}$, and $R^{VIII}$ are all H,
$R^{II}$ is CN,
$R^{VI}$ is CN,
$R^{Tz}$ is phenyl at each occurrence, which is optionally substituted with one or more substituents $R^6$, and
$R^{Tz\#}$ is phenyl at each occurrence, which is optionally substituted with one or more substituents $R^6$.

In one embodiment, T is 1,3,5-triazinyl, which is substituted with two substituents $R^{Tz}$.

In one embodiment, V is 1,3,5-triazinyl, which is substituted with two substituents $R^{Tz}$.

In one embodiment, W is 1,3,5-triazinyl, which is substituted with two substituents $R^{Tz}$.

In one embodiment, $R^T$ is 1,3,5-triazinyl, which is substituted with two substituents $R^{Tz}$.

In one embodiment, $R^V$ is 1,3,5-triazinyl, which is substituted with two substituents $R^{Tz}$.

In one embodiment, $R^W$ is 1,3,5-triazinyl, which is substituted with two substituents $R^{Tz}$.

In a further embodiment of the invention, $R^{Tz}$ is independently from each other selected from the group consisting of H, methyl,
phenyl, which is optionally substituted with one or more substituents $R^6$, 1,3,5-triazinyl, which is optionally substituted with one or more substituents $R^6$, and pyrimidinyl, which is optionally substituted with one or more substituents $R^6$.

In a further embodiment of the invention, $R^{Tz}$ is independently from each other selected from the group consisting of H, methyl and phenyl, which is optionally substituted with one or more substituents $R^6$.

In a further embodiment of the invention, $R^{Tz}$ is independently from each other selected from the group consisting of H, methyl and phenyl.

In a further embodiment of the invention, $R^{Tz}$ is phenyl at each occurrence, which is optionally substituted with one or more substituents $R^6$.

In a further embodiment of the invention, $R^{Tz}$ is phenyl at each occurrence.

In one embodiment, $R^a$ is H at each occurrence.

In one embodiment, the two second chemical moieties each are different from each other, wherein exactly one $R^a$ is not H.

In a further embodiment of the invention, the two second chemical moieties of the organic molecule each at each occurrence independently from another comprise or consist of a structure of formula IIa:

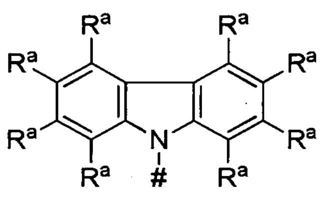

Formula IIa

wherein # and $R^a$ are defined as above.

In a further embodiment of the invention, $R^a$ is at each occurrence independently from another selected from the group consisting of H,

Me,
$^i$Pr,
$^t$Bu,
CN,
$CF_3$,
Ph, which is optionally substituted with one or more substituents independently from each other selected from the

group consisting of Me, ${}^{i}$Pr,${}^{t}$Bu,CN, $CF_3$, and Ph,
pyridinyl, which is optionally substituted with one or more substituents independently from each other selected from the group consisting of Me, ${}^{i}$Pr,${}^{t}$Bu, CN, $CF_3$, and Ph,
pyrimidinyl, which is optionally substituted with one or more substituents independently from each other selected from the group consisting of Me, ${}^{i}$Pr,${}^{t}$Bu, CN, $CF_3$, and Ph,
carbazolyl, which is optionally substituted with one or more substituents independently from each other selected from the group consisting of Me, ${}^{i}$Pr,${}^{t}$Bu, CN; $CF_3$, and Ph,
triazinyl, which is optionally substituted with one or more substituents independently from each other selected from the group consisting of Me, ${}^{i}$Pr,${}^{t}$Bu, CN, $CF_3$, and Ph,
and $N(Ph)_2$.

In a further embodiment of the invention, $R^a$ is at each occurrence independently from another selected from the group consisting of H,

Me,
${}^{i}$Pr,
${}^{t}$Bu,
CN,
$CF_3$,
Ph, which is optionally substituted with one or more substituents independently from each other selected from the group consisting of Me, ${}^{i}$Pr,${}^{t}$Bu,CN, $CF_3$, and Ph,
pyridinyl, which is optionally substituted with one or more substituents independently from each other selected from the group consisting of Me, ${}^{i}$Pr, ${}^{t}$Bu, CN, $CF_3$, and Ph,
pyrimidinyl, which is optionally substituted with one or more substituents independently from each other selected from the group consisting of Me, ${}^{i}$Pr,${}^{t}$Bu, CN, $CF_3$, and Ph, and
triazinyl, which is optionally substituted with one or more substituents independently from each other selected from the group consisting of Me, ${}^{i}$Pr,${}^{t}$Bu, CN, $CF_3$, and Ph.

In a further embodiment of the invention, $R^a$ is at each occurrence independently from another selected from the group consisting of H,

Me,
${}^{t}$Bu,
Ph, which is optionally substituted with one or more substituents independently from each other selected from the group consisting of Me, ${}^{i}$Pr,${}^{t}$Bu,CN, $CF_3$, and Ph,
triazinyl, which is optionally substituted with one or more substituents independently from each other selected from the group consisting of Me, ${}^{i}$Pr,${}^{t}$Bu, CN, $CF_3$, and Ph.

In a further embodiment of the invention, $R^a$ is at each occurrence independently from another selected from the group consisting of H, Me, ${}^{i}$Pr,${}^{t}$Bu,CN, $CF_3$, ${}^{t}$Bu, and Ph.
In a further embodiment of the invention, $R^a$ is at each occurrence independently from another selected from the group consisting of H, Me, ${}^{t}$Bu, and Ph.
In a further embodiment of the invention, $R^a$ is H at each occurrence.
In a further embodiment of the invention, the two second chemical moieties each at each occurrence independently from another comprise or consist of a structure of formula Iib, a structure of formula IIb-2, a structure of formula IIb-3 or a structure of formula IIb-4:

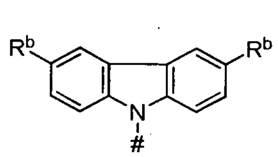
Formula IIb

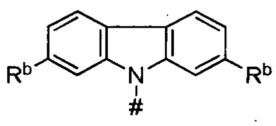
Formula IIb-2

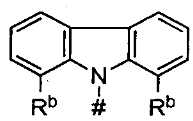
Formula IIb-3

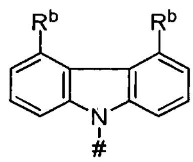
Formula IIb-4

wherein

$R^b$ is at each occurrence independently from another selected from the group consisting of

H,
deuterium,
$N(R^5)_2$,
$OR^5$,
$Si(R^5)_3$,
$B(OR^5)_2$,
$OSO_2R^5$,
$CF_3$,
CN,
F,
Br,
I,
$C_1$-$C_{40}$-alkyl,

which is optionally substituted with one or more substituents $R^5$ and
wherein one or more non-adjacent $CH_2$-groups are optionally substituted by $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;

$C_1$-$C_{40}$-alkoxy,
which is optionally substituted with one or more substituents $R^5$ and wherein one or more non-adjacent $CH_2$-groups are optionally substituted by $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;
$C_1$-$C_{40}$-thioalkoxy,

which is optionally substituted with one or more substituents $R^5$ and
wherein one or more non-adjacent $CH_2$-groups are optionally substituted by $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;

$C_2$-$C_{40}$-alkenyl,
which is optionally substituted with one or more substituents $R^5$ and wherein one or more non-adjacent $CH_2$-groups are optionally substituted by $R^5C{=}CR^5$, C≡C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;
$C_2$-$C_{40}$-alkynyl,
which is optionally substituted with one or more substituents $R^5$ and wherein one or more non-adjacent $CH_2$-groups are optionally substituted by $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S or $CONR^5$;
$C_6$-$C_{60}$-aryl,

which is optionally substituted with one or more substituents $R^5$; and $C_3$-$C_{57}$-heteroaryl,
which is optionally substituted with one or more substituents $R^5$.

Apart from that the aforementioned definitions apply.

In another embodiment of the invention, the two second chemical moieties each at each occurrence independently from another comprise or consist of a structure of formula IIc, a structure of formula IIc-2, a structure of formula IIc-3 or a structure of formula IIc-4:

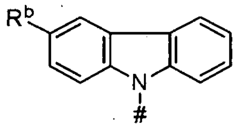
Formula IIc

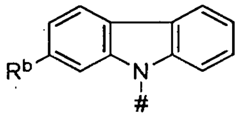
Formula IIc-2

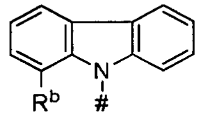
Formula IIc-3

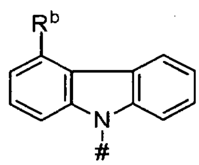
Formula IIc-4

wherein the aforementioned definitions apply.

In a further embodiment of the invention, $R^b$ is at each occurrence independently from another selected from the group consisting of

H,
Me,
$^{i}Pr$,
$^{t}Bu$,
CN,
$CF_3$,
Ph, which is optionally substituted with one or more substituents independently from each other selected from the group consisting of Me, $^{i}Pr$,$^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$,$^{t}Bu$,CN, $CF_3$, and Ph, and
carbazolyl, which is optionally substituted with one or more substituents independently from each other selected from the group consisting of Me, $^{i}Pr$,$^{t}Bu$,CN, $CF_3$, and Ph,
triazinyl, which is optionally substituted with one or more substituents independently from each other selected from the group consisting of Me, $^{i}Pr$, $^{t}Bu$, CN, $CF_3$, and Ph,
and $N(Ph)_2$.

In a further embodiment of the invention, $R^b$ is at each occurrence independently from another selected from the group consisting of

Me,
$^{i}Pr$,
$^{t}Bu$,
CN,
CF3,
Ph, which is optionally substituted with one or more substituents independently from each other selected from the group consisting of Me, $^{i}Pr$,$^{t}Bu$, CN, $CF_3$, and Ph,
pyridinyl, which is optionally substituted with one or more substituents independently from each other selected from the group consisting of Me, $^{i}Pr$,$^{t}Bu$, CN, $CF_3$, and Ph,
pyrimidinyl, which is optionally substituted with one or more substituents independently from each other selected from the group consisting of Me, $^{i}Pr$,$^{t}Bu$,CN, $CF_3$, and Ph,
carbazolyl, which is optionally substituted with one or more substituents independently from each other selected from the group consisting of Me, $^{i}Pr$,$^{t}Bu$, CN, $CF_3$ and Ph; and
triazinyl, which is optionally substituted with one or more substituents independently from each other selected from the group consisting of Me, $^{i}Pr$,$^{t}Bu$, CN, $CF_3$, and Ph.

In a further embodiment of the invention, $R^b$ is at each occurrence independently from another selected from the group consisting of

H,
Me,
$^{t}Bu$,
Ph, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^b$ is at each occurrence independently from another selected from the group consisting of

Me,
$^{t}Bu$,
Ph, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph.

In a further embodiment of the invention, $R^b$ is at each occurrence independently from another selected from the group consisting of H, Me, $^i$Pr, $^t$Bu, CN, $CF_3$, $^t$Bu, and Ph.

In a further embodiment of the invention, $R^b$ is at each occurrence independently from another selected from the group consisting of Me, $^i$Pr, $^t$Bu, CN, $CF_3$, $^t$Bu, and Ph.

In a further embodiment of the invention, $R^b$ is at each occurrence independently from another selected from the group consisting of H, Me, $^t$Bu, and Ph.

In a further embodiment of the invention, $R^b$ is at each occurrence independently from another selected from the group consisting of Me, $^t$Bu, and Ph.

In the following, exemplary embodiments of the second chemical moiety are shown:

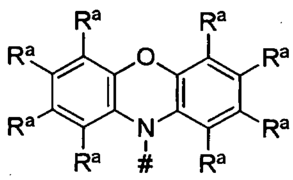
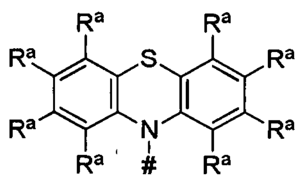
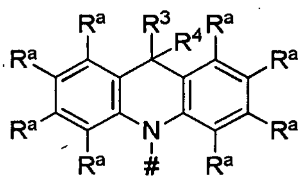
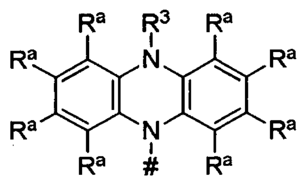
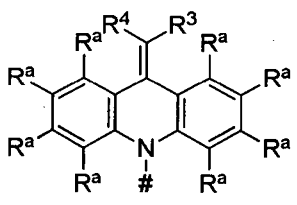
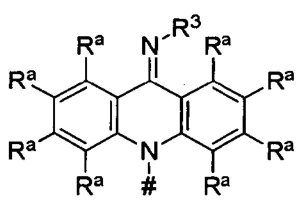
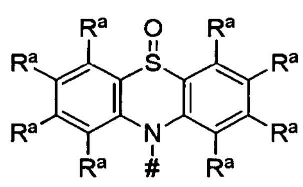
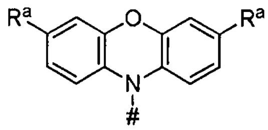
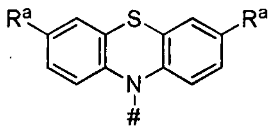
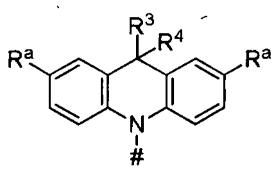
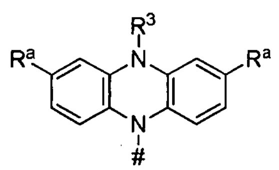
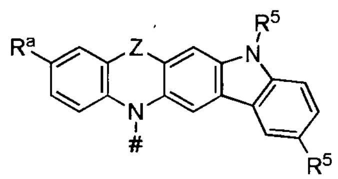
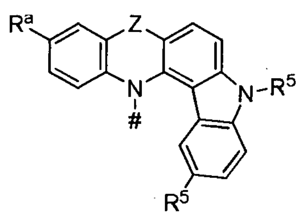
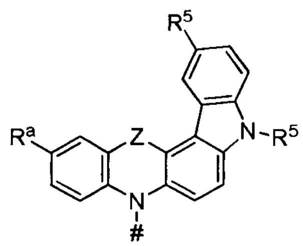
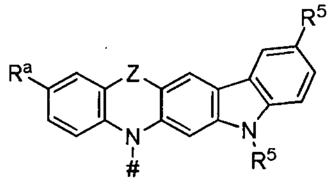
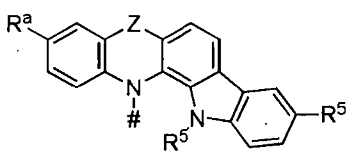
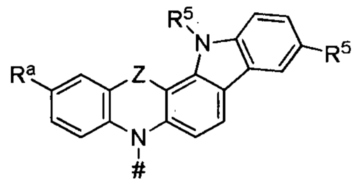
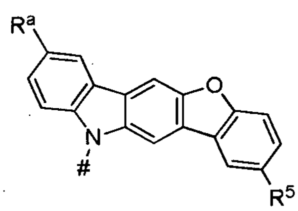
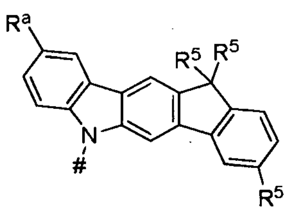
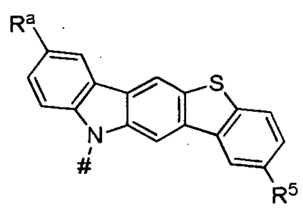

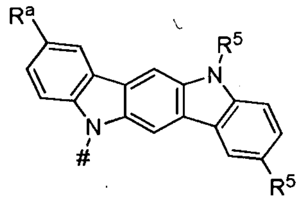

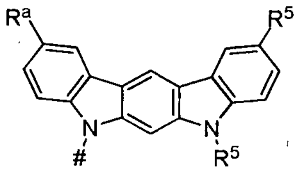

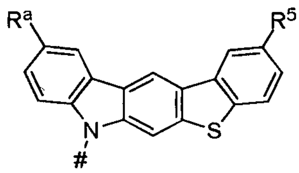

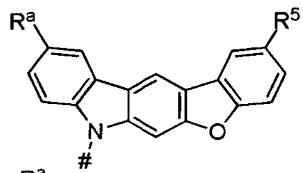

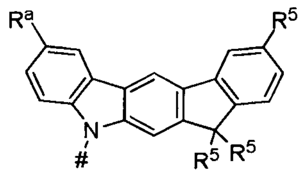

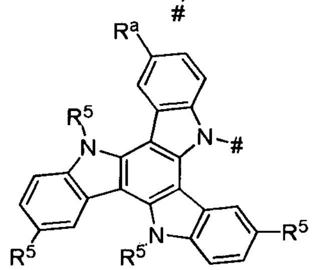

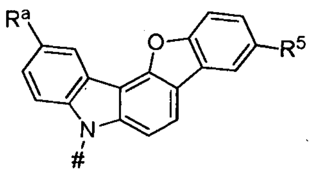

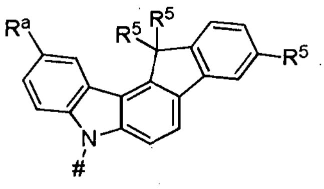

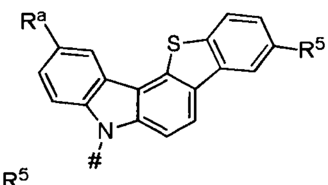

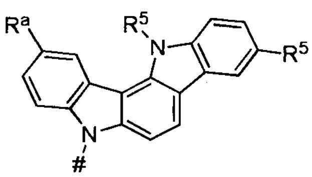

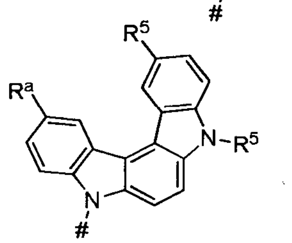

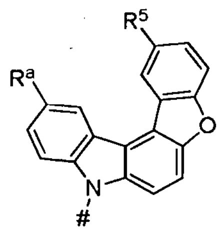

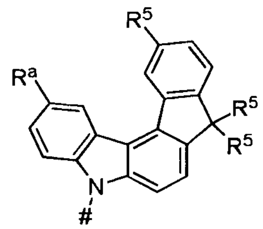

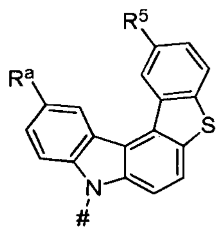

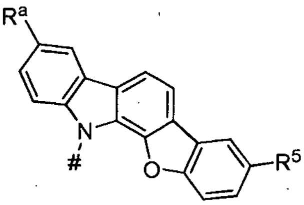
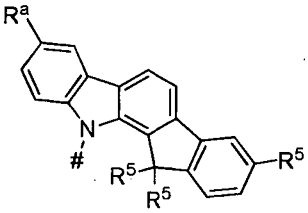
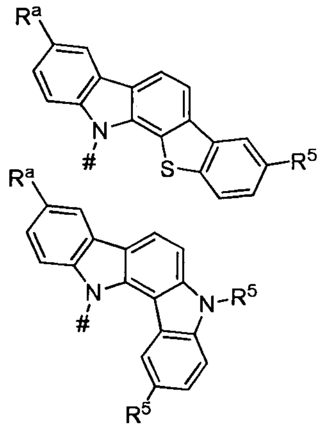
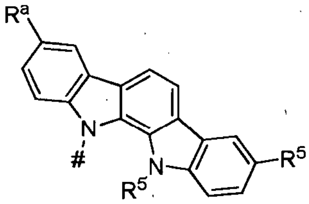
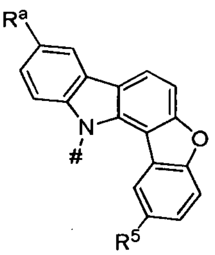
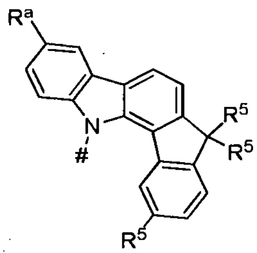
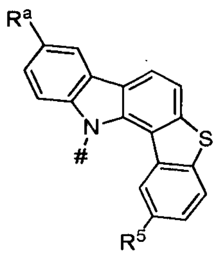

wherein for #, Z, $R^a$, $R^3$, $R^4$ and $R^5$, the aforementioned definitions apply.

In one embodiment, $R^a$ and $R^5$ is at each occurrence independently from another selected from the group consisting of hydrogen (H), methyl (Me), i-propyl ($CH(CH_3)_2$) ($^iPr$), t-butyl ($^tBu$), phenyl (Ph), CN, $CF_3$, and diphenylamine ($NPh_2$).

In one embodiment of the invention, the organic molecules comprise or consist of a structure of formula III:

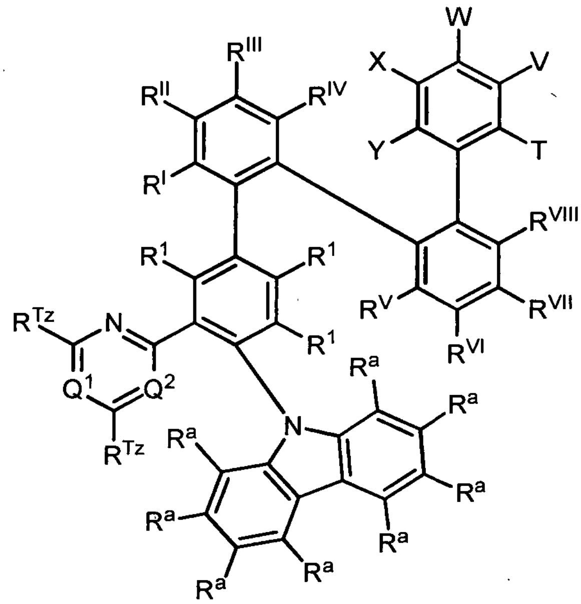

Formula III

wherein any one of the aforementioned definitions apply.

In a further embodiment of the invention, the organic molecules comprise or consist of a structure selected from the group consisting of formula IIIa-1 to formula IIIa-3:

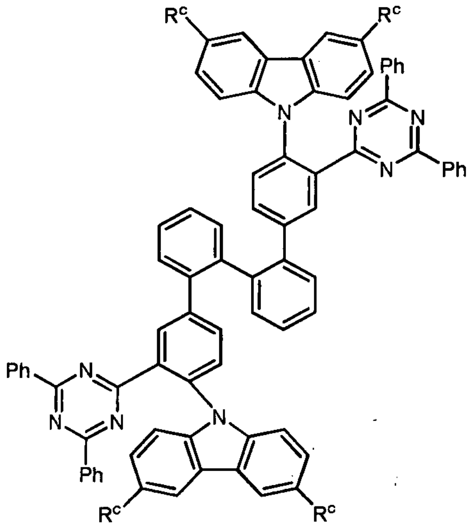

Formula IIIa-1

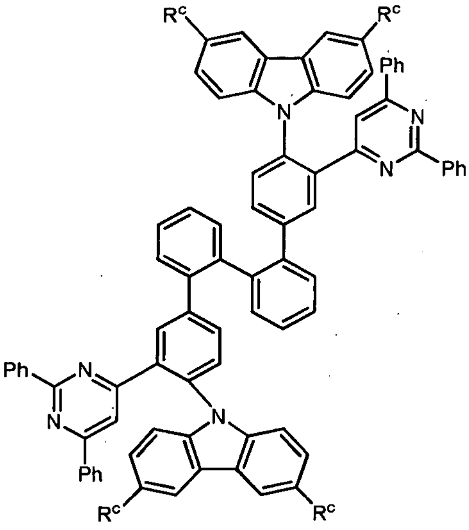

Formula IIIa-2

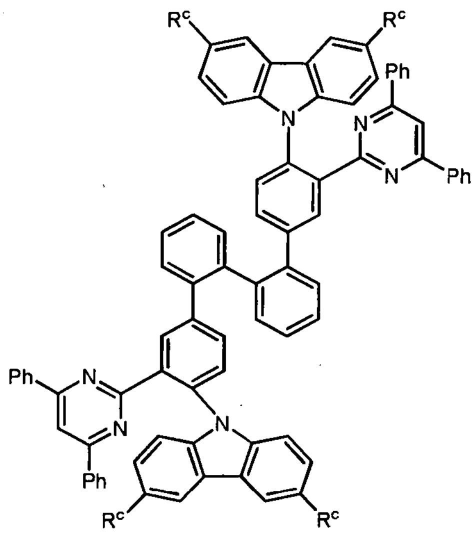

Formula IIIa-3

wherein

$R^c$ is at each occurrence independently from another selected from the group consisting of
H,
Me,
$^iPr$,
$^tBu$,
Ph, which is optionally substituted with one or more substituents independently from each other selected from the group consisting of Me, $^iPr$, $^tBu$, CN, $CF_3$, and Ph,
pyridinyl, which is optionally substituted with one or more substituents independently from each other selected from the group consisting of Me, $^iPr$, $^tBu$, CN, $CF_3$, and Ph,
pyrimidinyl, which is optionally substituted with one or more substituents independently from each other selected from the group consisting of Me, $^iPr$, $^tBu$, CN, $CF_3$, and Ph,
carbazolyl, which is optionally substituted with one or more substituents independently from each other selected from the group consisting of Me, $^iPr$, $^tBu$, CN, $CF_3$, and Ph,
triazinyl, which is optionally substituted with one or more substituents independently from each other selected from the group consisting of Me, $^iPr$, $^tBu$, CN, $CF_3$, and Ph,

and $N(Ph)_2$
and wherein apart from that any one of the aforementioned definitions apply.

In one embodiment of the invention, $R^c$ is at each occurrence independently from another selected from the group consisting of

Me,
$^{i}$Pr,
$^{t}$Bu,
Ph, which is optionally substituted with one or more substituents independently from each other selected from the group consisting of Me, $^{i}$Pr, $^{t}$Bu, CN, $CF_3$, and Ph,
pyridinyl, which is optionally substituted with one or more substituents independently from each other selected from the group consisting of Me, $^{i}$Pr, $^{t}$Bu, CN, $CF_3$, and Ph,
pyrimidinyl, which is optionally substituted with one or more substituents independently from each other selected from the group consisting of Me, $^{i}$Pr, $^{t}$Bu, CN, $CF_3$, and Ph,
carbazolyl, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph,
and $N(Ph)_2$.

In a preferred embodiment of the invention, the organic molecules comprise or consist of a structure selected from the group consisting of formula IIIa-1 and formula IIIa-2:

In a further embodiment of the invention, the organic molecules comprise or consist of a structure selected from the group consisting of formula IIIb-1 to formula IIIb-3:

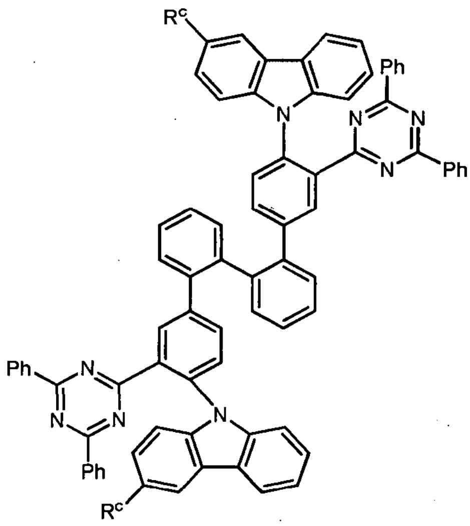

Formula IIIb-1

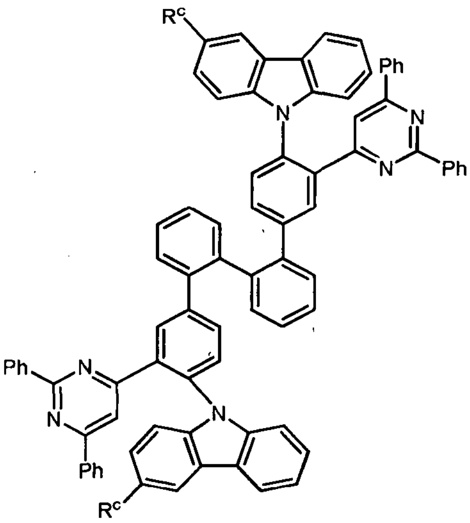

Formula IIIb-2

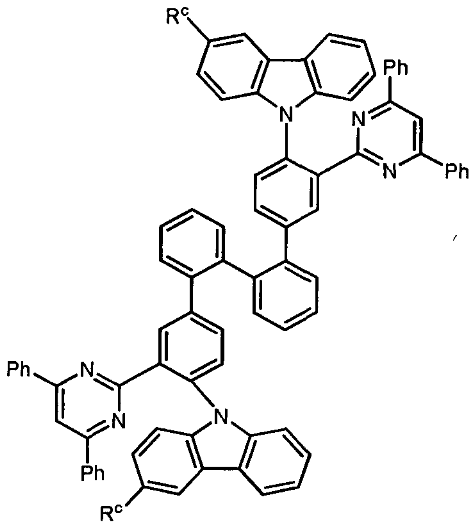

Formula IIIb-3

wherein any one of the aforementioned definitions apply.

In a preferred embodiment of the invention, the organic molecules comprise or consist of a structure selected from the group consisting of formula IIIb-1 and formula IIIb-2.

In one embodiment of the invention, the organic molecules comprise or consist of a structure of formula III-CN-1, formula III-CN-2 or formula III-CN-3:

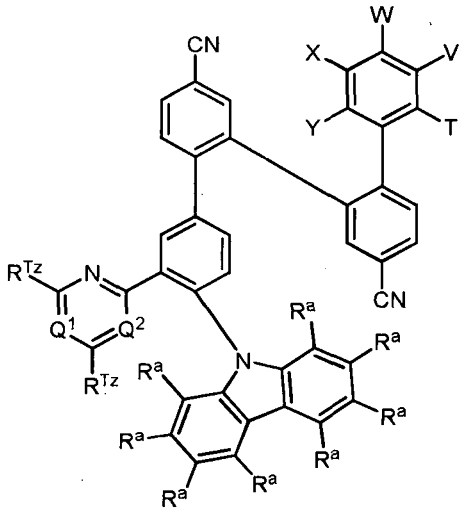

Formula III-CN-1

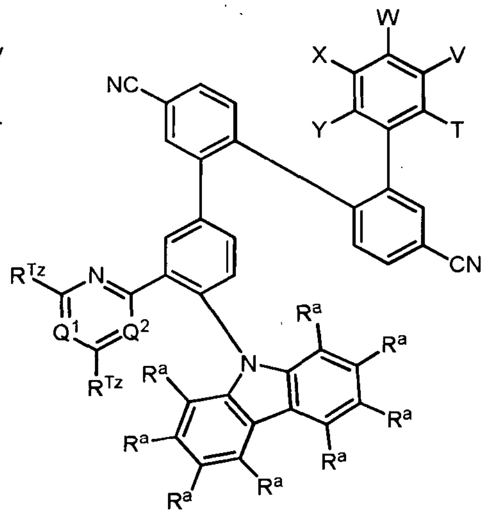

Formula III-CN-2

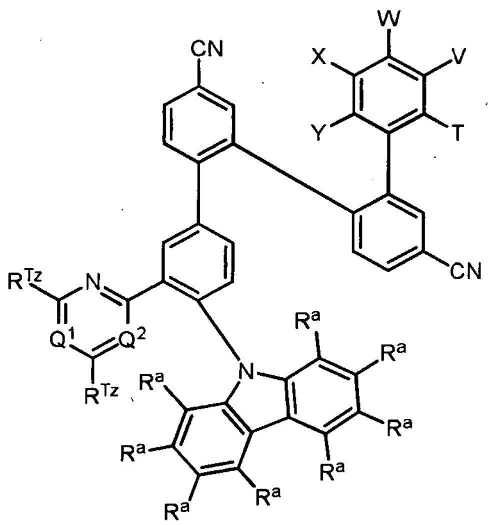

Formula III-CN-3

wherein any one of the aforementioned definitions apply.

In one embodiment of the invention, the organic molecules comprise or consist of a structure of formula IV:

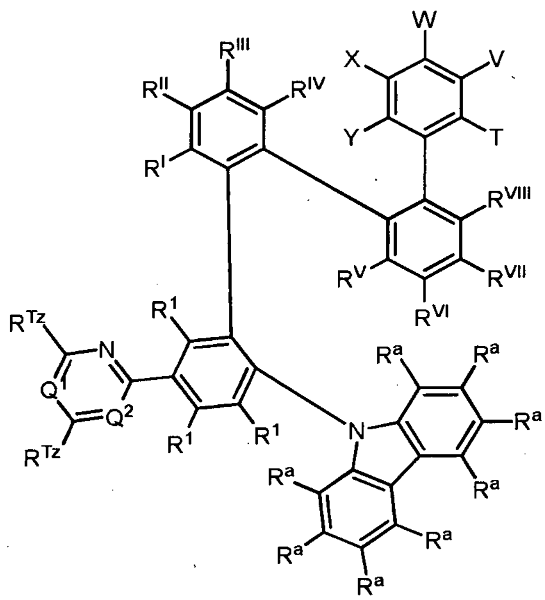

Formula IV

wherein any one of the aforementioned definitions apply.

In a further embodiment of the invention, the organic molecules comprise or consist of a structure selected from the group consisting of formula IVa-1 to formula IVa-3:

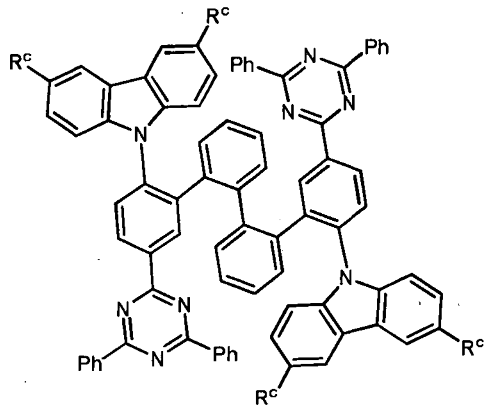

Formula IVa-1

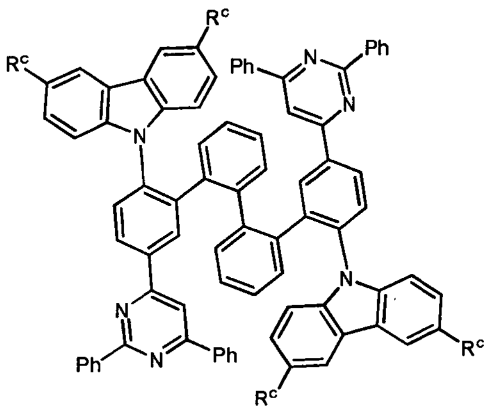

Formula IVa-2

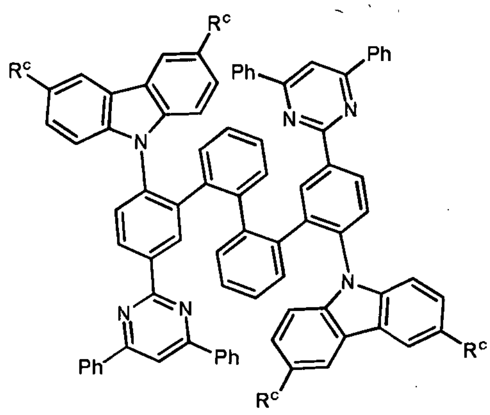

Formula IVa-3

wherein any one of the aforementioned definitions apply.

In a preferred embodiment of the invention, the organic molecules comprise or consist of a structure selected from the group consisting of formula IVa-1 and formula IVa-2.

In a further embodiment of the invention, the organic molecules comprise or consist of a structure selected from the group consisting of formula IVb-1 to formula IVb-3:

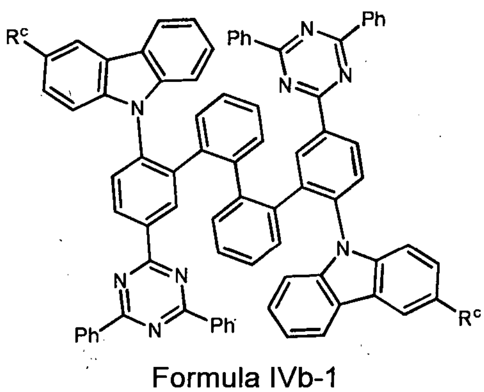

Formula IVb-1

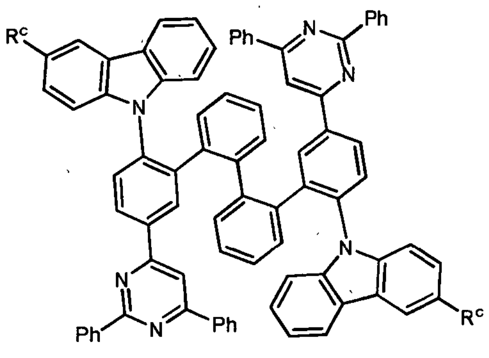

Formula IVb-2

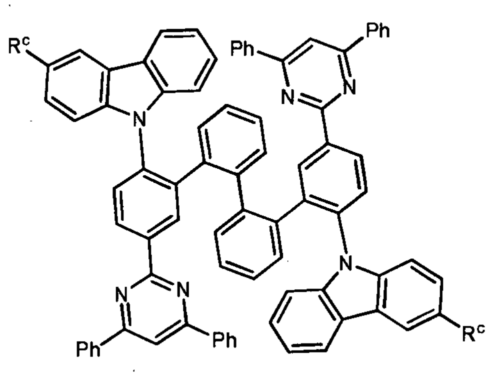

Formula IVb-3

wherein any one of the aforementioned definitions apply.

In a preferred embodiment of the invention, the organic molecules comprise or consist of a structure selected from the group consisting of formula IVb-1 and formula IVb-2.

In one embodiment of the invention, the organic molecules comprise or consist of a structure consisting of formula IV-CN-1, formula IV-CN-2, formula IV-CN-3 or formula IV-CN-4:

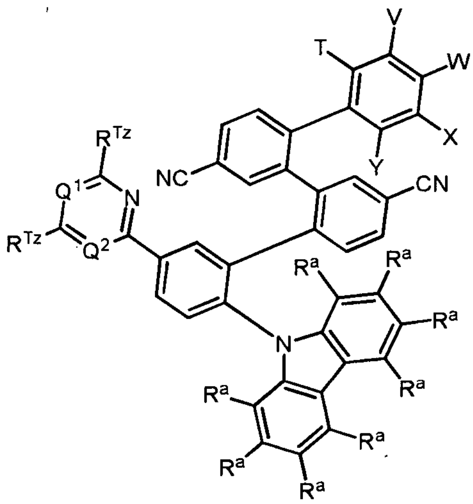

Formula IV-CN-1

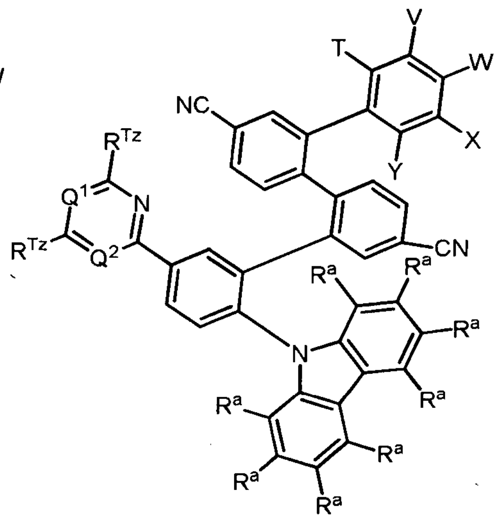

Formula IV-CN-2

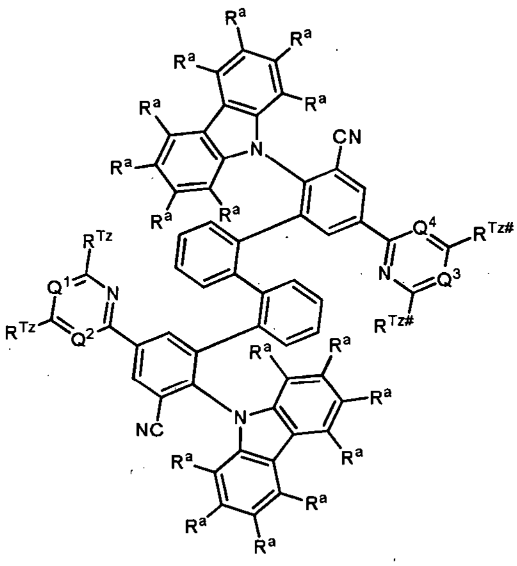

Formula IV-CN-3

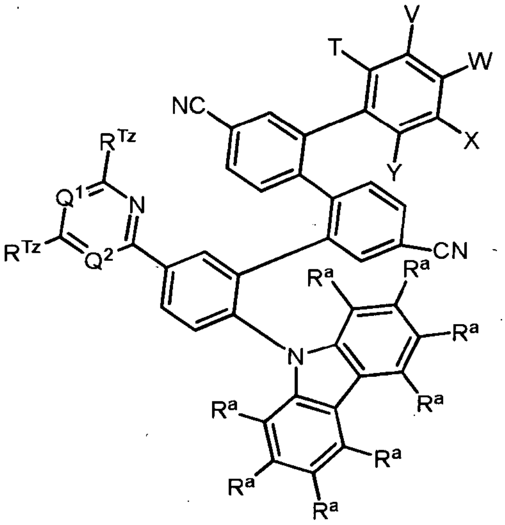

Formula IV-CN-4

wherein any one of the aforementioned definitions apply.

In one embodiment of the invention, the organic molecules comprise or consist of a structure of formula V:

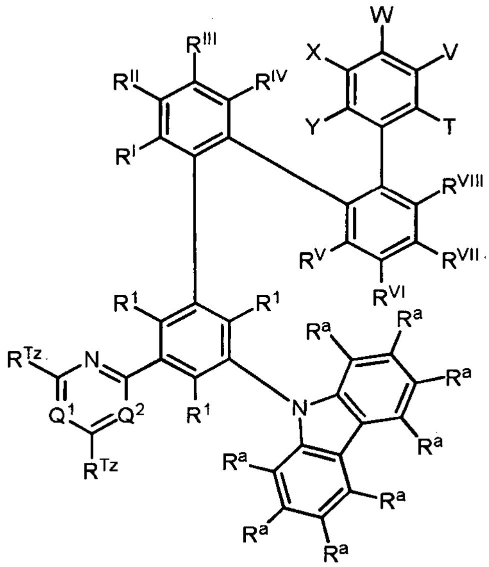

Formula V

wherein any one of the aforementioned definitions apply.

In one embodiment of the invention, the organic molecules comprise or consist of a structure of formula V-2:

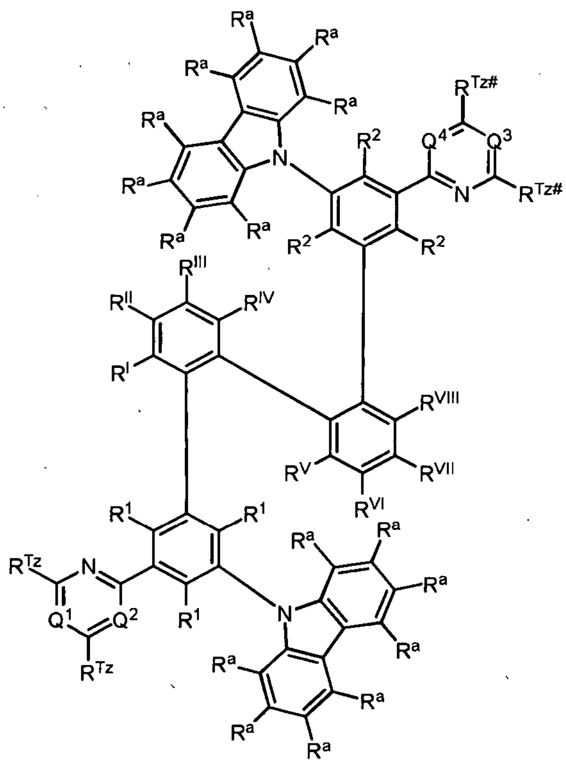

Formula V-2

wherein any one of the aforementioned definitions apply.

In one embodiment of the invention, the organic molecules comprise or consist of a structure of formula VI:

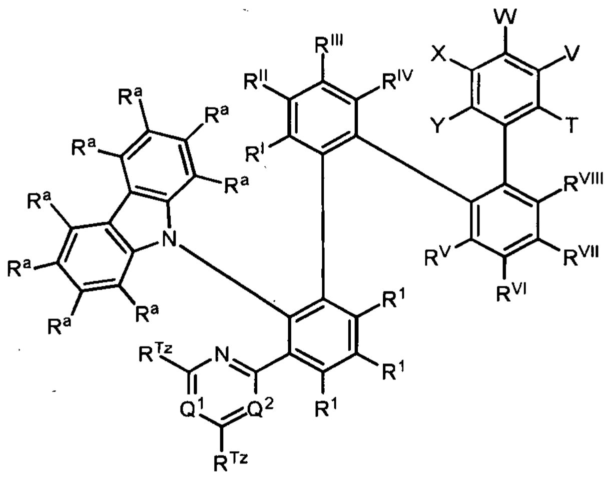

Formula VI

wherein any one of the aforementioned definitions apply.

In one embodiment of the invention, the organic molecules comprise or consist of a structure of formula VI-2:

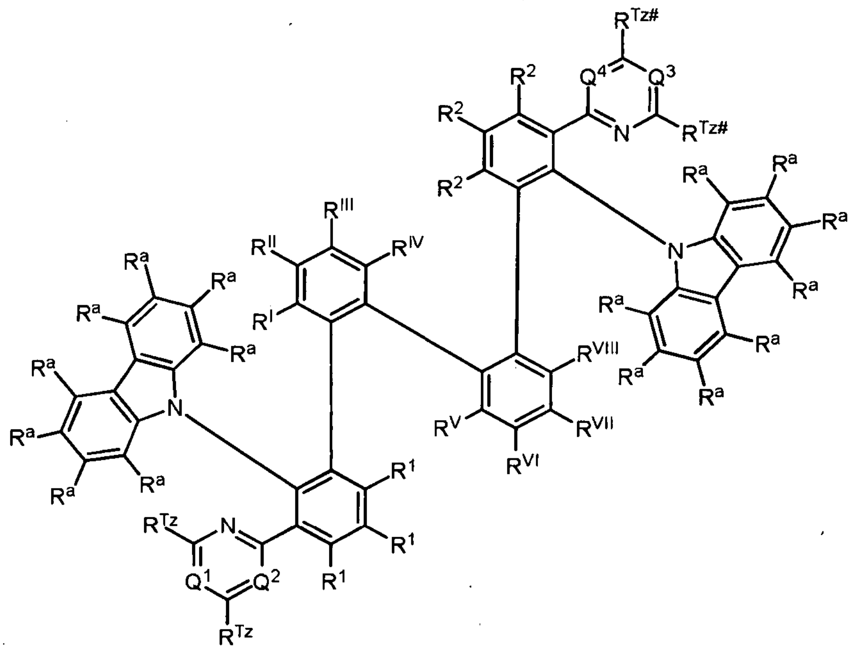

Formula VI-2

wherein any one of the aforementioned definitions apply.

In one embodiment of the invention, the organic molecules comprise or consist of a structure of formula VII:

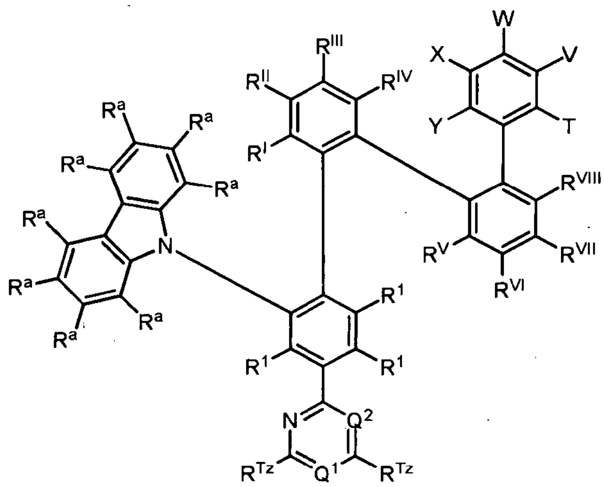

Formula VII

wherein any one of the aforementioned definitions apply.

In one embodiment of the invention, the organic molecules comprise or consist of a structure of formula VII-2:

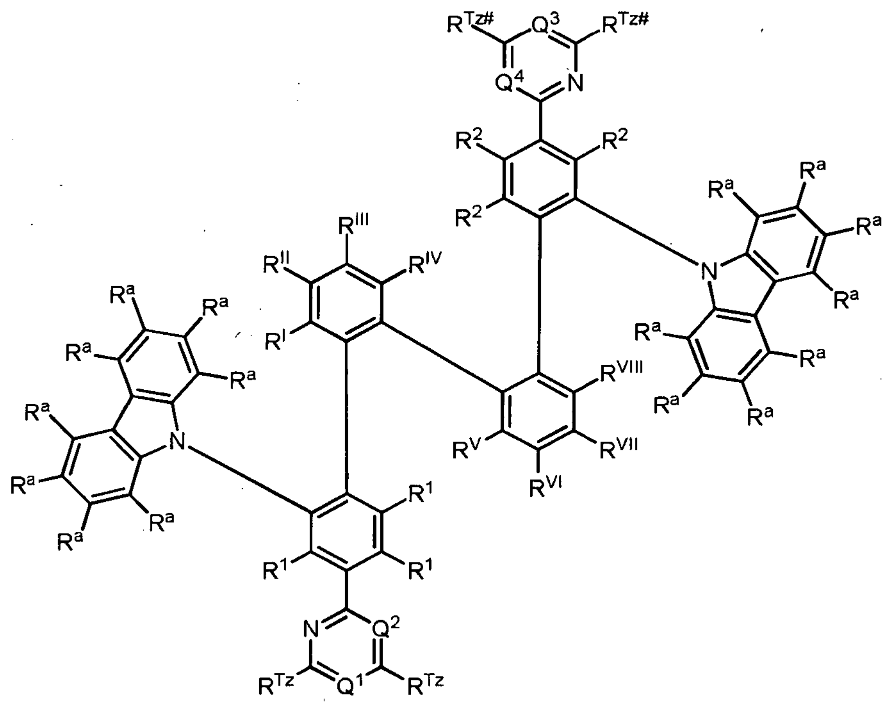

Formula VII-2

wherein any one of the aforementioned definitions apply.

In one embodiment of the invention, the organic molecules comprise or consist of a structure of formula VIII:

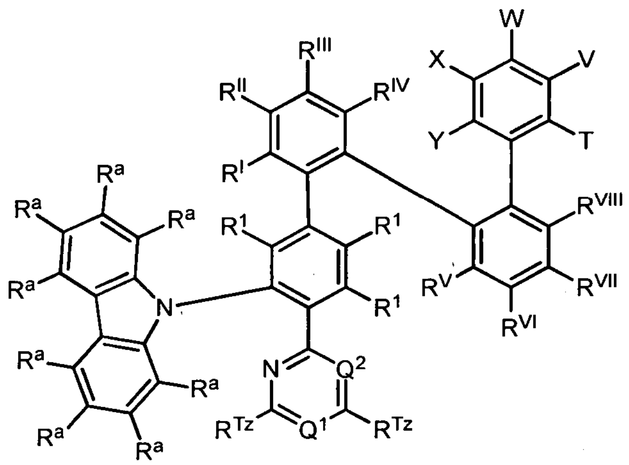

Formula VIII

wherein any one of the aforementioned definitions apply.

In one embodiment of the invention, the organic molecules comprise or consist of a structure of formula VIII-2:

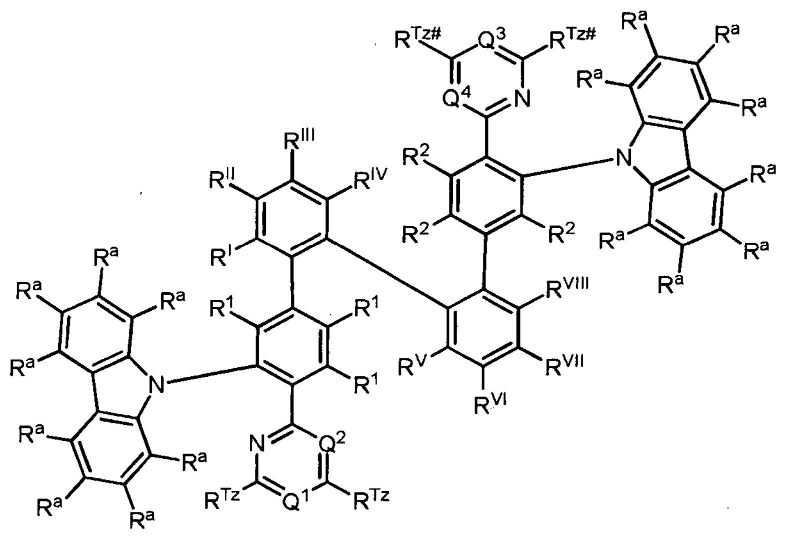

Formula VIII-2

wherein any one of the aforementioned definitions apply.

In one embodiment of the invention, the organic molecules comprise or consist of a structure of formula IX:

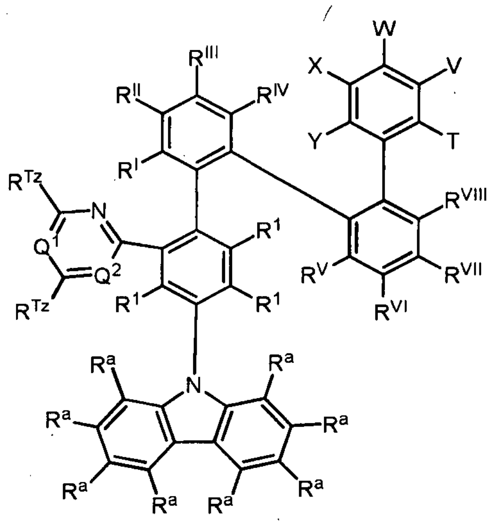

Formula IX

wherein any one of the aforementioned definitions apply.

In one embodiment of the invention, the organic molecules comprise or consist of a structure of formula IX-2:

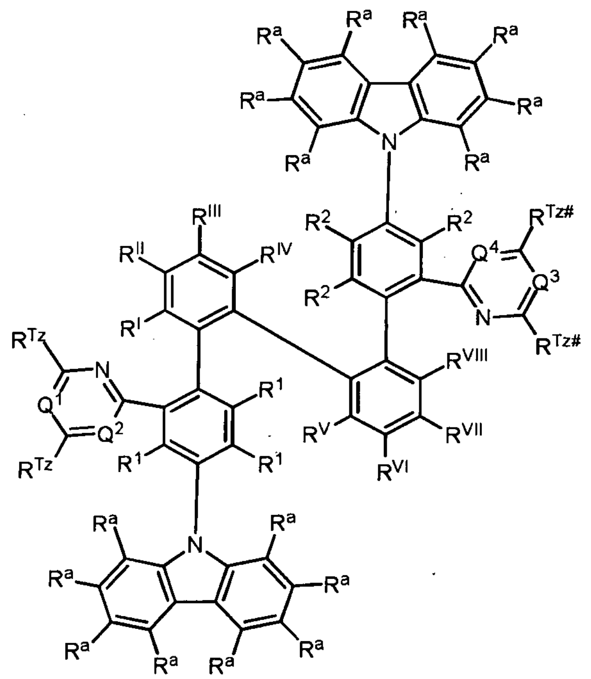

Formula IX-2

wherein any one of the aforementioned definitions apply.

In one embodiment of the invention, the organic molecules comprise or consist of a structure of formula X:

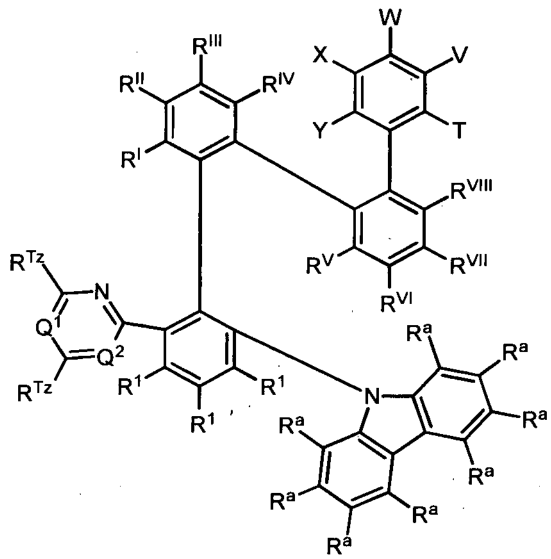

Formula X

wherein any one of the aforementioned definitions apply.

In one embodiment of the invention, the organic molecules comprise or consist of a structure of formula X-2:

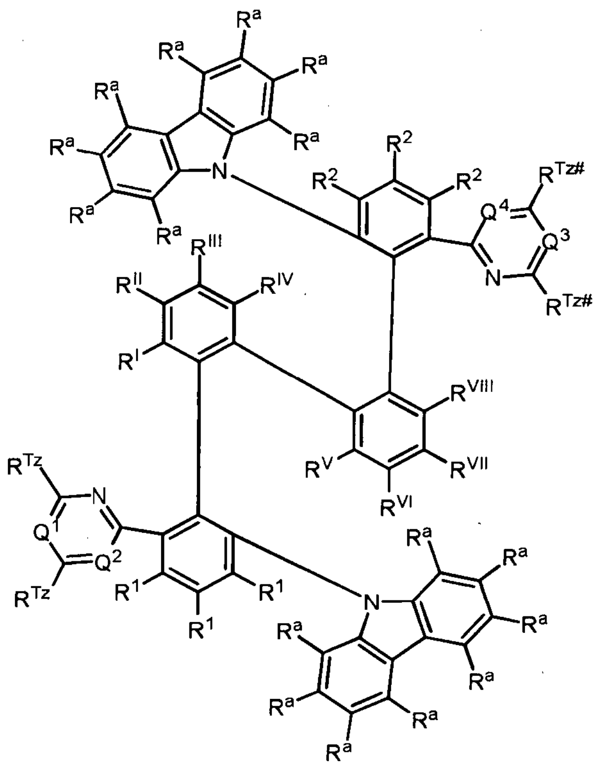

Formula X-2

wherein any one of the aforementioned definitions apply.

In one embodiment of the invention, the organic molecules comprise or consist of a structure of formula XI:

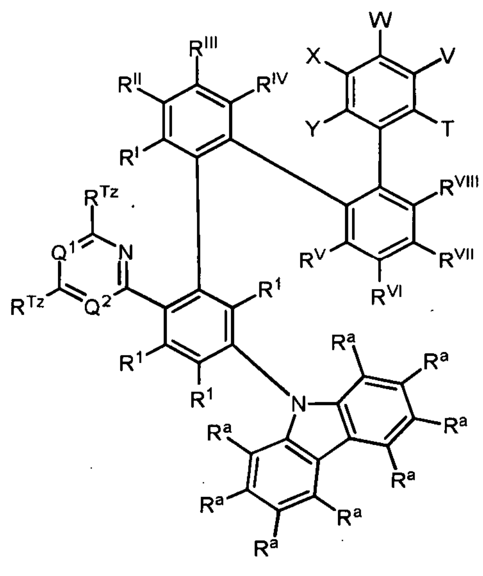

Formula XI

wherein any one of the aforementioned definitions apply.

In one embodiment of the invention, the organic molecules comprise or consist of a structure of formula XI-2:

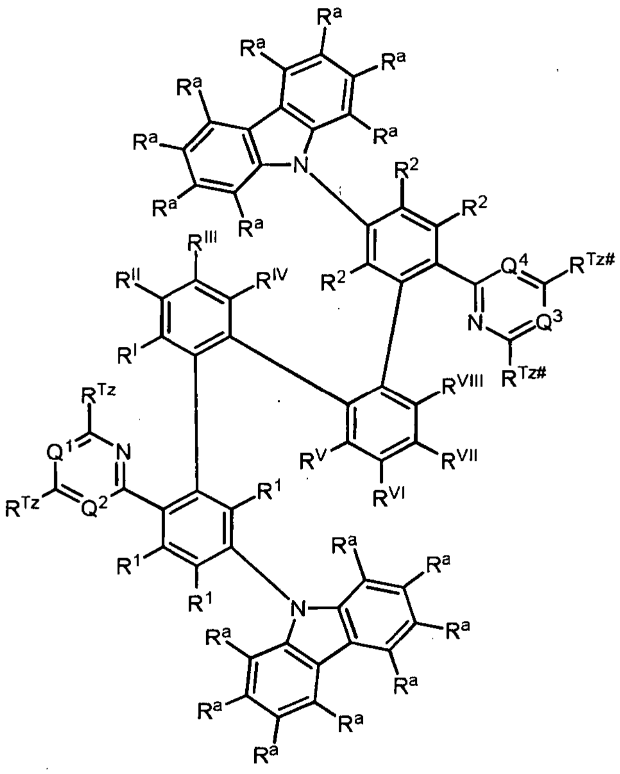

Formula XI-2

wherein any one of the aforementioned definitions apply.

In one embodiment of the invention, the organic molecules comprise or consist of a structure of formula XII:

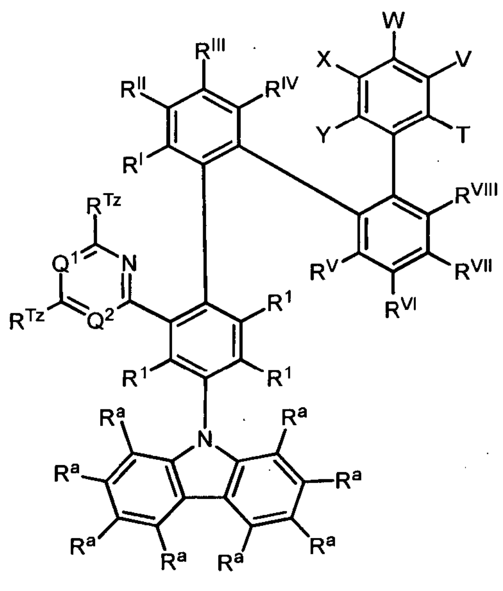

Formula XII

wherein any one of the aforementioned definitions apply.

In one embodiment of the invention, the organic molecules comprise or consist of a structure of formula XII-2:

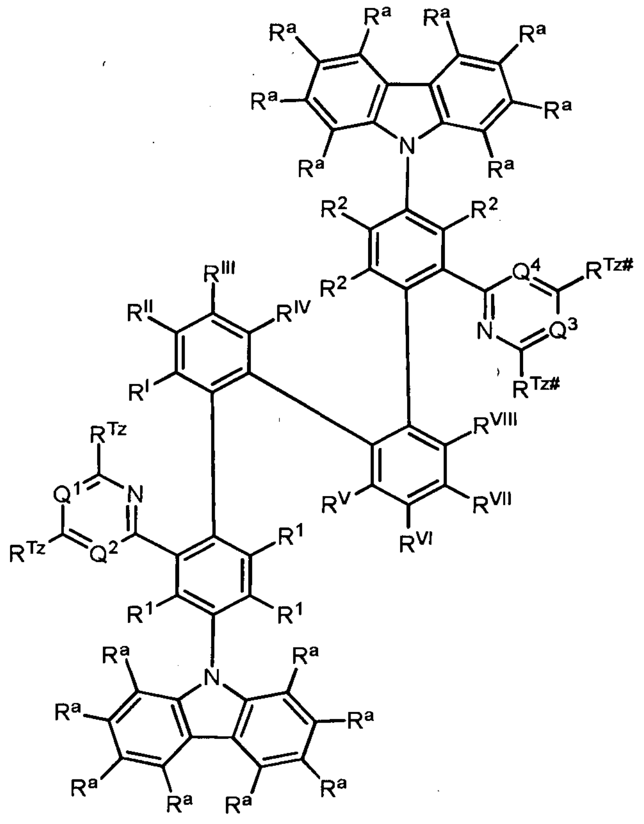

Formula XII-2

wherein any one of the aforementioned definitions apply.

As used throughout here, the terms "aryl" and "aromatic" may be understood in the broadest sense as any mono-, bi- or polycyclic aromatic moieties. Accordingly, an aryl group contains 6 to 60 aromatic ring atoms, and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case; a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used throughout, the term aryl group or heteroaryl group comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used throughout, the term cyclic group may be understood in the broadest sense as any mono-, bi- or polycyclic moieties.

As used throughout, the term biphenyl as a substituent may be understood in the broadest sense as ortho-biphenyl, meta-biphenyl, or para-biphenyl, wherein ortho, meta and para is defined in regard to the binding site to another chemical moiety.

As used throughout, the term alkyl group may be understood in the broadest sense as any linear, branched, or cyclic alkyl substituent. In particular, the term alkyl comprises the substituents methyl (Me), ethyl (Et), n-propyl ($^n$Pr), i-propyl ($^i$Pr), cyclopropyl, n-butyl ($^n$Bu), i-butyl ($^i$Bu), s-butyl ($^s$Bu), t-butyl ($^t$Bu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used throughout, the term alkenyl comprises linear, branched, and cyclic alkenyl substituents. The term alkenyl group, for example, comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout, the term alkynyl comprises linear, branched, and cyclic alkynyl substituents. The term alkynyl group exemplarily comprises ethynyl, propynyl, butynyl, pentynyl, hexynyl, heptynyl or octynyl.

As used throughout, the term alkoxy comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy and 2-methylbutoxy.

As used throughout, the term thioalkoxy comprises linear, branched, and cyclic thioalkoxy substituents, in which the O of the exemplarily alkoxy groups is replaced by S.

As used throughout, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphtyl, dibenzofuryl) or as if it were the whole molecule (e.g. naphthalene, dibenzofuran). As used herein, these different ways of designating a substituent or attached fragment are considered to be equivalent.

In one embodiment, the organic molecules according to the invention have an excited state lifetime of not more than 150 μs, of not more than 100 μs, in particular of not more than 50 μs, more preferably of not more than 10 μs or not more than 7 μs in a film of poly(methyl methacrylate) (PMMA) with 10 % by weight of organic molecule at room temperature.

In one embodiment of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In a further embodiment of the invention, the organic molecules according to the invention have an emission peak in the visible or nearest ultraviolet range, i.e., in the range of a wavelength of from 380 to 800 nm, with a full width at half maximum of less than 0.50 eV, preferably less than 0.48 eV, more preferably less than 0.45 eV, even more preferably less than 0.43 eV or even less than 0.40 eV in a film of poly(methyl methacrylate) (PMMA) with 10 % by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is calculated as $E^{HOMO} + E^{gap}$, wherein $E^{gap}$ is determined as follows: For host compounds, the onset of the emission spectrum of a film with 10 % by weight of host in poly(methyl methacrylate) (PMMA) is used as $E^{gap}$, unless stated otherwise. For emitter molecules, $E^{gap}$ is determined as the energy at which the excitation and emission spectra of a film with 10 % by weight of emitter in PMMA cross.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF.

The triplet energy can thus be determined as the onset of the phosphorescence spectrum. For TADF emitter molecules, the energy of the first excited triplet state T1 is determined from the onset of the delayed emission spectrum at 77 K, if not otherwise stated measured in a film of PMMA with 10 % by weight of emitter. Both for host and emitter compounds, the energy of the first excited singlet state S1 is determined from the onset of the emission spectrum, if not otherwise stated measured in a film of PMMA with 10 % by weight of host or emitter compound.

The onset of an emission spectrum is determined by computing the intersection of the tangent to the emission spectrum with the x-axis. The tangent to the emission spectrum is set at the high-energy side of the emission band and at the point at half maximum of the maximum intensity of the emission spectrum.

A further aspect of the invention relates to a process for preparing organic molecules (with an optional subsequent reaction) according to the invention, wherein a 2,2'-di-Hal$^a$-biphenyl **E3-3** is used as a reactant and Hal$^a$ is selected from the group consisting of Br and Cl :

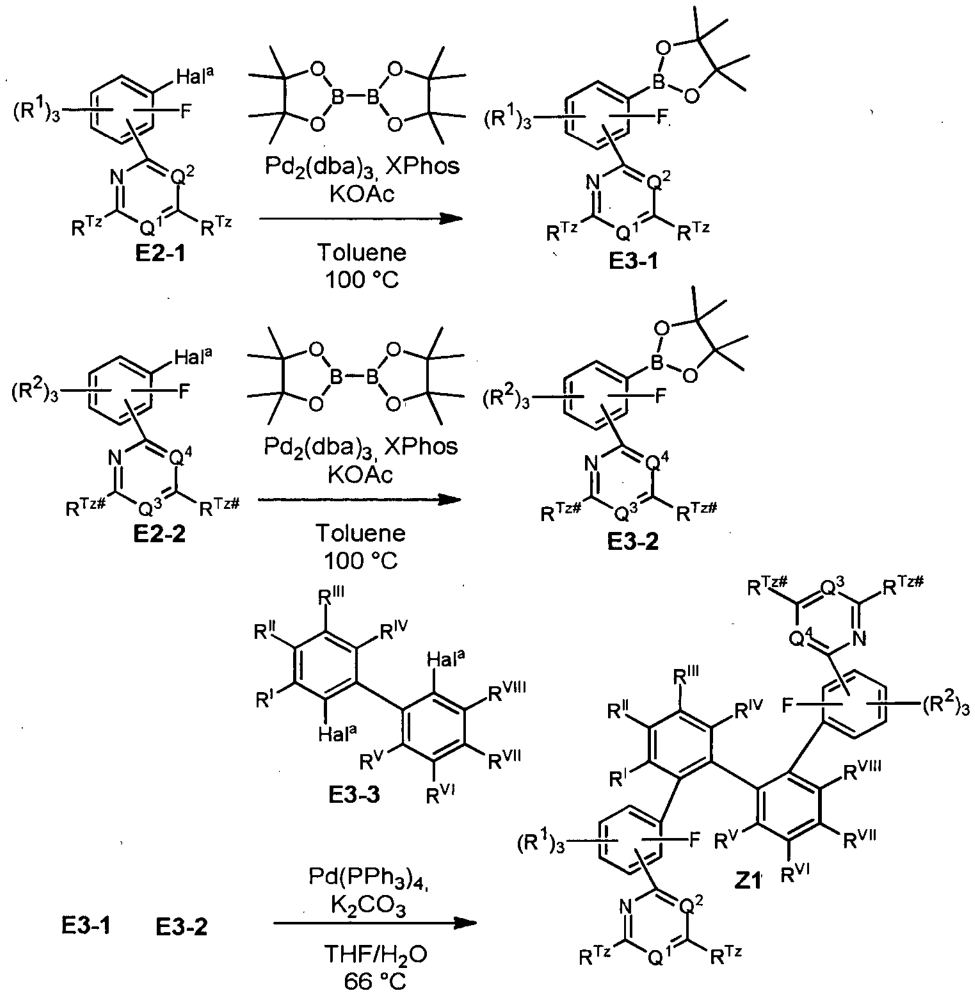

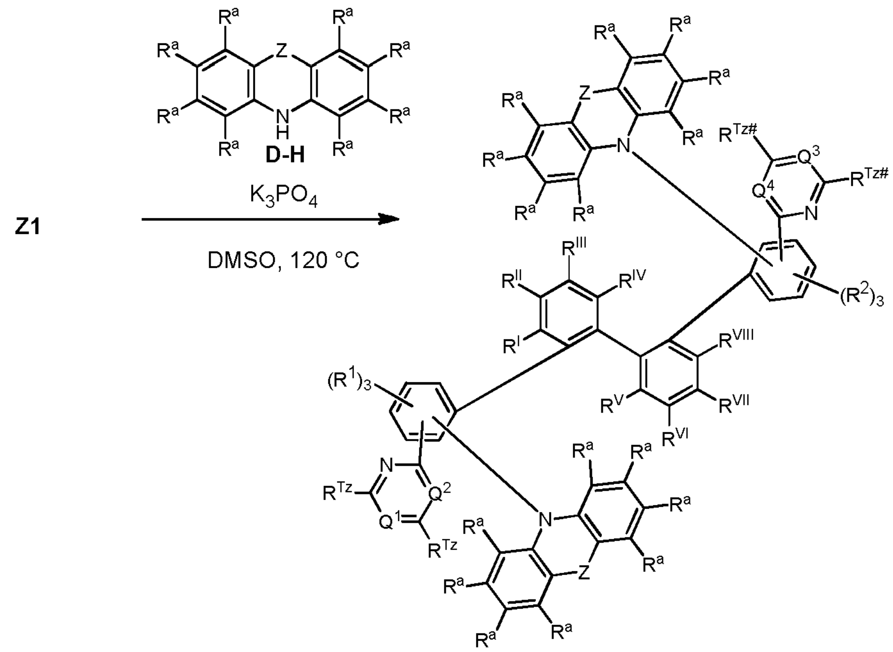


An alternative synthesis route comprises the introduction of a nitrogen heterocycle via copper- or palladium-catalyzed coupling to an aryl halide or aryl pseudohalide, preferably an aryl bromide, an aryl iodide, aryl triflate or an aryl tosylate.

A further aspect of the invention relates to the use of an organic molecule according to the invention as a luminescent emitter in an organic optoelectronic device.

The organic electroluminescent device may be understood in the broadest sense as any device based on organic materials that is suitable for emitting light in the visible or nearest ultraviolet (UV) range, i.e., in the range of a wavelength of from 380 to 800 nm. More preferably, organic electroluminescent device may be able to emit light in the visible range, i.e., of from 400 to 800 nm.

In the context of such use, the organic optoelectronic device is more particularly selected from the group consisting of:

- organic light-emitting diodes (OLEDs),
- light-emitting electrochemical cells,
- OLED sensors, especially in gas and vapor sensors not hermetically externally shielded,
- organic diodes,
- organic solar cells,
- organic transistors,
- organic field-effect transistors,
- organic lasers, and
- down-conversion elements.

In a preferred embodiment in the context of such use, the organic electroluminescent device is a device selected from the group consisting of an organic light emitting diode (OLED), a light emitting electrochemical cell (LEC), and a light-emitting transistor.

In the case of the use, the fraction of the organic molecule according to the invention in the emission layer in an organic optoelectronic device, more particularly in OLEDs, is 1 % to 99 % by weight, more particularly 5 % to 80 % by weight. In an alternative embodiment, the proportion of the organic molecule in the emission layer is 100 % by weight.

In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention in the form of an emitter, and
(b) one or more emitter and/or host materials, which differ from the organic molecule according to the invention and
(c) optional one or more dyes and/or one or more solvents.

In one embodiment, the light-emitting layer comprises or consists of a composition comprising or consisting of:

(a) at least one organic molecule according to the invention, in particular in the form of an emitter, and
(b) one or more emitter and/or host materials, which differ from the organic molecule according to the invention and
(c) optional one or more dyes and/or one or more solvents.

Particularly preferably the light-emitting layer EML comprises or consists of a composition comprising or consisting of:

(i) 1-50 % by weight, preferably 5-40 % by weight, in particular 10-30 % by weight, of one or more organic molecules according to the invention E;
(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention E, in particular transferred from the first excited triplet state T1(H) of the host compound H to the first excited triplet state T1(E) of the one or more organic molecules according to the invention E and/ or from the first excited singlet state S1(H) of the host compound H to the first excited singlet state S1(E) of the one or more organic molecules according to the invention E.

In a further embodiment, the light-emitting layer EML comprises (or (essentially) consists of) a composition comprising or consisting of:

(i) 1-50 % by weight, preferably 5-40 % by weight, in particular 10-30 % by weight, of one organic molecule according to the invention E;
(ii) 5-99 % by weight, preferably 30-94.9 % by weight, in particular 40-89% by weight, of one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy $E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.

In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.

In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention E has a highest occupied molecular orbital HOMO(E) having an energy $E^{HOMO}(E)$ and a lowest unoccupied molecular orbital LUMO(E) having an energy $E^{LUMO}(E)$,
wherein
$E^{HOMO}(H) > E^{HOMO}(D)$ and the difference between the energy level of the highest occupied molecular orbital HOMO(E) of the organic molecule according to the invention E ($E^{HOMO}(E)$) and the energy level of the highest occupied molecular orbital HOMO(H) of the host compound H ($E^{HOmO}(H)$) is between -0.5 eV and 0.5 eV, more preferably between -0.3 eV and 0.3 eV, even more preferably between -0.2 eV and 0.2 eV or even between -0.1 eV and 0.1 eV; and $E^{LUMO}(H) > E^{LUMO}(D)$ and the difference between the energy level of the lowest unoccupied molecular orbital LUMO(E) of the organic molecule according to the invention E ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between

-0.1 eV and 0.1 eV.

In a further aspect, the invention relates to an organic optoelectronic device comprising an organic molecule or a composition of the type described here, more particularly in the form of a device selected from the group consisting of organic light-emitting diode (OLED), light-emitting electrochemical cell, OLED sensor, more particularly gas and vapour sensors not hermetically externally shielded, organic diode, organic solar cell, organic transistor, organic field-effect transistor, organic laser and down-conversion element.

In a preferred embodiment, the organic electroluminescent device is a device selected from the group consisting of an organic light emitting diode (OLED), a light emitting electrochemical cell (LEC), and a light-emitting transistor.

In one embodiment of the organic optoelectronic device of the invention, the organic molecule according to the invention E is used as emission material in a light-emitting layer EML.

In one embodiment of the organic optoelectronic device of the invention the light-emitting layer EML consists of the composition according to the invention described here.

For example, when the organic electroluminescent device is an OLED, it may exhibit the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL
10. cathode layer,

wherein the OLED comprises each layer only optionally, different layers may be merged and the OLED may comprise more than one layer of each layer type defined above.

Furthermore, the organic electroluminescent device may optionally comprise one or more protective layers protecting the device from damaging exposure to harmful species in the environment including, exemplarily moisture, vapor and/or gases.

In one embodiment of the invention, the organic electroluminescent device is an OLED, which exhibits the following inverted layer structure:

1. substrate
2. cathode layer
3. electron injection layer, EIL
4. electron transport layer, ETL
5. hole blocking layer, HBL
6. emitting layer, B
7. electron blocking layer, EBL
8. hole transport layer, HTL
9. hole injection layer, HIL
10. anode layer A

Wherein the OLED with an inverted layer structure comprises each layer only optionally, different layers may be merged and the OLED may comprise more than one layer of each layer types defined above.

In one embodiment of the invention, the organic electroluminescent device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the organic electroluminescent device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers

between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may exemplarily comprise indium tin oxide, aluminum zinc oxide, fluorine doped tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene.

Particularly preferably, the anode layer A (essentially) consists of indium tin oxide (ITO) (e.g., $(InO_3)0.9(SnO_2)0.1$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may, for example, comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bisphenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may exemplarily be used as inorganic dopant. Tetrafluorotetracyanoquinodimethane ($F_4$-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particularly, the EML comprises at least one light emitting molecule according to the invention E. In one embodiment, the light-emitting layer comprises only the organic molecules according to the invention E. Typically, the EML additionally comprises one or more host materials H. Exemplarily, the host material H is selected from CBP (4,4'-Bis-(N-carbazolyl)-biphenyl), mCP, mCBP Sif87 (dibenzo[b,d]thiophen-2-yltriphenylsilane), CzSi, Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material H typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule according to the invention E and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. Exemplarily, electron-poor compounds such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may exemplarily comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), $Alq_3$ (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. For example, the cathode layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) intransparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may optionally further, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds H.

In order to modify the emission spectrum and/or the absorption spectrum of the light-emitting layer EML further, the light-emitting layer EML may further comprise one or more further emitter molecules F. Such an emitter molecule F may be any emitter molecule known in the art. Preferably such an emitter molecule F is a molecule with a structure differing from the structure of the molecules according to the invention E. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. For example, the triplet and/or singlet excitons may be transferred from the emitter molecule according to the invention E to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an organic electroluminescent device (e.g., an OLED) may exemplarily be an essentially white organic electroluminescent device. For example, such white organic electroluminescent device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm. |

With respect to emitter molecules, such colors refer to the emission maximum. Therefore, exemplarily, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 cd/$m^2$ of more than 8 %, more preferably of more than 10 %, more preferably of more than 13 %, even more preferably of more than 15 % or even more than 20 % and/or exhibits an emission maximum between 420 nm and 500 nm, preferably between 430 nm and 490 nm, more preferably between 440 nm and 480 nm, even more preferably between 450 nm and 470 nm and/or exhibits a LT80 value at 500 cd/$m^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.50 eV, preferably less than 0.45 eV, more preferably less than 0.40 eV, even more preferably less than 0.38 eV or even less than 0.36 eV.

A further aspect of the present invention relates to an OLED, which emits light with CIEx and CIEy color coordinates close to the CIEx (= 0.131) and CIEy (= 0.046) color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. Accordingly, a further. aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The organic electroluminescent device, in particular the OLED according to the present invention can be fabricated by any means of vapor deposition and/ or liquid processing. Accordingly, at least one layer is

- prepared by means of a sublimation process,
- prepared by means of an organic vapor phase deposition process,
- prepared by means of a carrier gas sublimation process,
- solution processed or printed.

The methods used to fabricate the organic electroluminescent device, in particular the OLED according to the present invention are known in the art. The different layers are individually and successively deposited on a suitable substrate by means of subsequent deposition processes. The individual layers may be deposited using the same or differing deposition methods.

Vapor deposition processes, for example, comprise thermal (co)evaporation, chemical vapor deposition and physical vapor deposition. For active matrix OLED display, an AMOLED backplane is used as substrate. The individual layer may be processed from solutions or dispersions employing adequate solvents. A solution deposition process, for example, may comprise spin coating, dip coating and jet printing. Liquid processing may optionally be carried out in an inert atmosphere (e.g., in a nitrogen atmosphere) and the solvent may optionally be completely or partially removed by means known in the state of the art.

## Examples

General synthesis scheme I

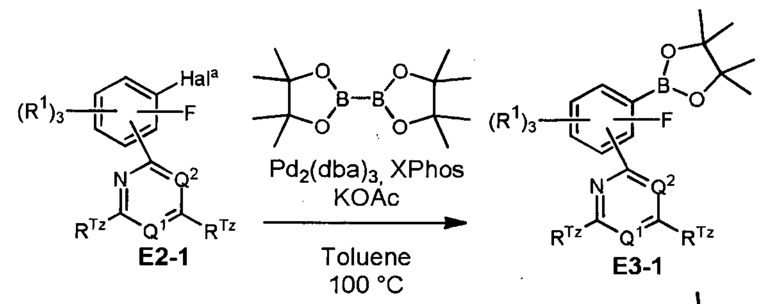
E2-1
$Pd_2(dba)_3$, XPhos
KOAc
Toluene
100 °C
E3-1

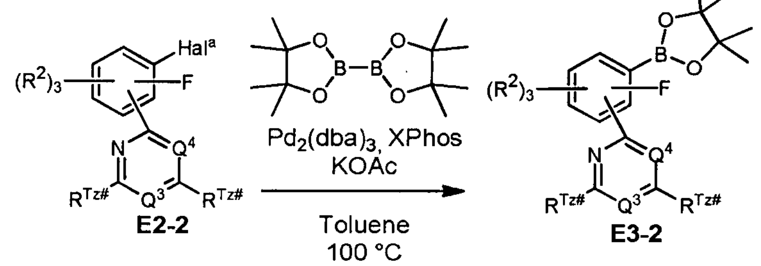
E2-2
$Pd_2(dba)_3$, XPhos
KOAc
Toluene
100 °C
E3-2

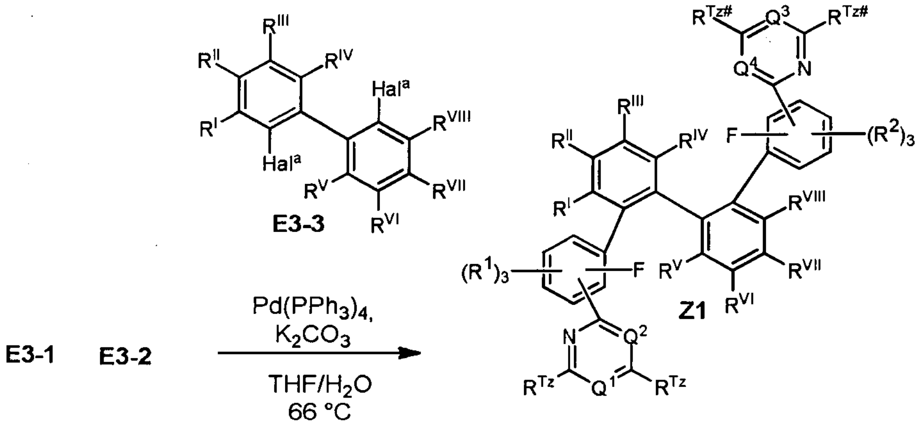
E3-3
E3-1
E3-2
$Pd(PPh_3)_4$,
$K_2CO_3$
$THF/H_2O$
66 °C
Z1

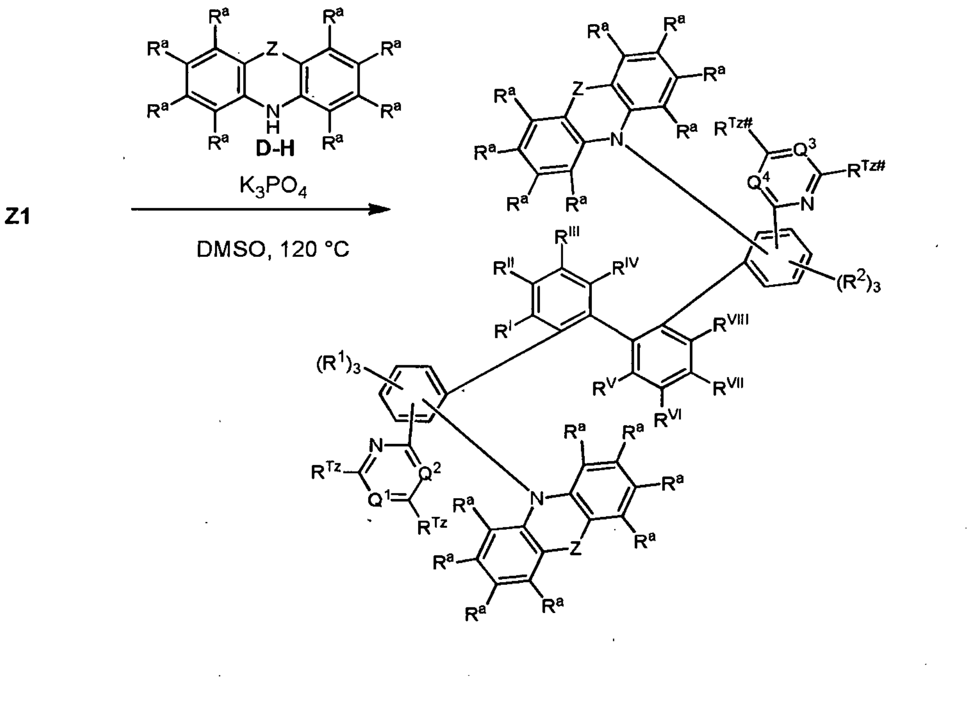
D-H
$K_3PO_4$
Z1
DMSO, 120 °C

*General procedure* ***for*** *synthesis* ***AAV0***:

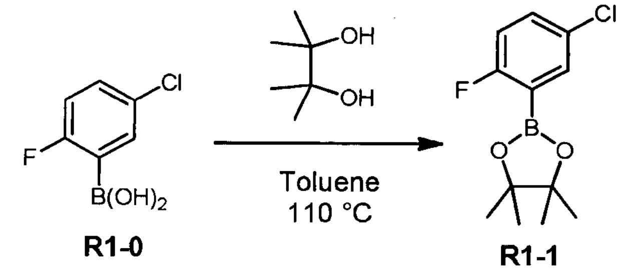


**R1-0** (1.00 equivalents, CAS: 352535-83-2), pinacol (2.4 equivalents, CAS: 76-09-5) are stirred under nitrogen atmosphere in a dry toluene at reflux for 2 h. After that time reaction mixture is cooled down to room temperature and filtrated through silica gel. Solvent is removed completely and dried under vacuum overnight.

*General procedure for synthesis* ***AAV0-1***:

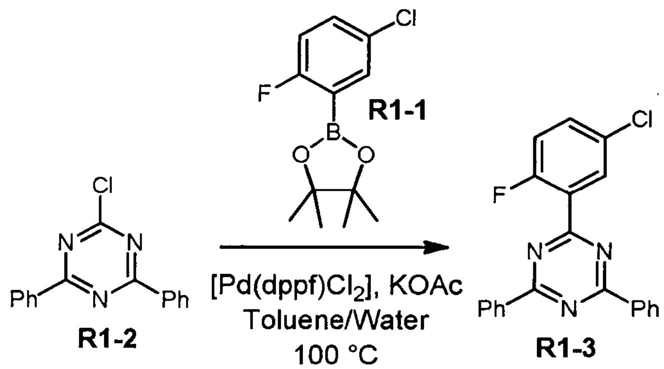


**R1-2** (1.30 equivalents), R1-1 (1.00 equivalents, CAS: 3842-55-5), [Pd(dppf)$Cl_2$] (0.05 equivalent, CAS: 72287-26-4) and potassium acetate (3.00 equivalents) are stirred under nitrogen atmosphere in a toluene/water mixture (ratio of 10:1) at reflux for 20 h. After that time reaction mixture is cooled down to room temperature and extracted with dichloromethane/brine. Organic phase is collected, washed with brine and dried over $MgSO_4$. The organic solvent is removed and crude product is purified by hot wash with EtOH.

*General procedure for synthesis* ***AAV1-1***:

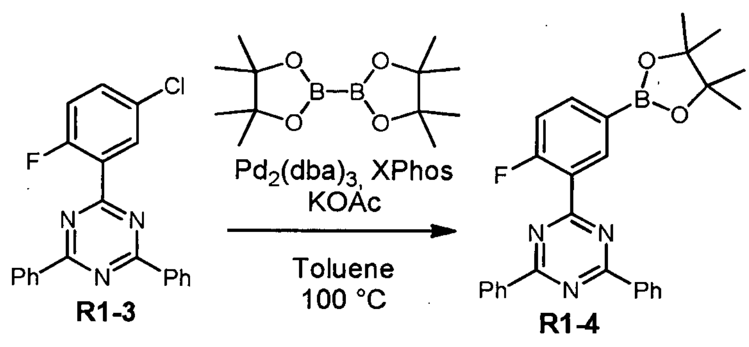


**R1-3** (1.00 equivalents), bis(pinacolato)diboron (1.30 equivalents, CAS: 73183-34-3), Tris(dibenzylideneacetone)dipalladium(0) (0.01 equivalent, CAS: 51364-51-3), X-Phos (0.04 equivalents, CAS: 564483-18-7) and potassium acetate (3.00 equivalents) are stirred under nitrogen atmosphere in a dry toluene at 100°C for 4 h. After that time reaction mixture is cooled down to room temperature and extracted with dichloromethane/brine. Organic phase is collected, washed with brine and dried over $MgSO_4$. The organic solvent is removed and crude product is purified by hot wash with EtOH.

*General procedure for synthesis **AAV1-2**:*

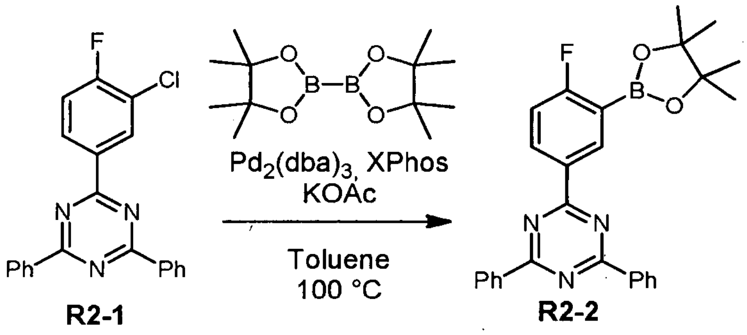


**R2-1** (1.00 equivalents), bis(pinacolato)diboron (1.50 equivalents, CAS: 73183-34-3), $Pd_2(dba)_3$ (0.04 equivalent, CAS: 51364-51-3), X-Phos (0.08 equivalents, CAS: 564483-18-7) and potassium acetate (3.00 equivalents) are stirred under nitrogen atmosphere in dry toluene at 100°C for 16 h. After that time reaction mixture is cooled down to room temperature and extracted with ethyl acetate/brine. The organic phase is collected, washed with brine and dried over $MgSO_4$. The organic solvent is removed and crude product is washed with cyclohexane and re-crystalized from EtOH.

*General procedure for synthesis **AAV2-1**:*

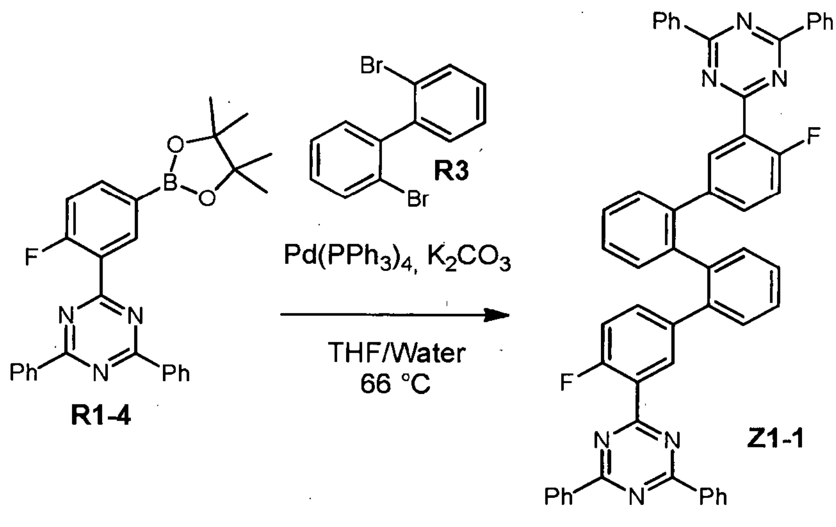


**R3** (1.00 equivalents, CAS: 13029-09-9), **R1-4** (2.00 equivalents), $Pd(PPh_3)_4$ (0.05 equivalent, CAS: 14221-01-3) and potassium carbonate (2.50 equivalents) are stirred under nitrogen atmosphere in a THF/water mixture (ratio of 30:2) at 66°C for 20 h. After that time reaction mixture is cooled down to room temperature and extracted with dichloromethane/brine. Organic phase is collected, washed with brine and dried over $MgSO_4$. The organic solvent is removed and crude product is purified by column chromatography (silica gel, eluent cyclohexane:dichloromethane 3:1).

*General procedure for synthesis **AAV2-2**:*

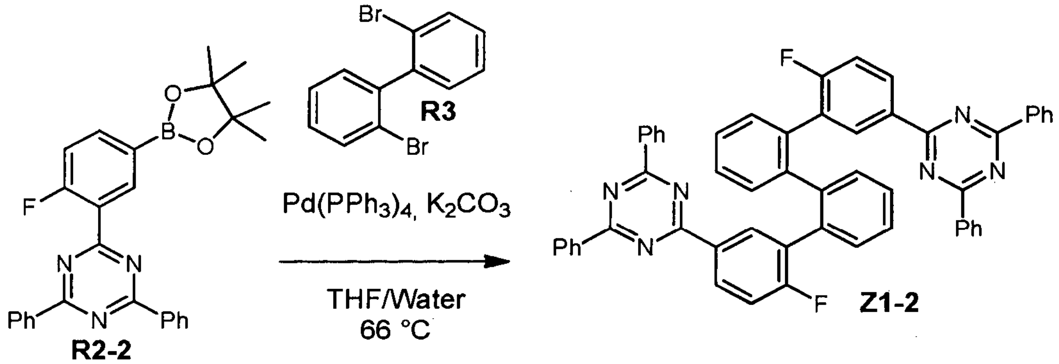


**R2-2** (2.00 equivalents), **R3** (1.00 equivalents, CAS: 13029-09-9), $Pd(PPh_3)_4$ (0.05 equivalent, CAS: 14221-04-3) and potassium carbonate (2.50 equivalents) are stirred under nitrogen atmosphere in THF/water mixture (ratio of 30:2) at 66°C for 20 h. After that time reaction mixture is cooled down to room temperature and extracted with ethyl acetate/brine. Organic phase is collected, washed with brine and dried over $MgSO_4$. The organic solvent is removed and crude product purified by column chromatography (silica gel, eluent cyclohexane:dichloromethane 3:1).

*General procedure for synthesis **AAV3-1**:*

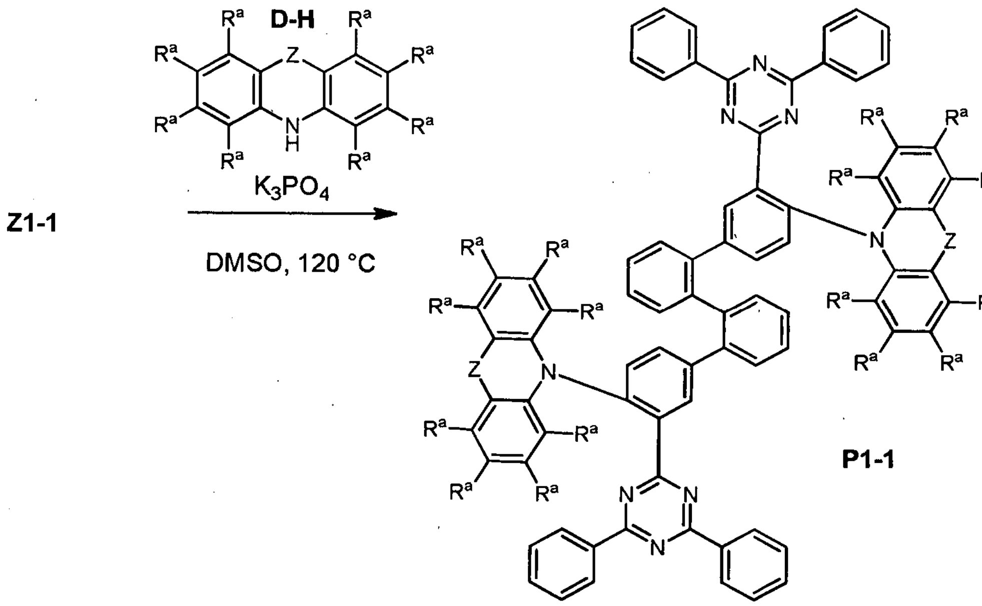


**Z1-1** (1.00 equivalent), **D-H** (2.05 equivalents) and potassium phosphate (6.00 equivalents) are stirred under nitrogen atmosphere in a dry DMSO at 120°C for 24 h. After that time reaction mixture is cooled down to room temperature and poured into water. The product is filtered off and washed with EtOH/EE (ratio of 10:1). Additionally, the product is re-crystallized from EtOH/n-hexane/DCM (ratio of 10:5:2), yielding **P1-1** as a solid.

*General procedure for synthesis **AAV3-2**:*

Z1-2

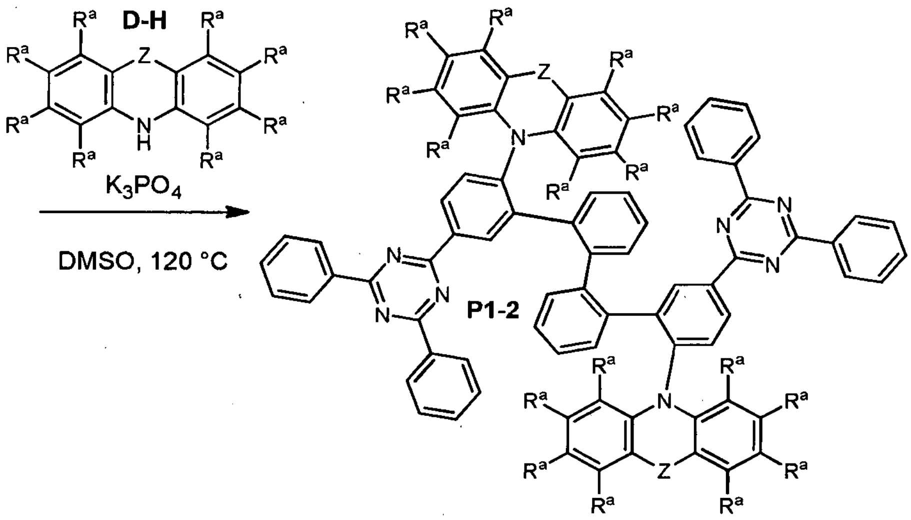


**Z1-2** (1.00 equivalents), **D-H** (2.10 equivalents) and potassium phosphate (6.00 equivalents) are stirred under nitrogen atmosphere in a dry DMSO at 120°C for 24 h. After that time reaction mixture is cooled down to room temperature and extracted with dichloromethane/brine. Organic phase is collected, washed with brine and dried over $MgSO_4$. The product is re-crystallized from cyclohexane/n-hexane/DCM (ratio of 3:3:1), yielding **P1-2** as a solid.

In particular, the donor molecule D-H is a 3,6-substituted carbazole (e.g., 3,6-dimethylcarbazole, 3,6-diphenylcarbazole, 3,6-di-tert-butylcarbazole), a 2,7-substituted carbazole (e.g., 2,7-dimethylcarbazole, 2,7-diphenylcarbazole, 2,7-di-tert-butylcarbazole), a 1,8-substituted carbazole (e.g., 1,8-dimethylcarbazole, 1,8-diphenylcarbazole, 1,8-di-tert-butylcarbazole), a 1-substituted carbazole (e.g., 1-methylcarbazole, 1-phenylcarbazole, 1-tert-butylcarbazole), a 2-substituted carbazole (e.g., 2-methylcarbazole, 2-phenylcarbazole, 2-tert-butylcarbazole), or a 3-substituted carbazole (e.g., 3-methylcarbazole, 3-phenylcarbazole, 3-tert-butylcarbazole).

For example, a halogen-substituted carbazole, particularly 3-bromocarbazole, can be used as D-H.

In a subsequent reaction, a boronic acid ester functional group or boronic acid functional group may, for example, be introduced at the position of the one or more halogen substituents, which was introduced via D-H, to yield the corresponding carbazol-3-ylboronic acid ester or carbazol-3-ylboronic acid, e.g., via the reaction with bis(pinacolato)diboron (CAS No. 73183-34-3). Subsequently, one or more substituents $R^a$ may be introduced in place of the boronic acid ester group or the boronic acid group via a coupling reaction with the corresponding halogenated reactant $R^a$-Hal, preferably $R^a$-Cl and $R^a$-Br.

Alternatively, one or more substituents $R^a$ may be introduced at the position of the one or more halogen substituents, which was introduced via D-H, via the reaction with a boronic acid of the substituent $R^a$ [$R^a$-$B(OH)_2$] or a corresponding boronic acid ester.

*Cyclic voltammetry*

Cyclic voltammograms are measured from solutions having concentration of $10^{-3}$ mol/L of the organic molecules in dichloromethane or a suitable solvent and a suitable supporting electrolyte (e.g. 0.1 mol/L of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against a saturated calomel electrode (SCE).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.
The sample concentration is 10 mg/ml, dissolved in a suitable solvent.
Program: 1) 3 s at 400 U/min; 20 s at 1000 U/min at 1000 Upm/s. 3) 10 s at 4000 U/min at 1000 Upm/s. After coating, the films are dried at 70 °C for 1 min.

Photoluminescence spectroscopy and TCSPC (*Time-correlated single-photon counting)* Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.
Excited state lifetimes are determined employing the same system using the TCSPC method with FM-2013 equipment and a Horiba Yvon TCSPC hub.
Excitation sources:

NanoLED 370 (wavelength: 371 nm, puls duration: 1,1 ns)
NanoLED 290 (wavelength: 294 nm, puls duration: <1 ns)
SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Photoluminescence quantum yield measurements

For photoluminescence quantum yield (PLQY) measurements an *Absolute PL Quantum Yield Measurement C9920-03G* system (*Hamamatsu Photonics*) is used. Quantum yields and CIE coordinates are determined using the software U6039-05 version 3.6.0.
Emission maxima are given in nm, quantum yields $\phi$ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the organic molecule is excited using this wavelength
3) Measurement

Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

**Production and characterization of organic electroluminescence devices**

OLED devices comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100 %.
The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc.

Accelerated lifetime measurements are performed (e.g. applying increased current densities). For example, LT80 values at 500 cd/m$^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.

The values correspond to the average of several pixels (typically two to eight), the standard deviation between these pixels is given.

**HPLC-MS**

HPLC-MS analysis is performed on an HPLC by Agilent (1100 series) with MS-detector (Thermo LTQ XL).

For example, a typical HPLC method is as follows: a reverse phase column 4.6 mm × 150 mm, particle size 3.5 µm from Agilent *(ZORBAX Eclipse Plus 95Å C18, 4.6 × 150 mm,* 3.5 *µm HPLC column)* is used in the HPLC. The HPLC-MS measurements are performed at room temperature (rt) following gradients.

| Flow rate [ml/min] | time [min] | A[%] | B[%] | C[%] |
|---|---|---|---|---|
| 2.5 | 0 | 40 | 50 | 10 |
| 2.5 | 5 | 40 | 50 | 10 |
| 2.5 | 25 | 10 | 20 | 70 |
| 2.5 | 35 | 10 | 20 | 70 |
| 2.5 | 35.01 | 40 | 50 | 10 |
| 2.5 | 40.01 | 40 | 50 | 10 |
| 2.5 | 41.01 | 40 | 50 | 10 |

using the following solvent mixtures:

| | | |
|---|---|---|
| Solvent A: | $H_2O$ (90%) | MeCN (10%) |
| Solvent B: | $H_2O$ (10%) | MeCN (90%) |
| Solvent C: | THF (50%) | MeCN (50%) |

An injection volume of 5 µL from a solution with a concentration of 0.5 mg/mL of the analyte is taken for the measurements.

Ionization of the probe is performed using an APCI (atmospheric pressure chemical ionization) source either in positive (APCI +) or negative (APCI -) ionization mode.

**Example 1**

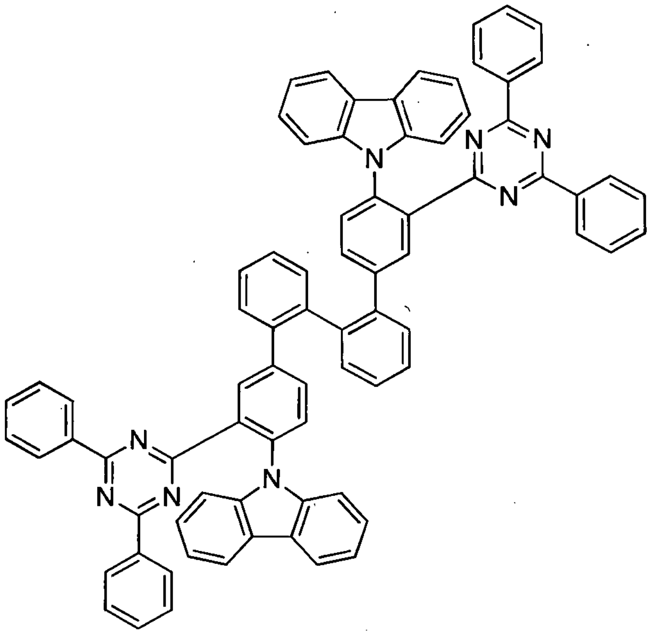

Example 1 was synthesized according to

***AAV0*** *(94%),*
***AAV0-1*** (84%),
***AAV1-1*** (95%),
***AAV2-1*** (12%), and
***AAV3-1*** (70%), wherein carbazole (CAS 86-74-8) was used as reactant.

HPLC-MS: exp.(neg. ionization): 25.68 min, 1098.48 m/z (100 %).

Figure 1 depicts the emission spectrum of example **1** (10 % by weight in PMMA). The emission maximum ($\lambda_{max}$) is at 463 nm. The photoluminescence quantum yield (PLQY) is 49 %, the full width at half maximum (FWHM) is 0.39 eV. The resulting $CIE_x$ coordinate is determined at 0.15 and the $CIE_y$ coordinate at 0.18.

**Example 2**

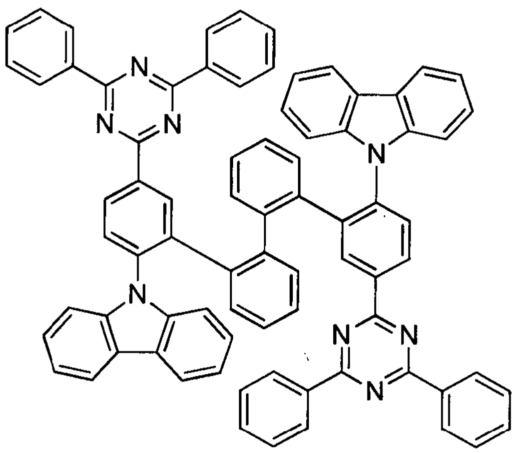

Example 2 was synthesized according to

***AAV1-2*** (85%),

***AAV2-2*** (10%), and
***AAV3-2*** (90%), wherein carbazole (CAS 86-74-8) was used as reactant.

HPLC-MS: exp.(neg. ionization): 30.80 min, 1098.41 m/z (100 %).
Figure 2 depicts the emission spectrum of example 2 (10 % by weight in PMMA). The emission maximum ($\lambda_{max}$) is at 443 nm. The photoluminescence quantum yield (PLQY) is 75 %, the full width at half maximum (FWHM) is 0.35 eV. The resulting $CIE_x$ coordinate is determined at 0.15 and the $CIE_y$ coordinate at 0.08.

**Additional Examples of Organic Molecules of the Invention**

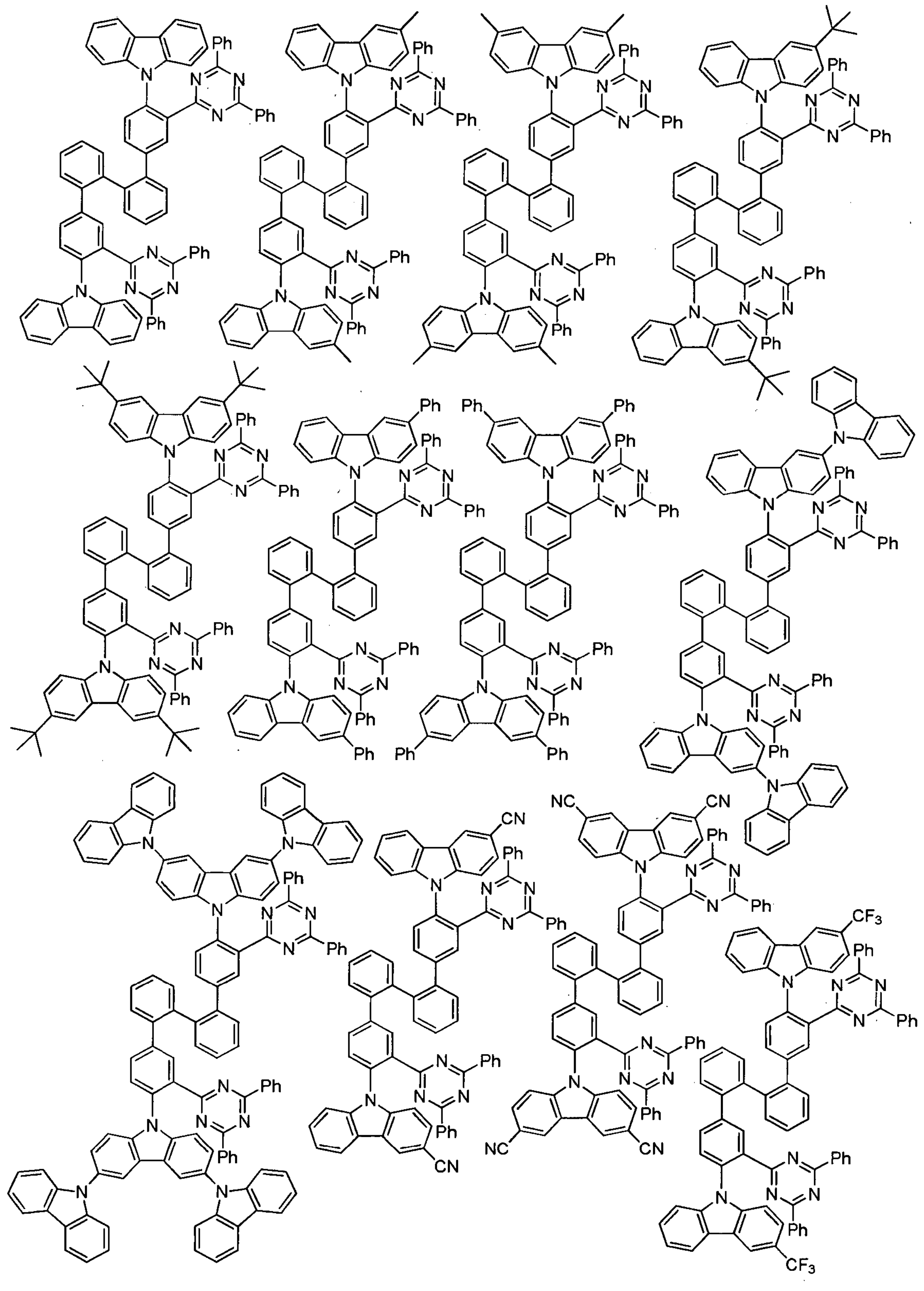

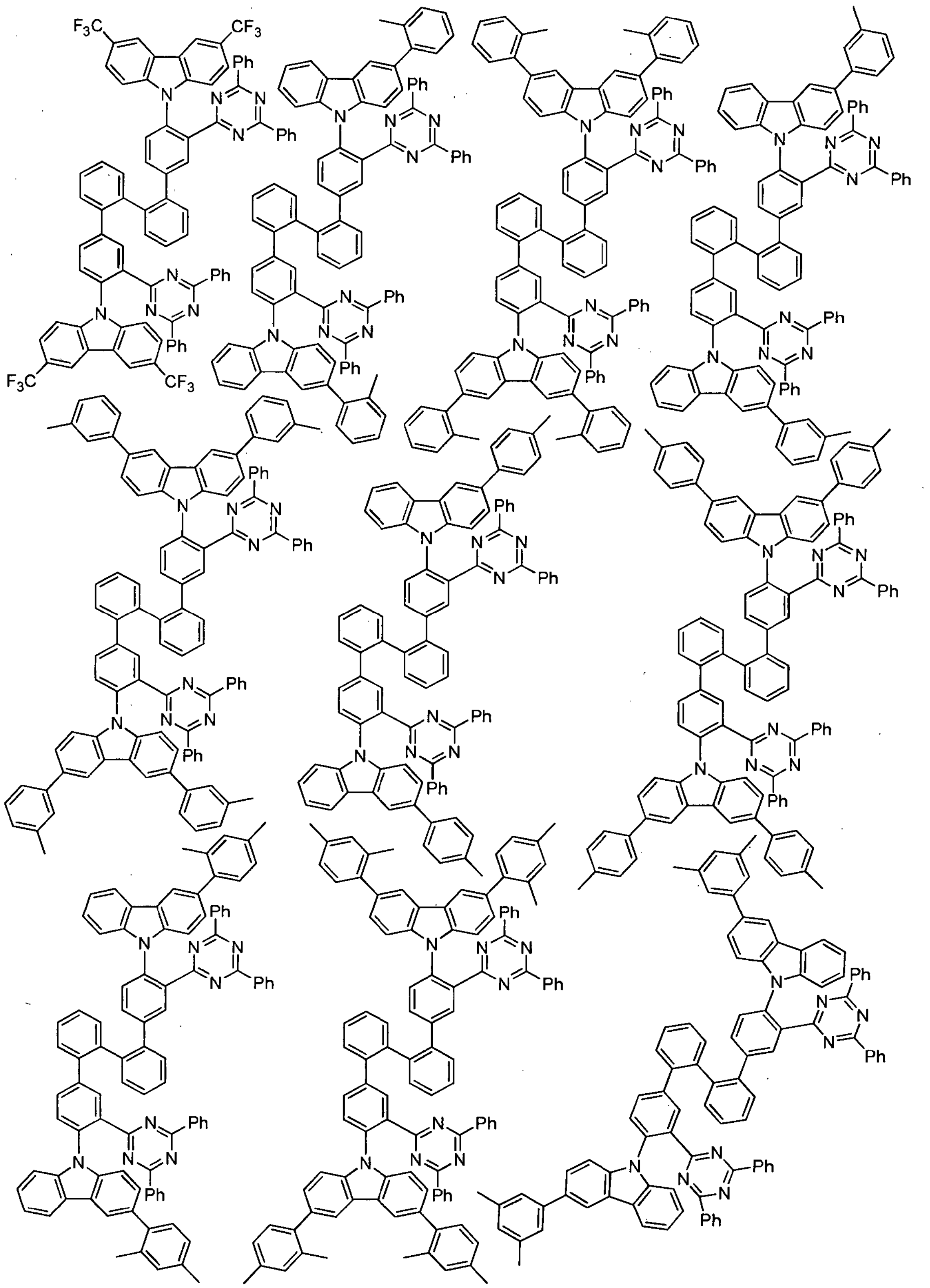

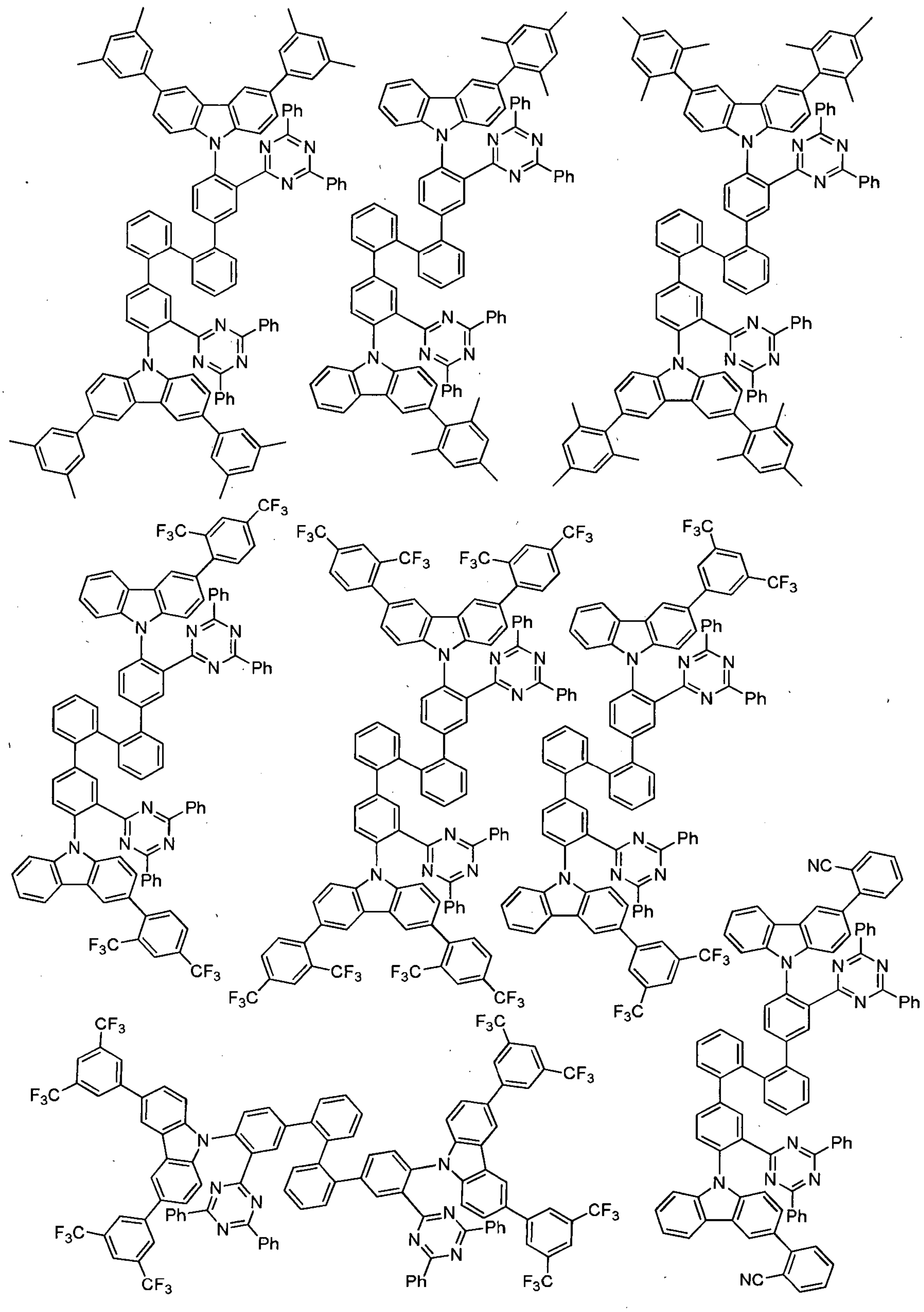

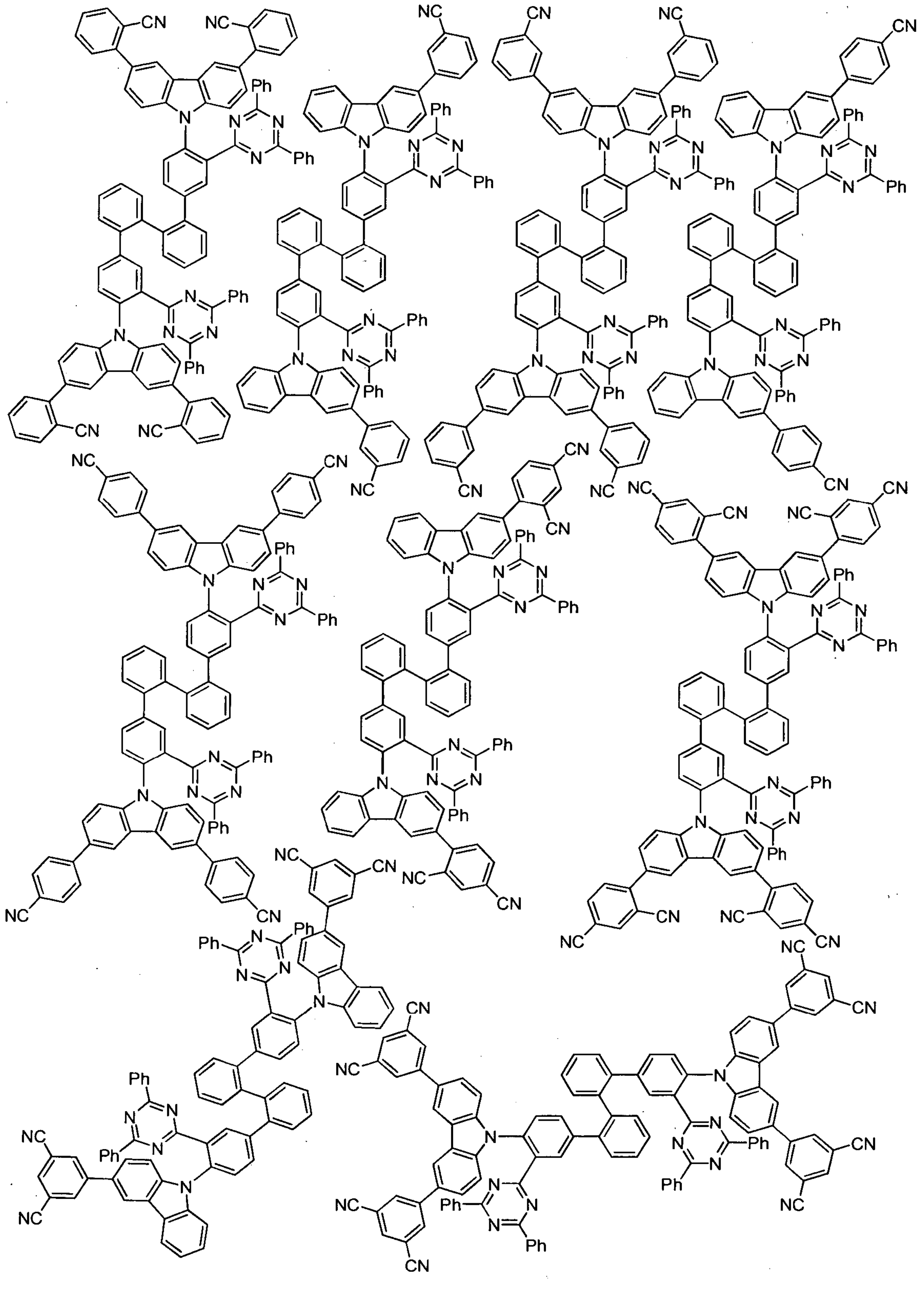

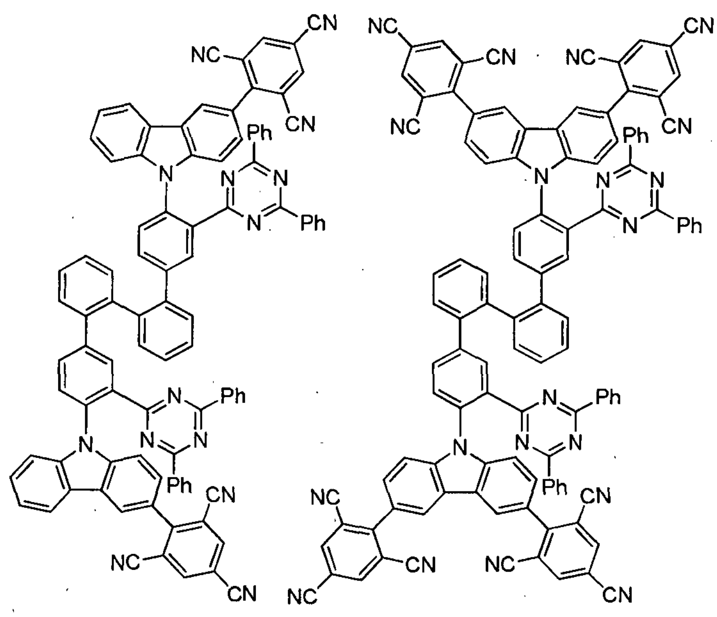

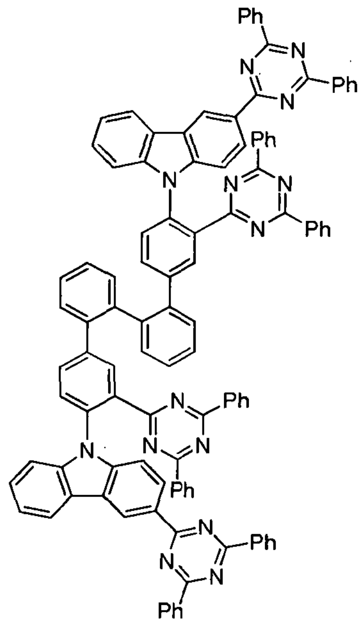

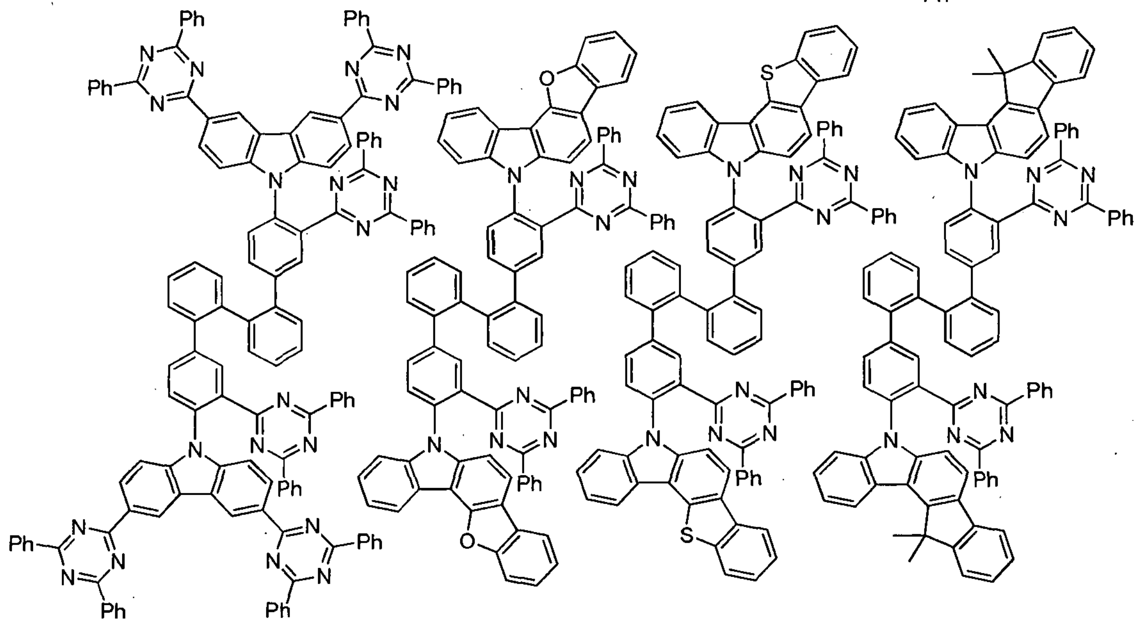

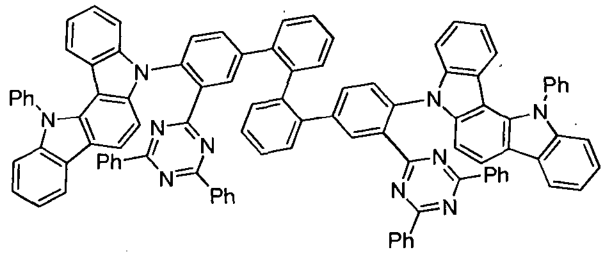

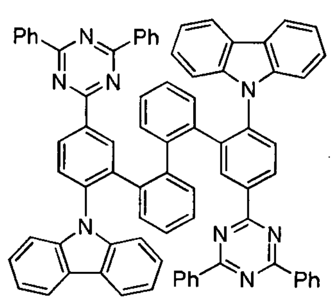

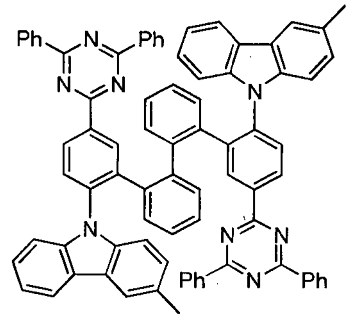

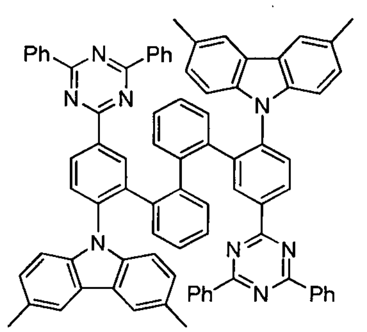

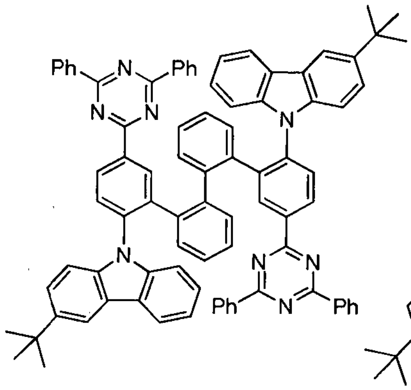

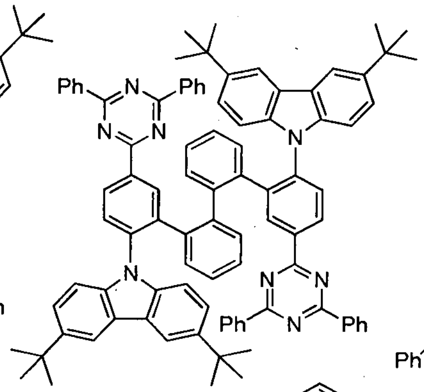

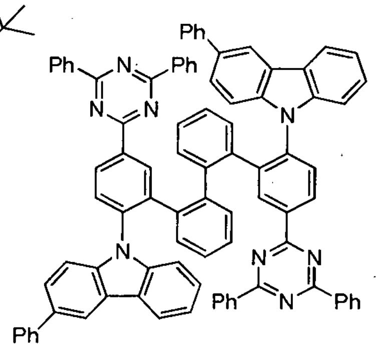

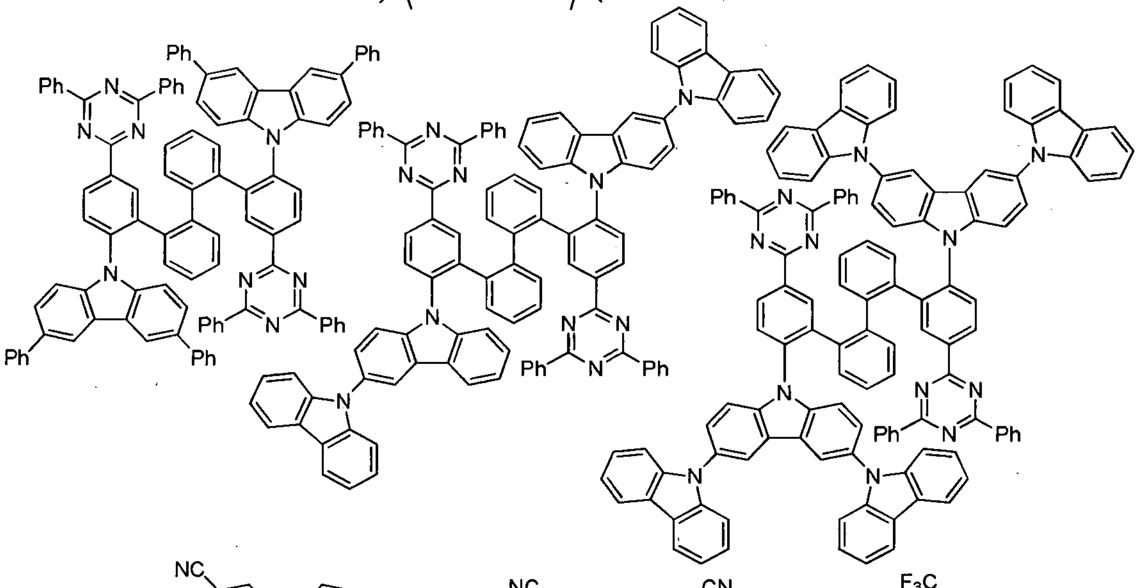

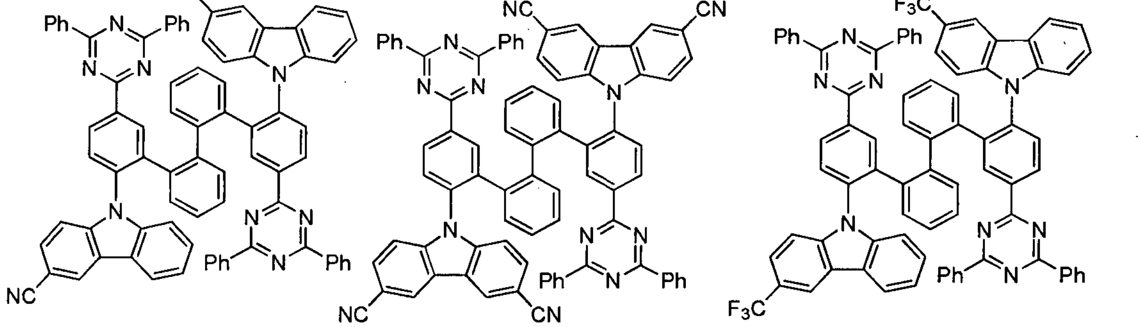

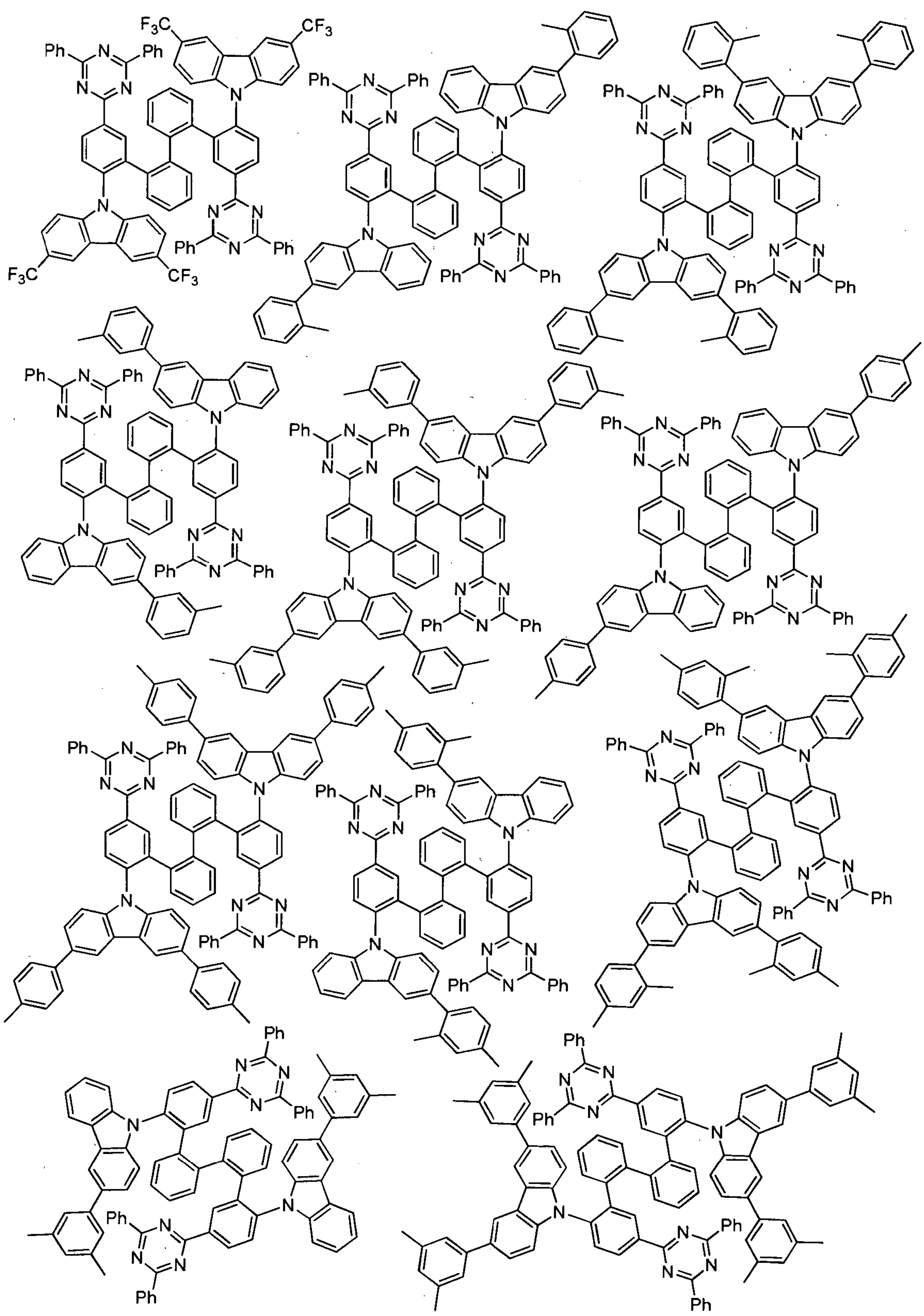

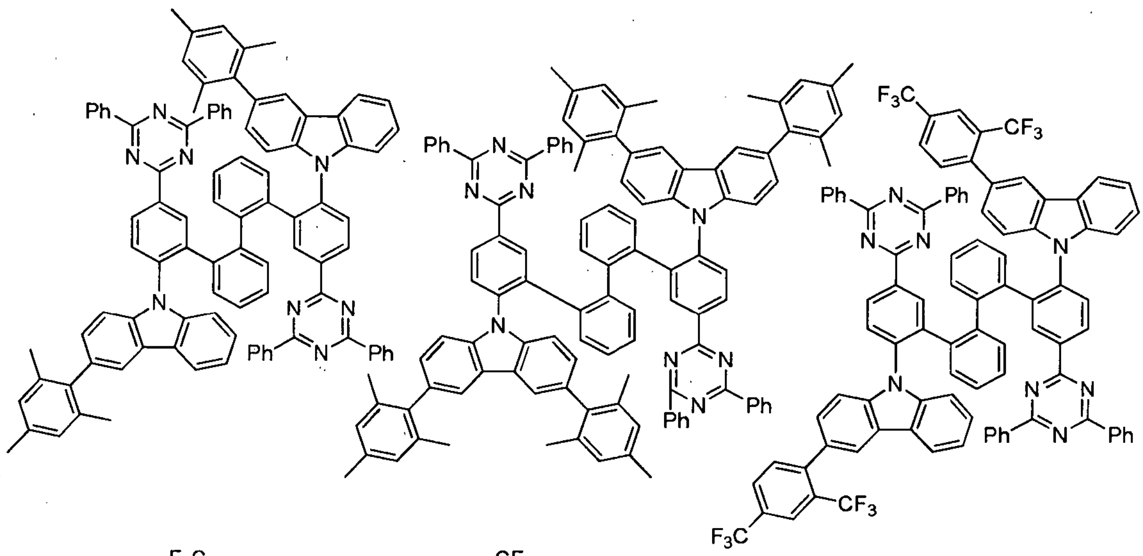

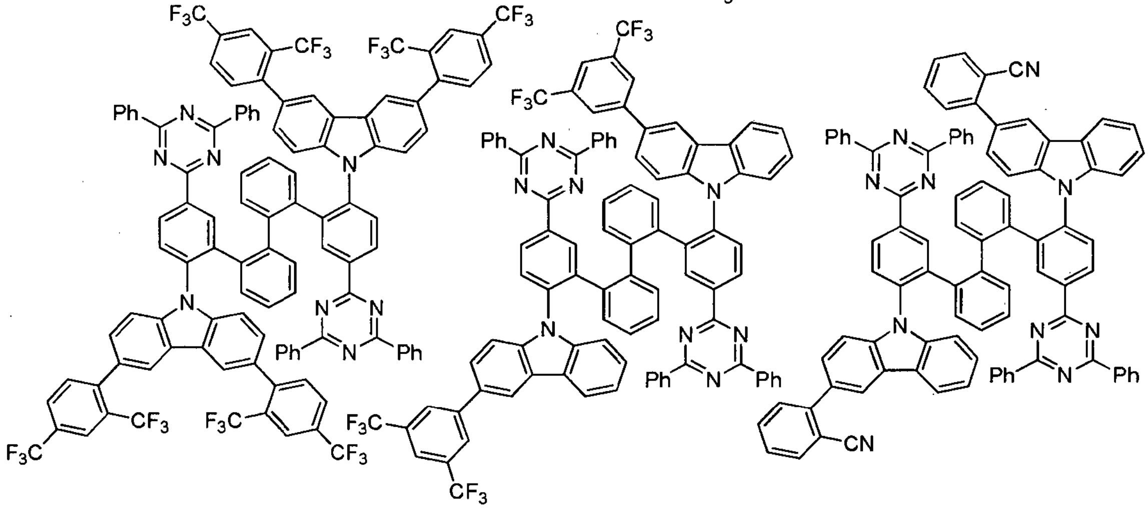

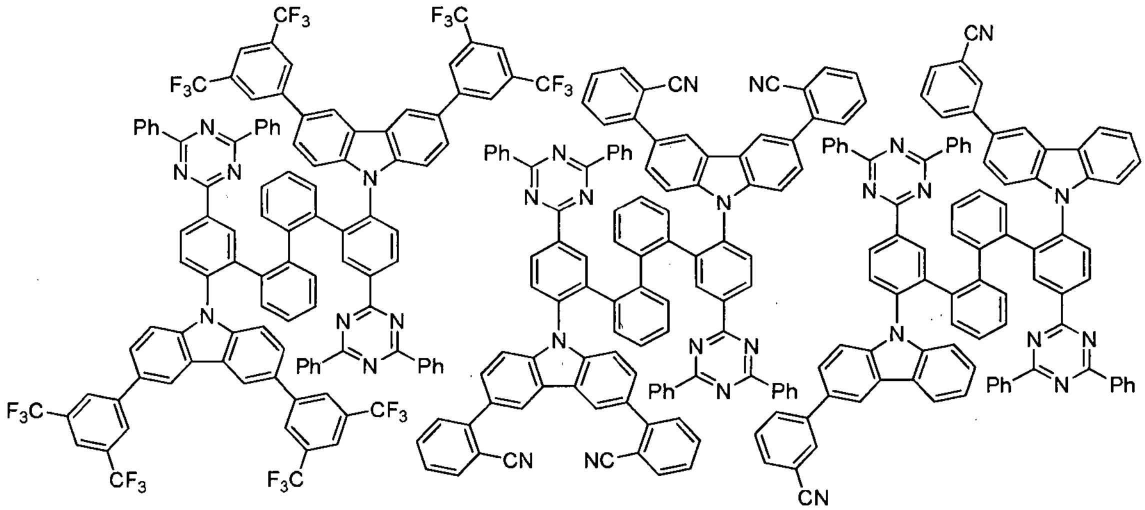

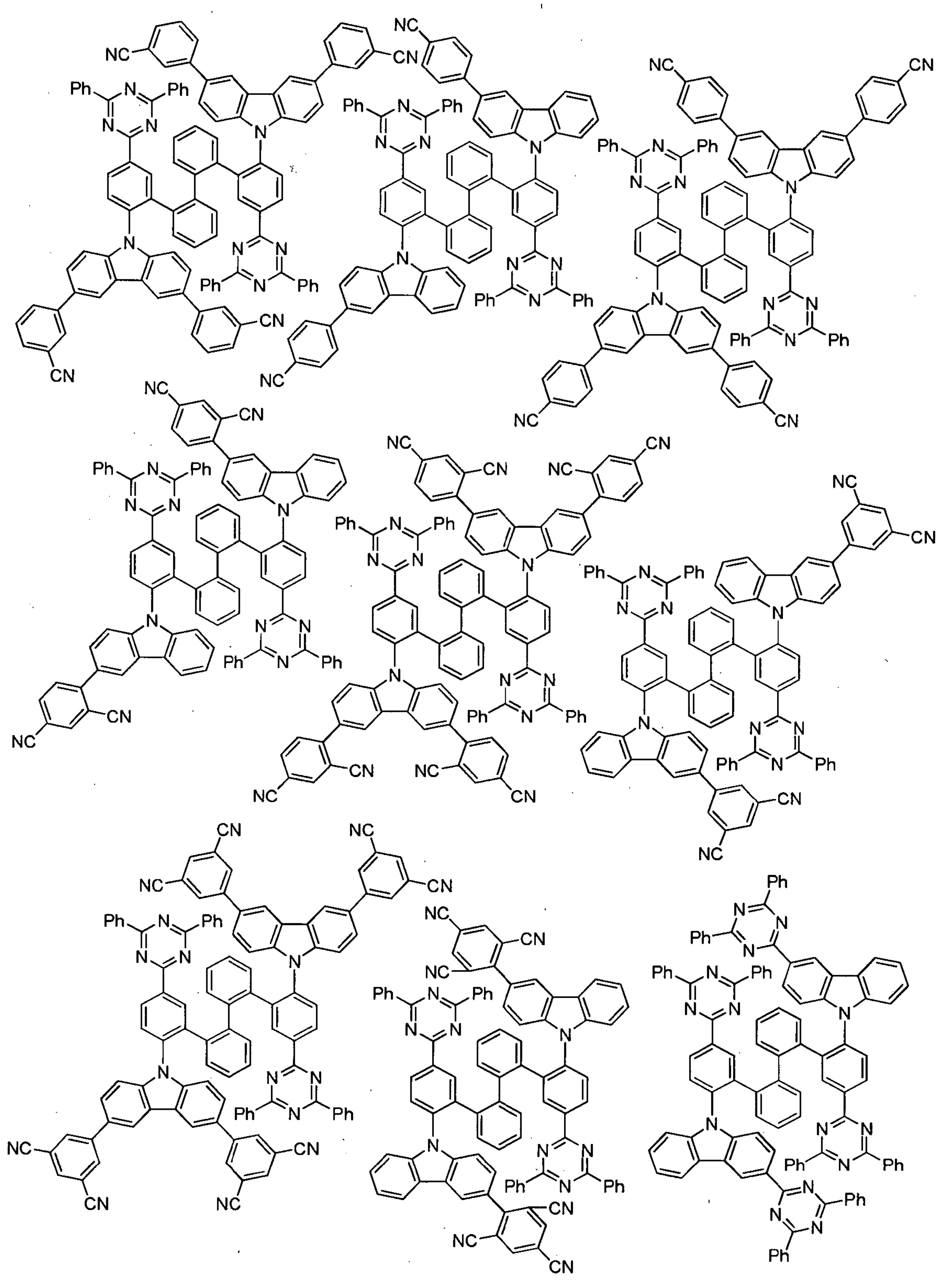

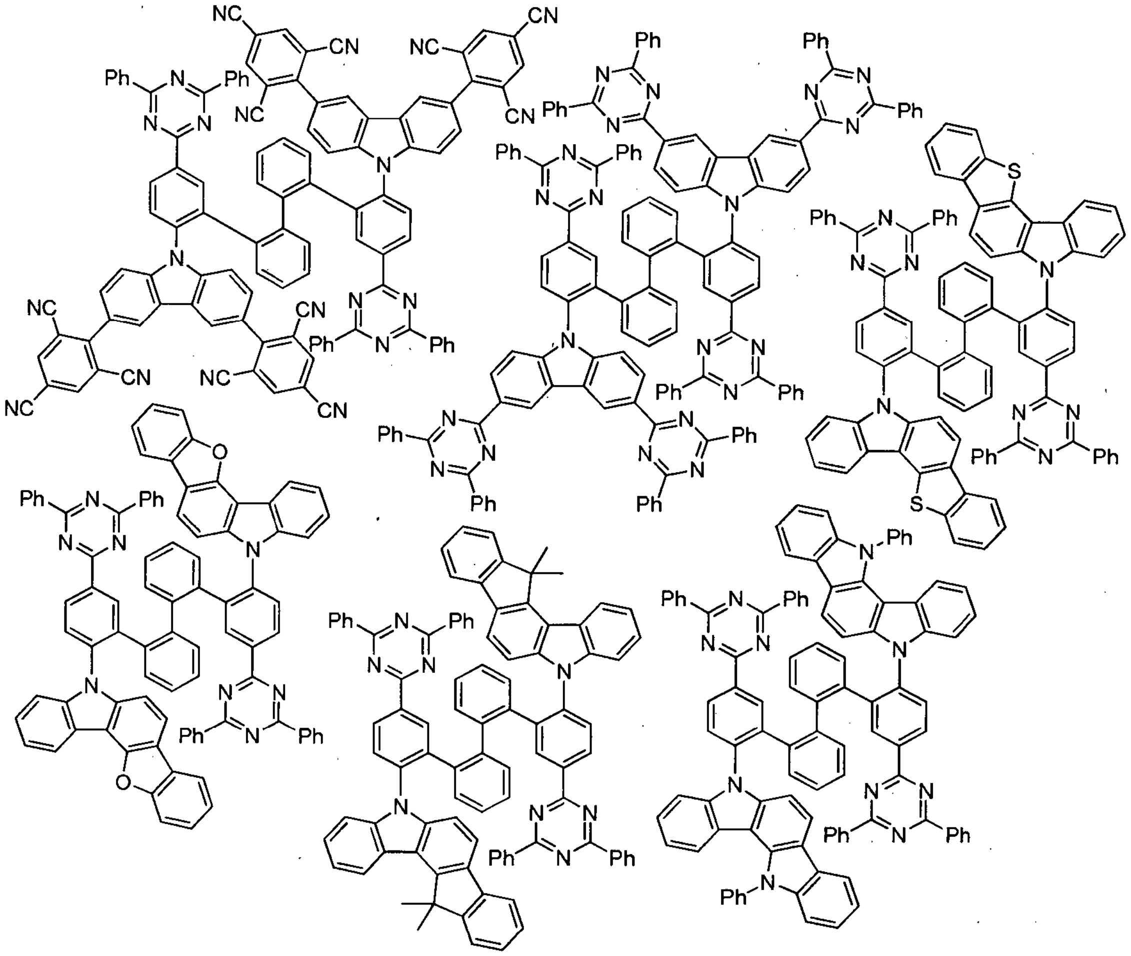

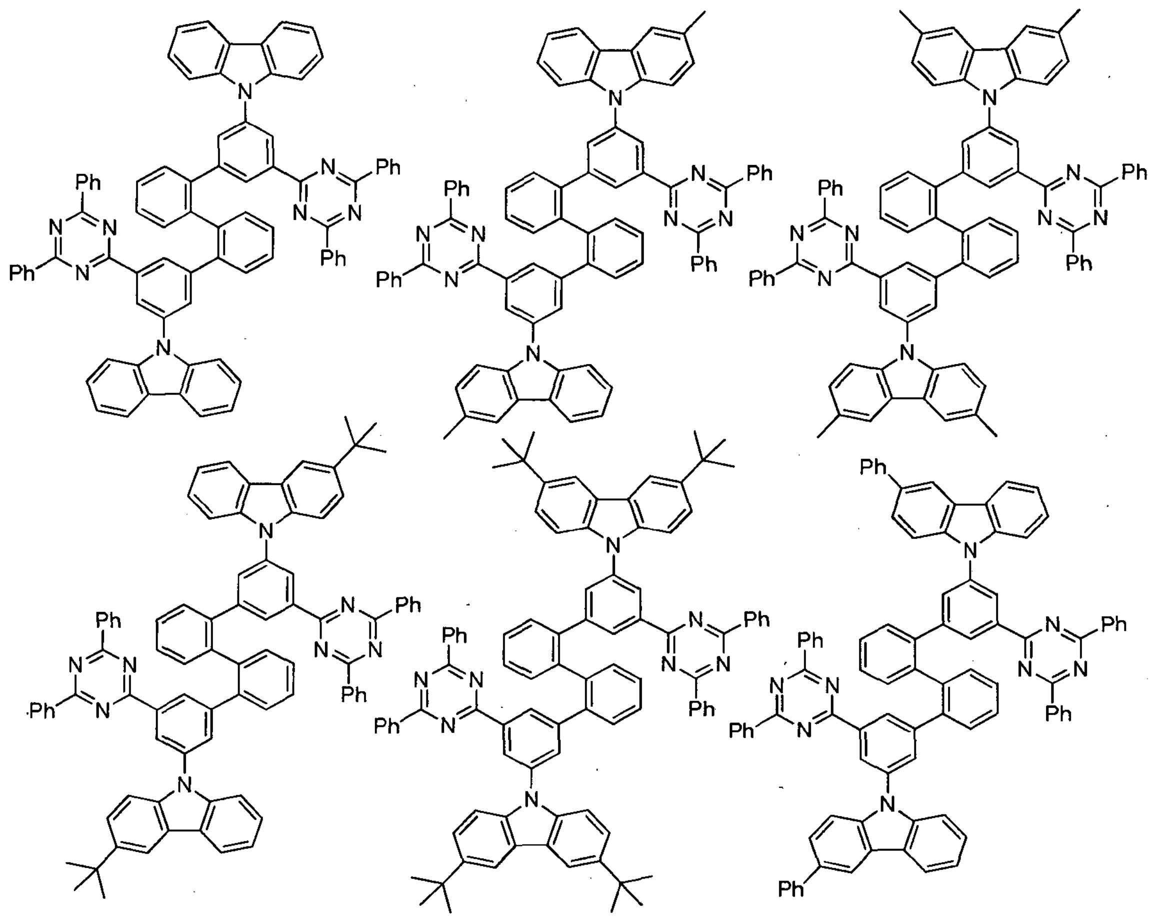

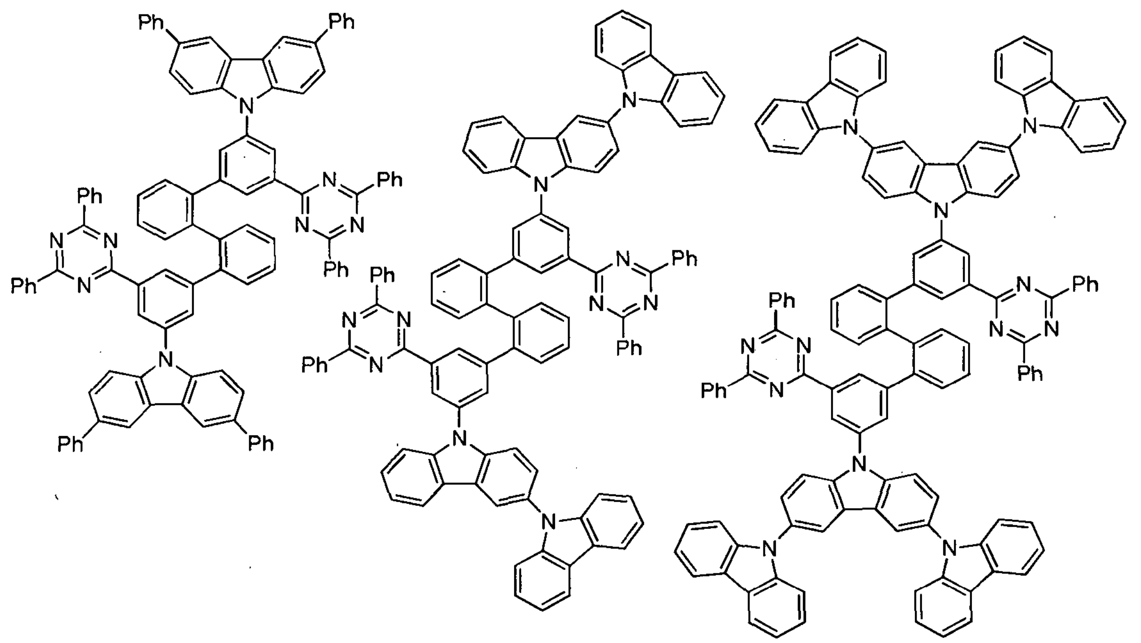

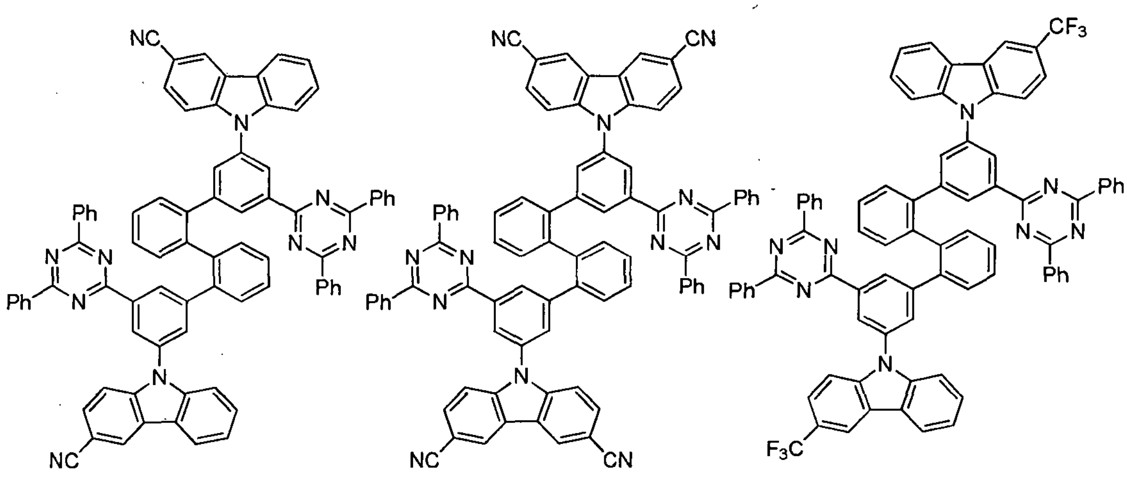

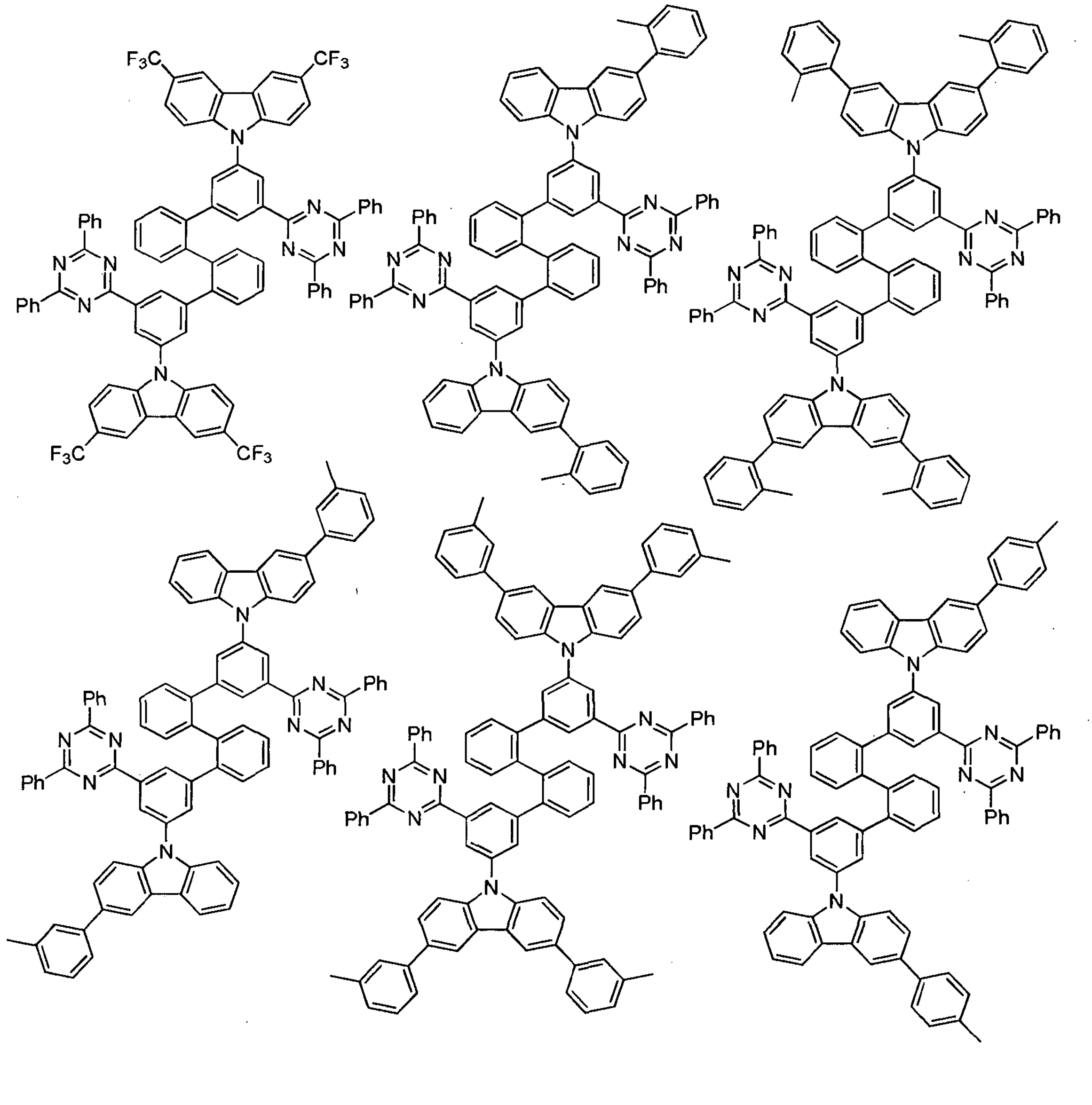

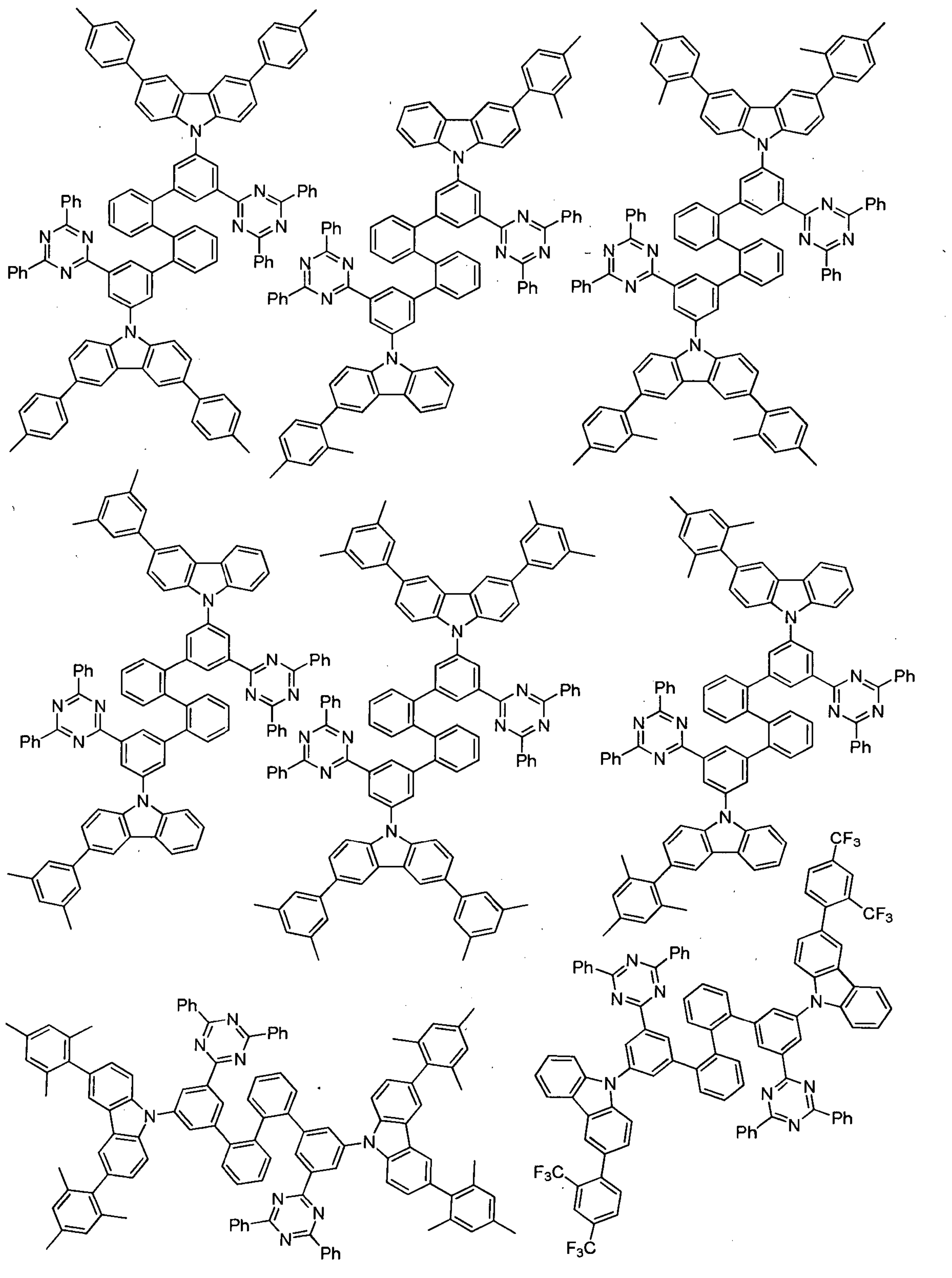

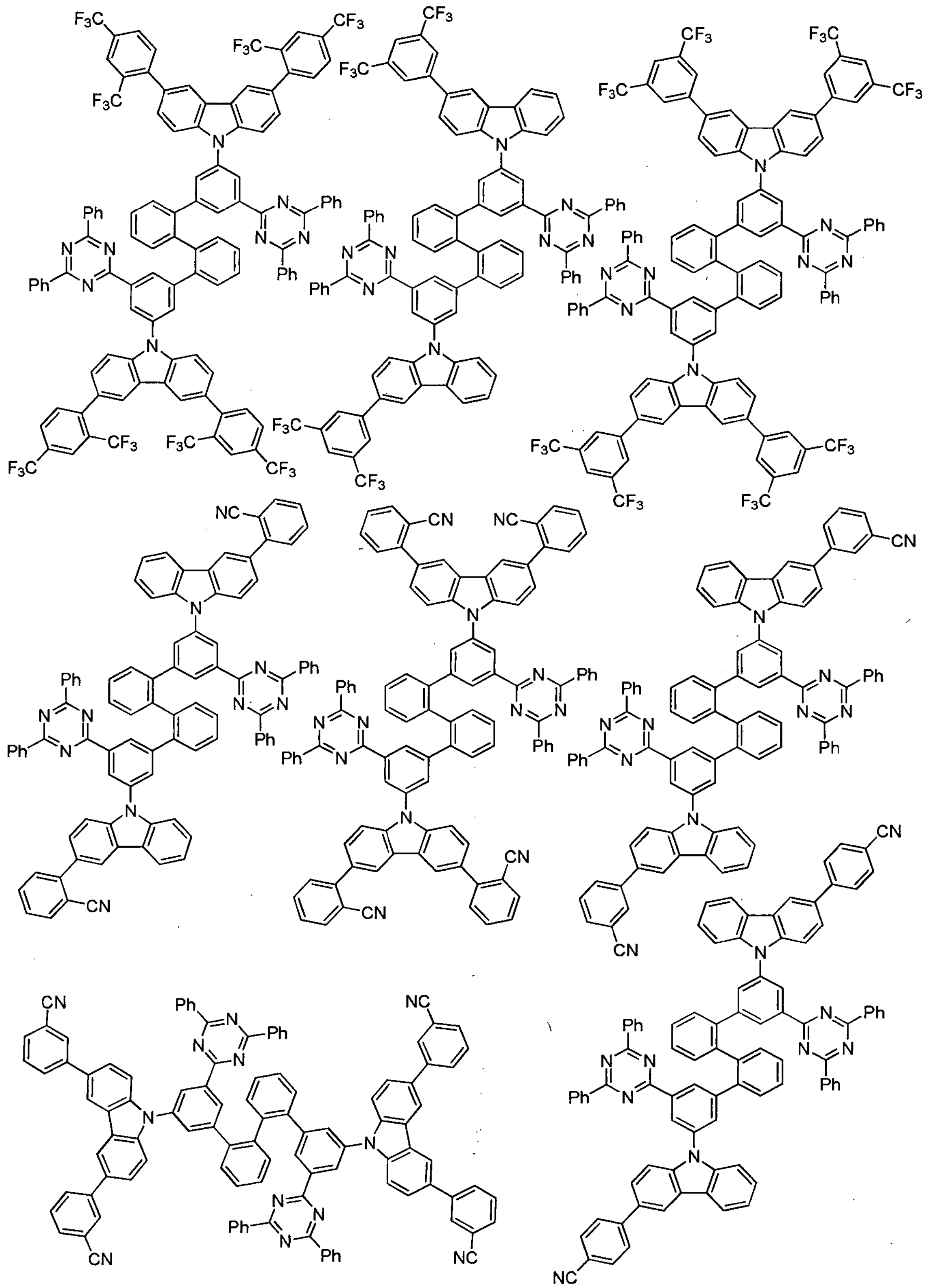

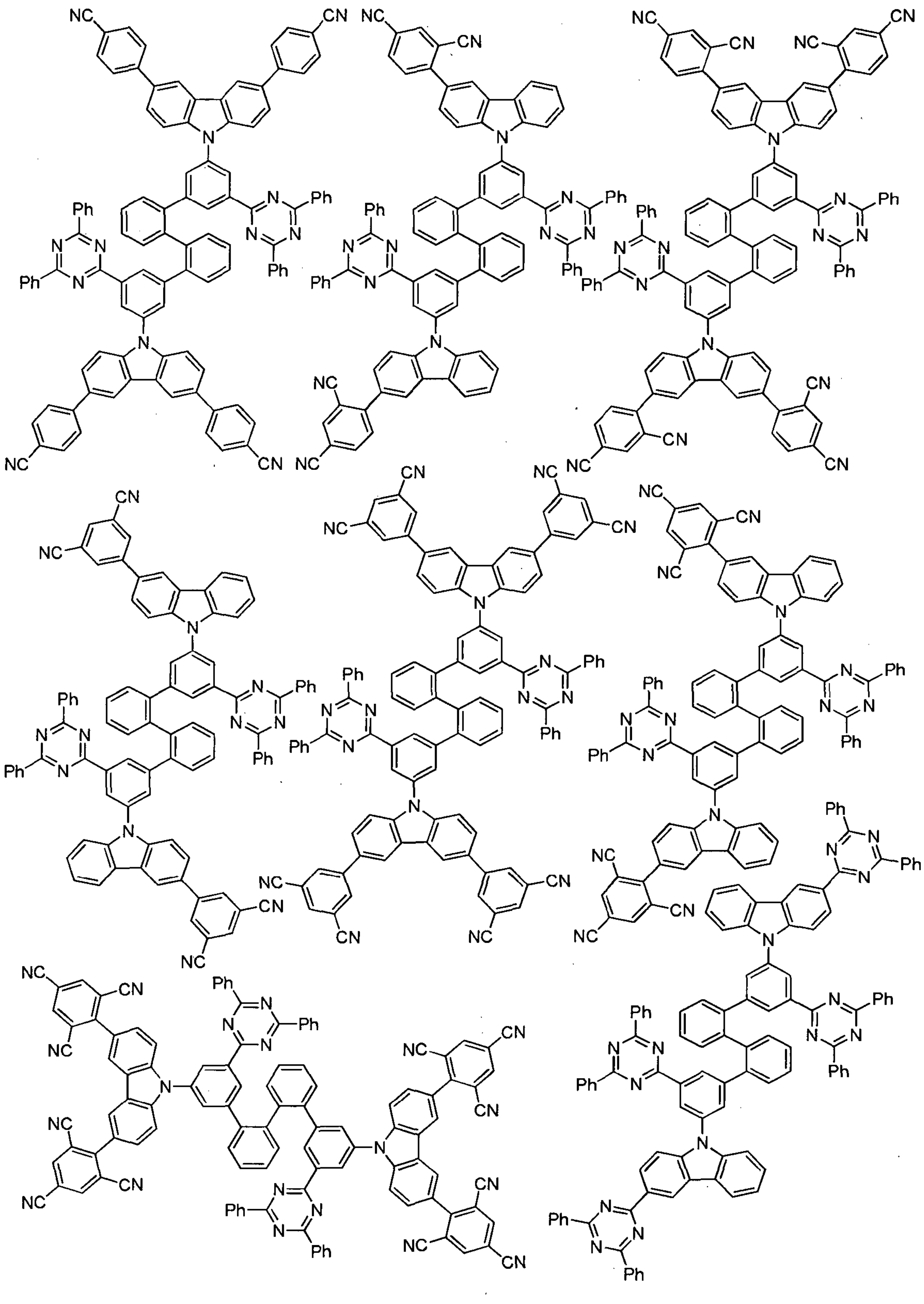

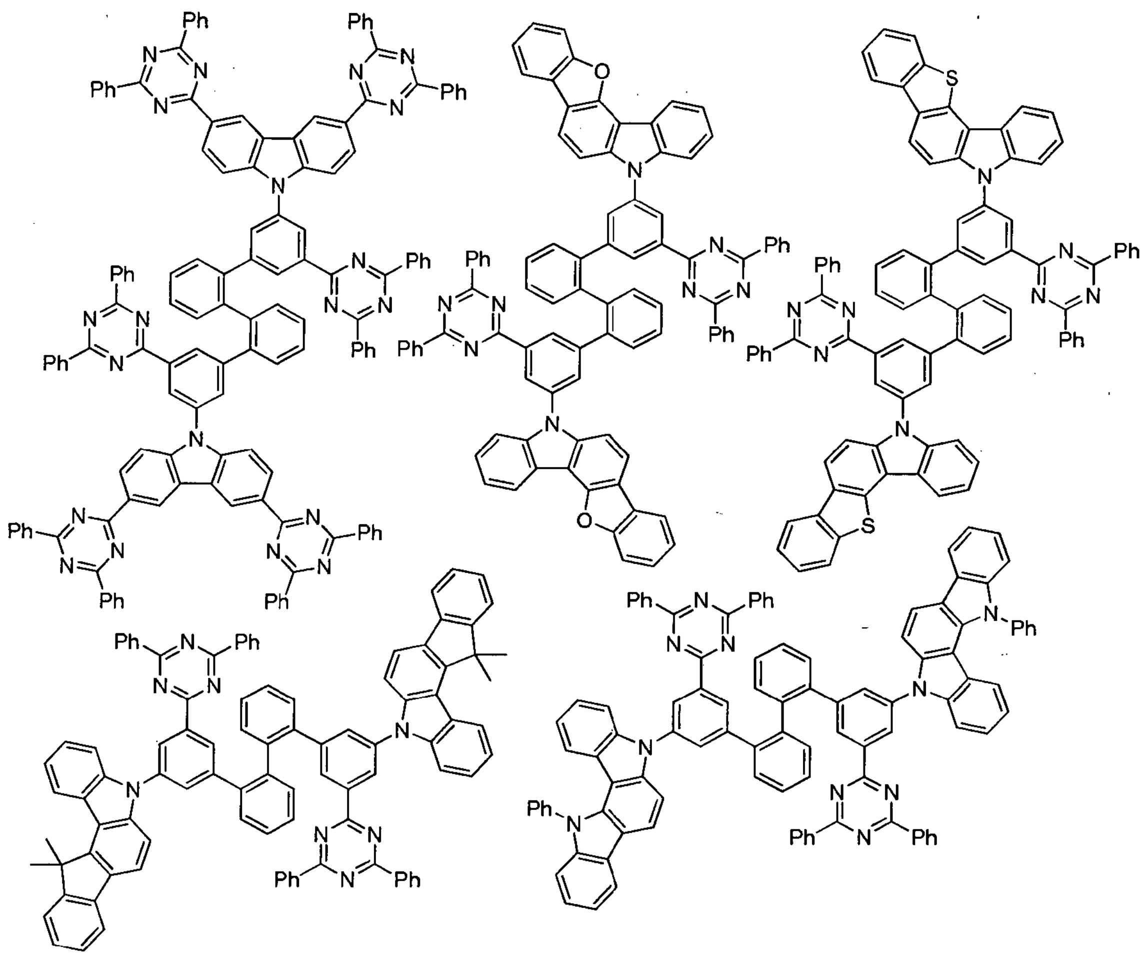

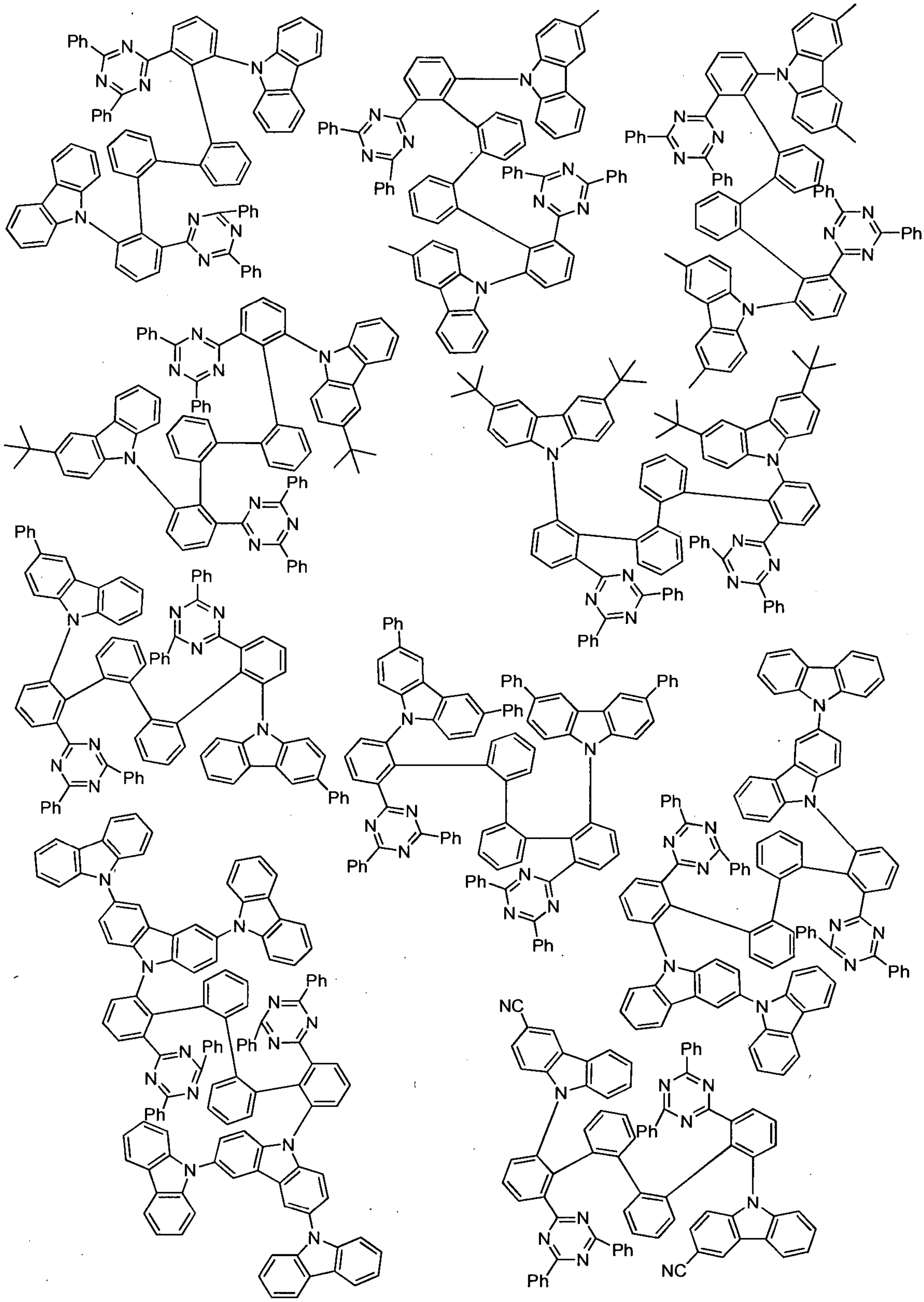

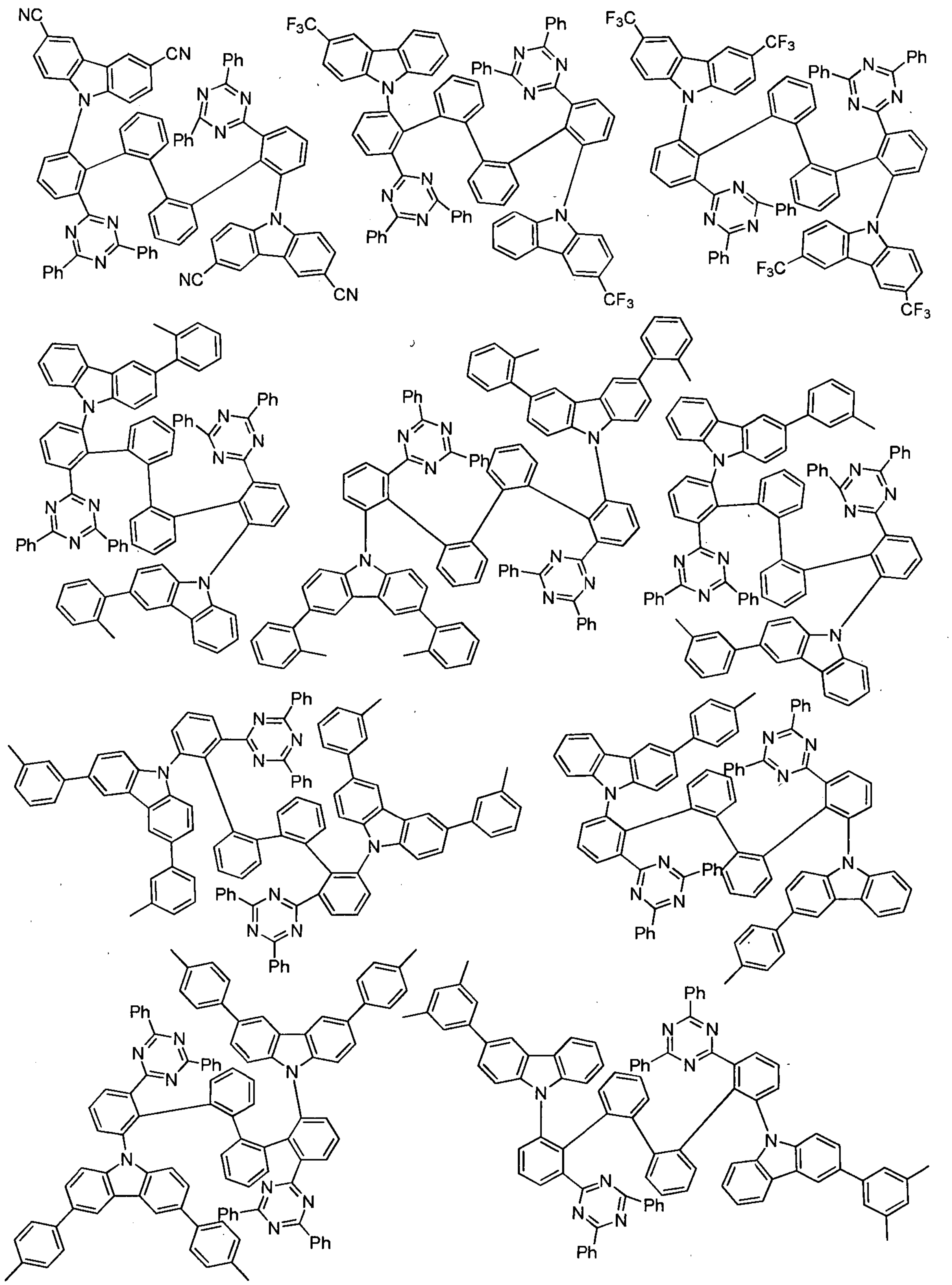

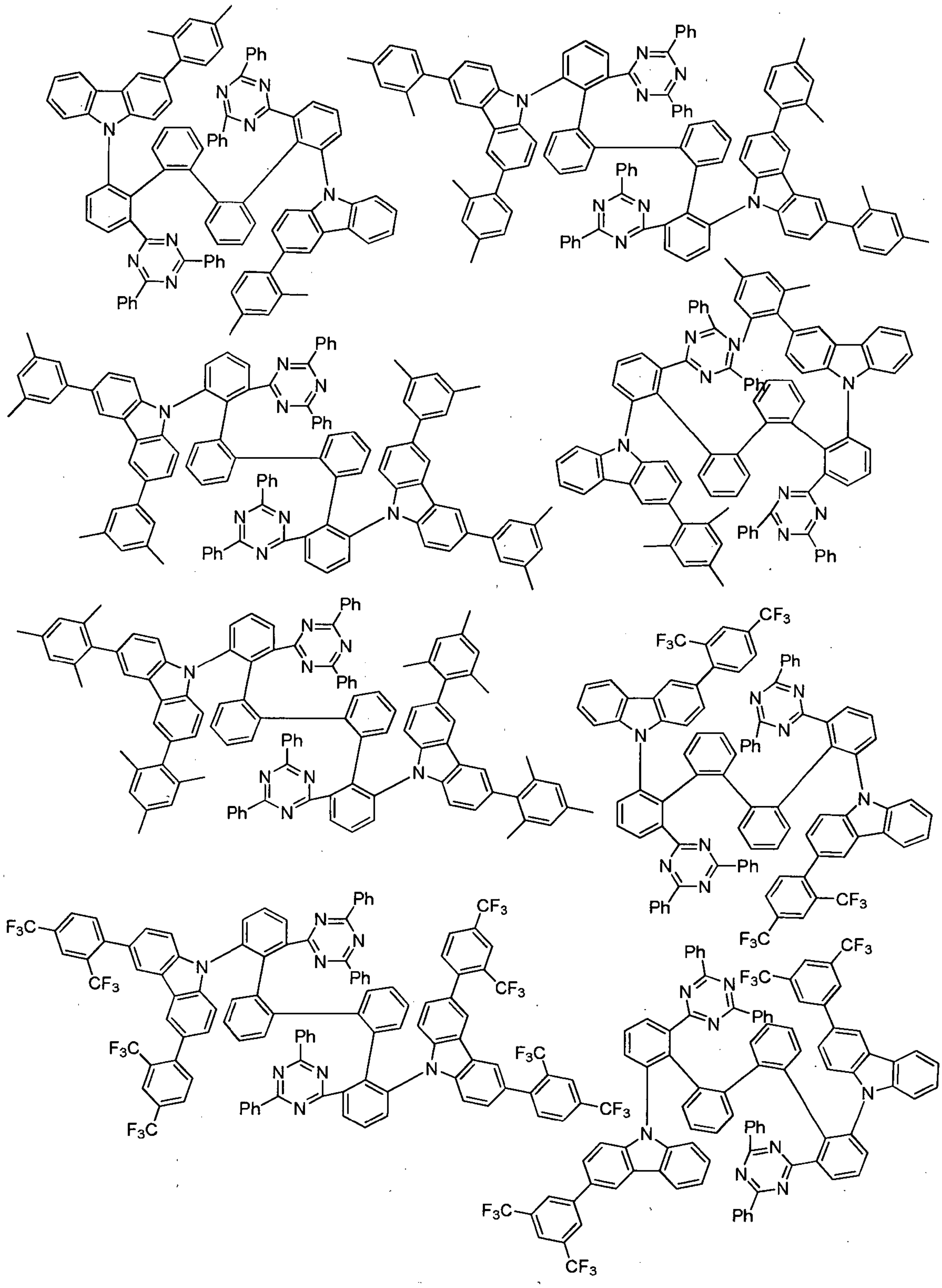

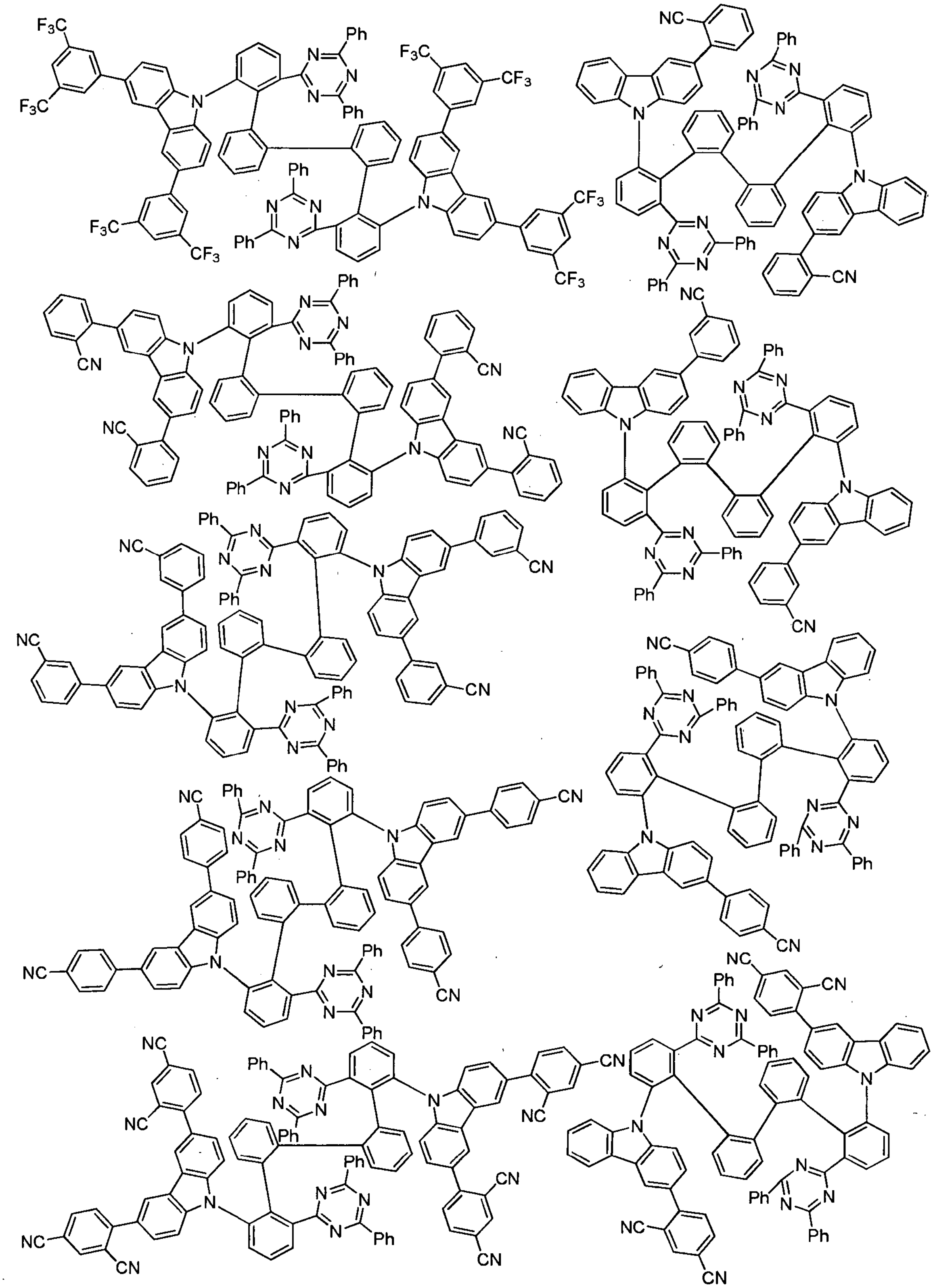

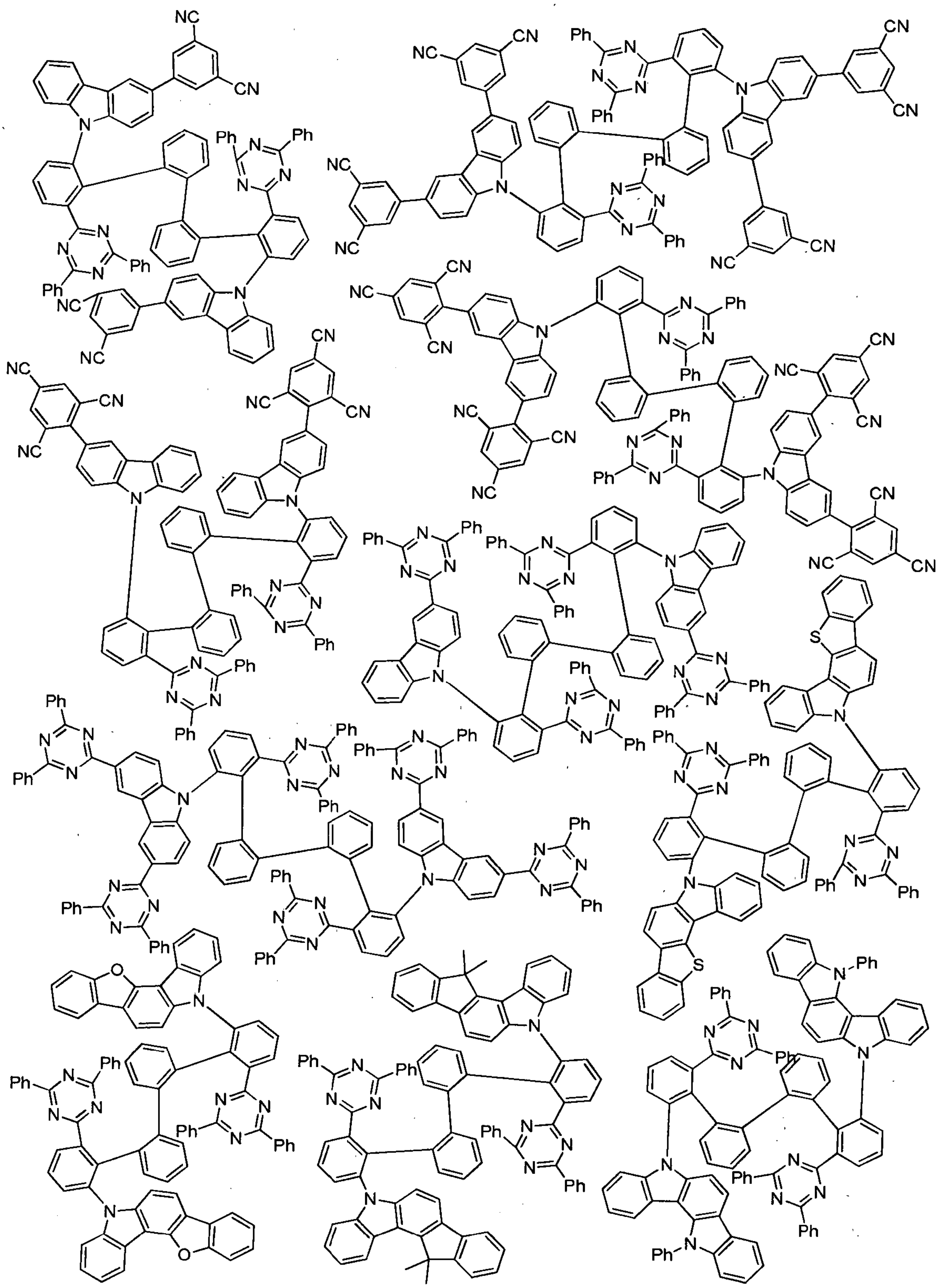

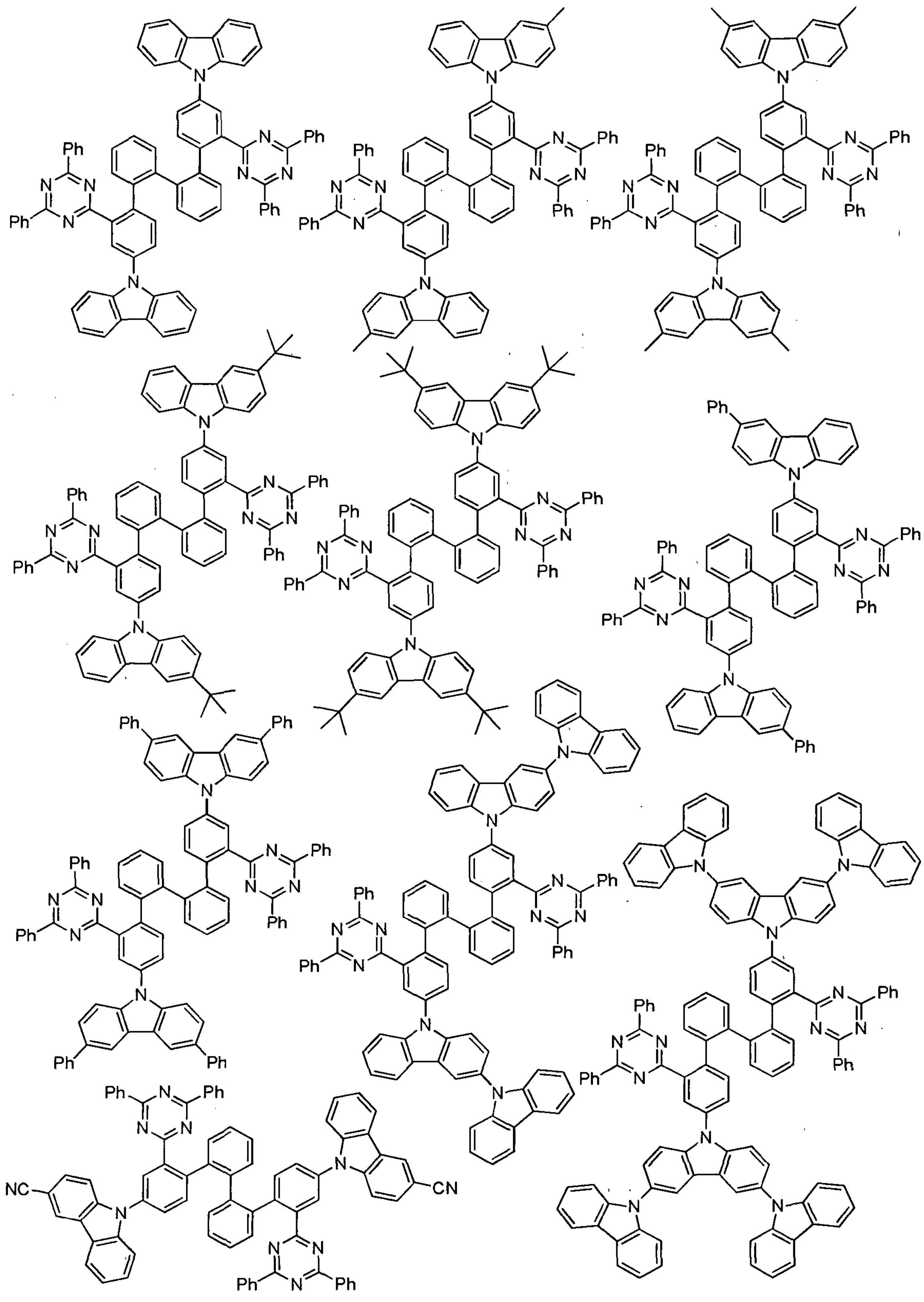

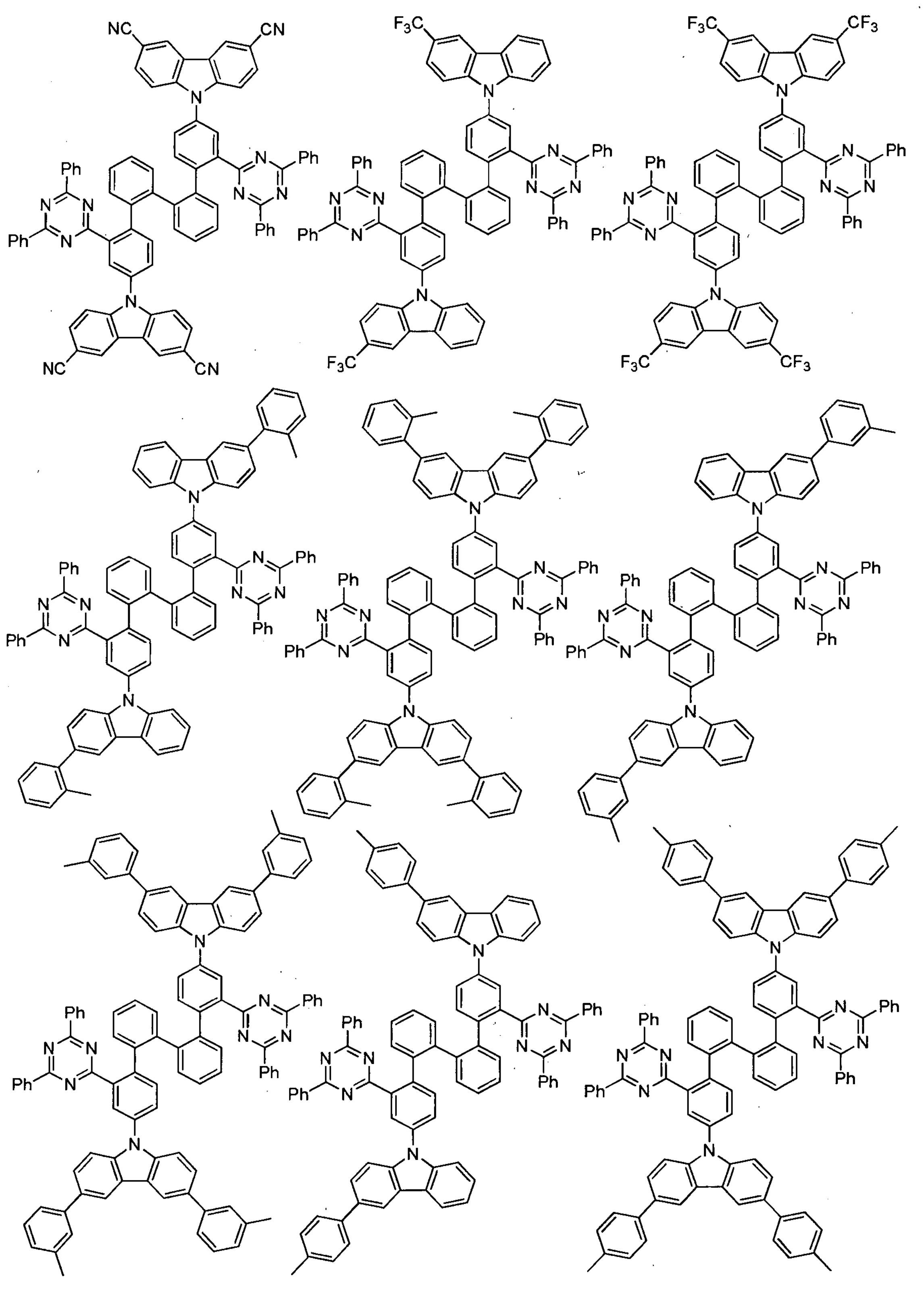

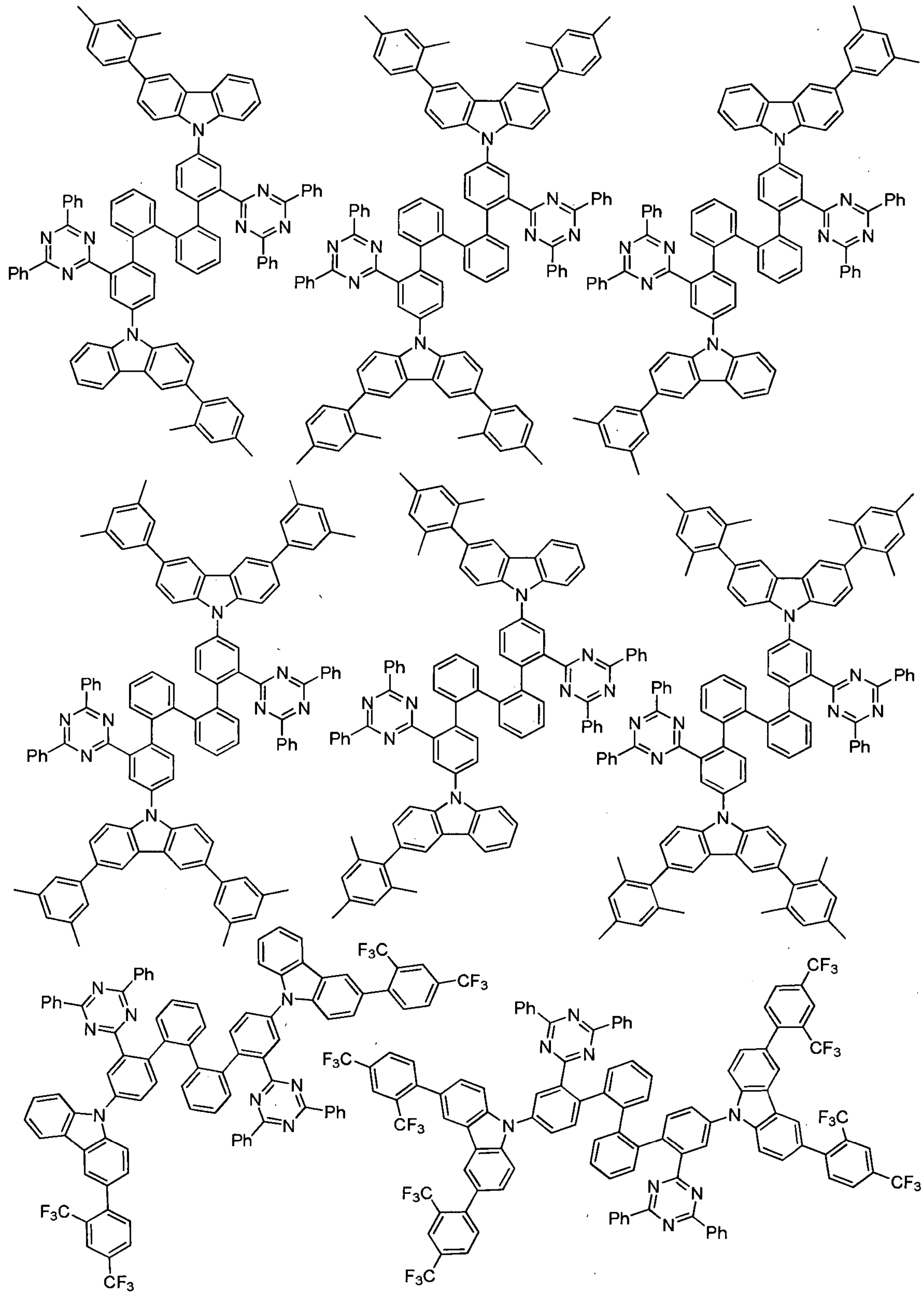

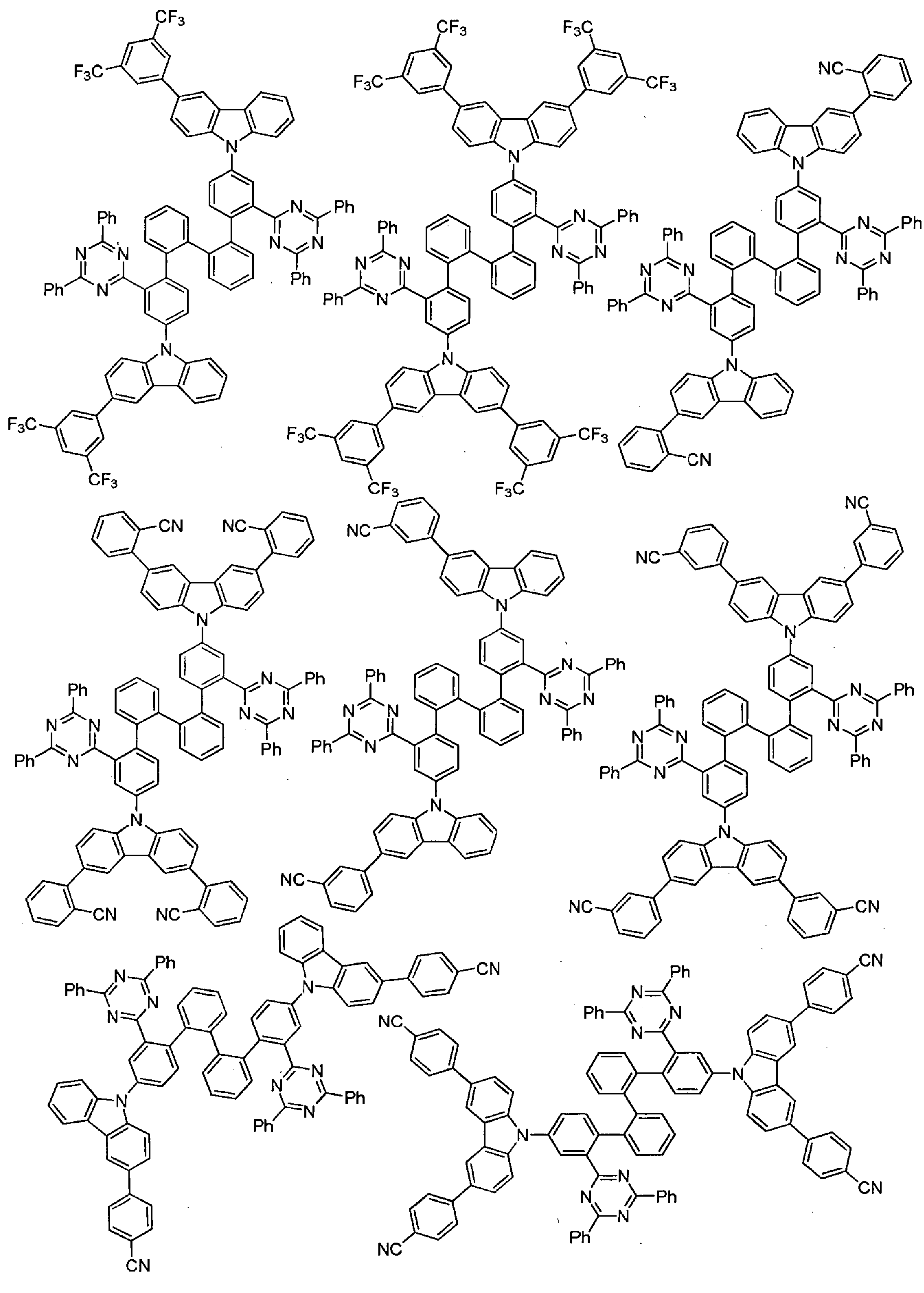

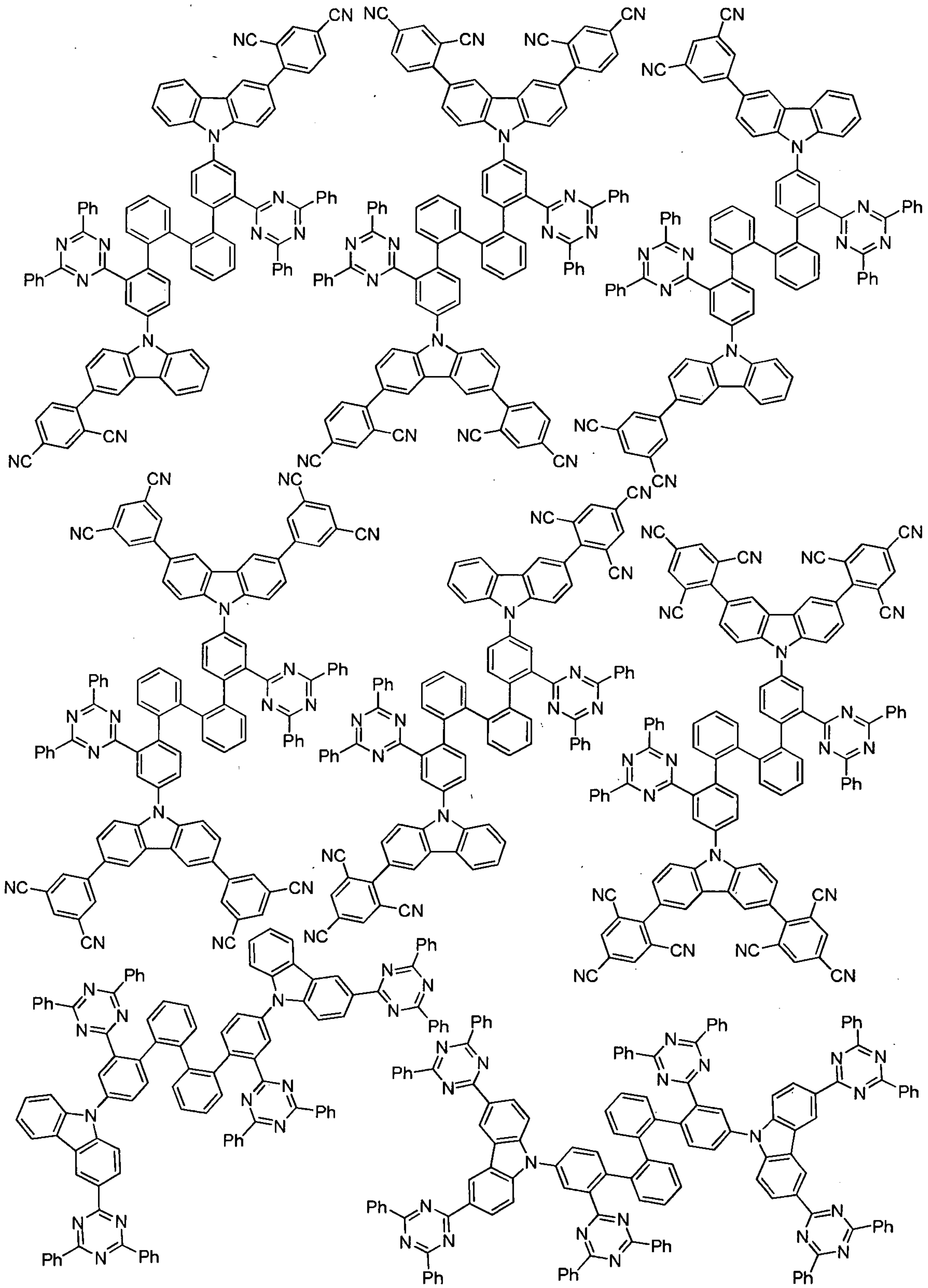

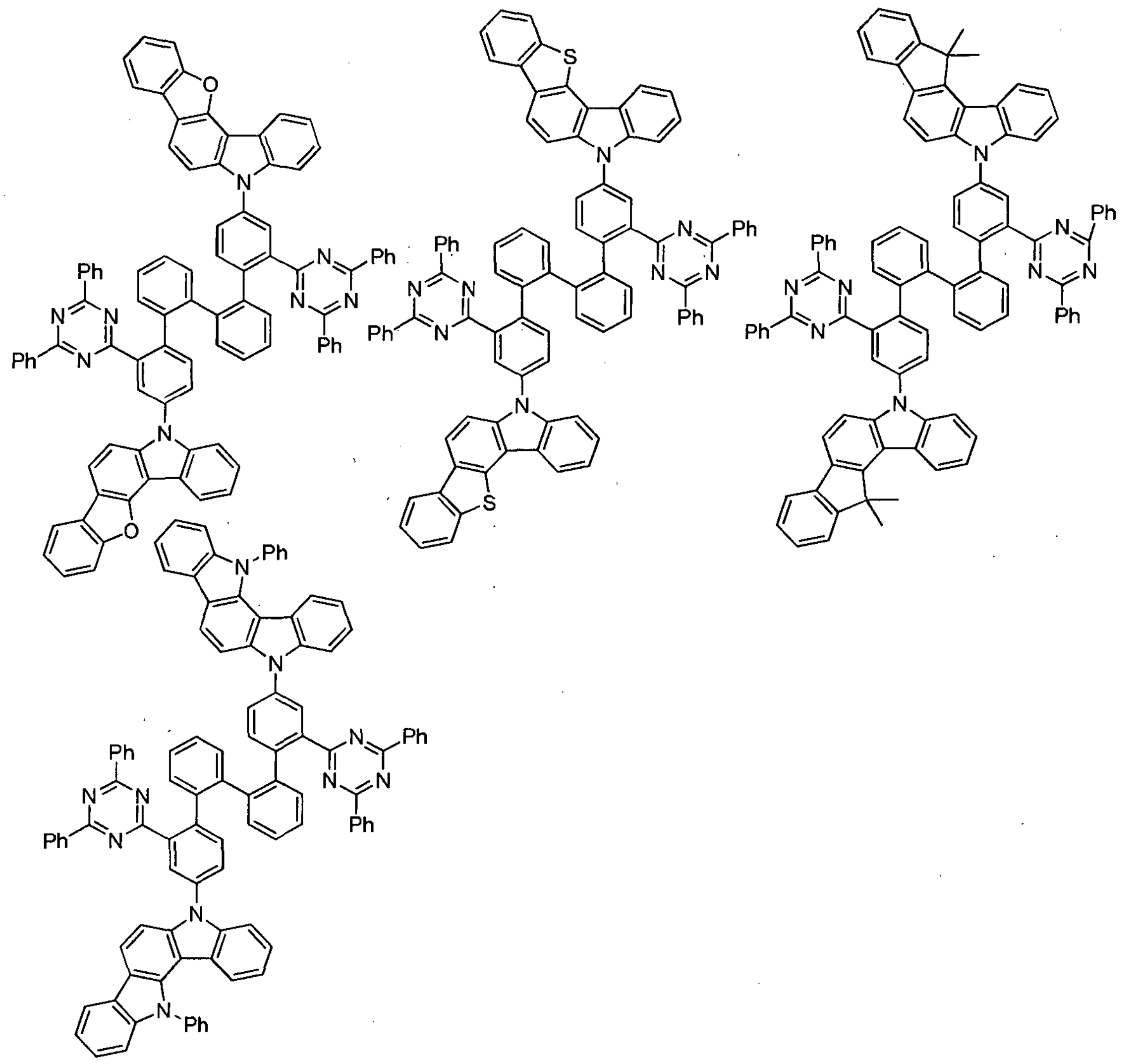

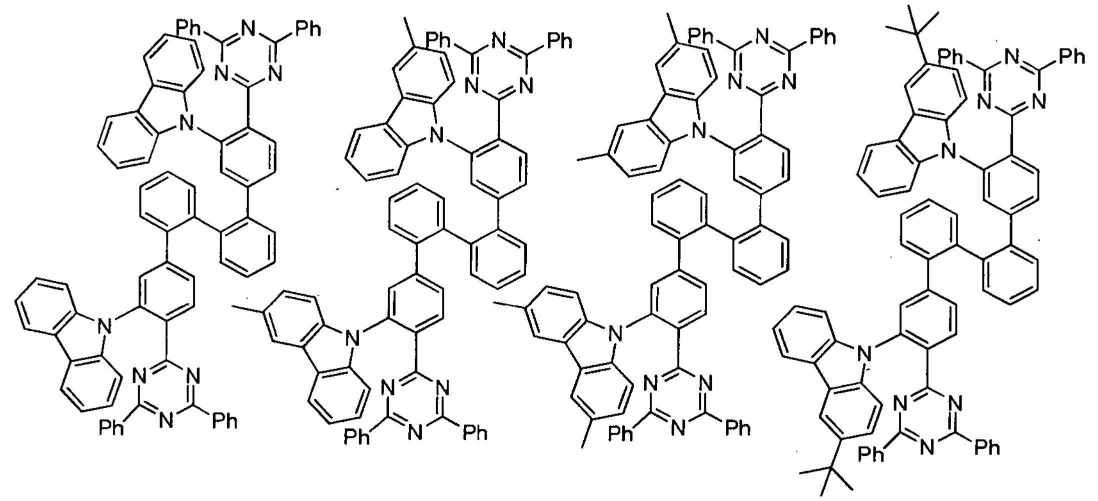

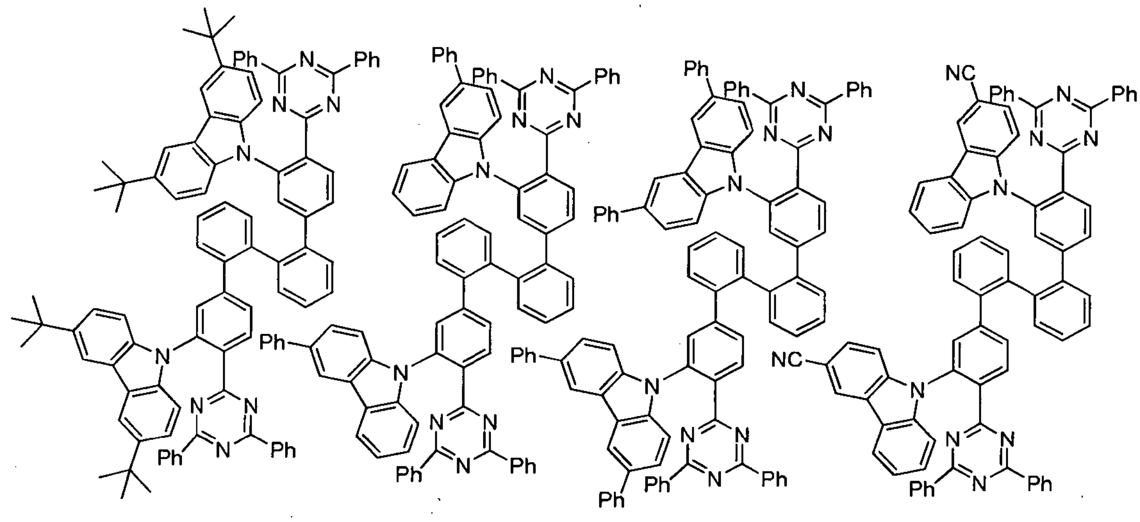

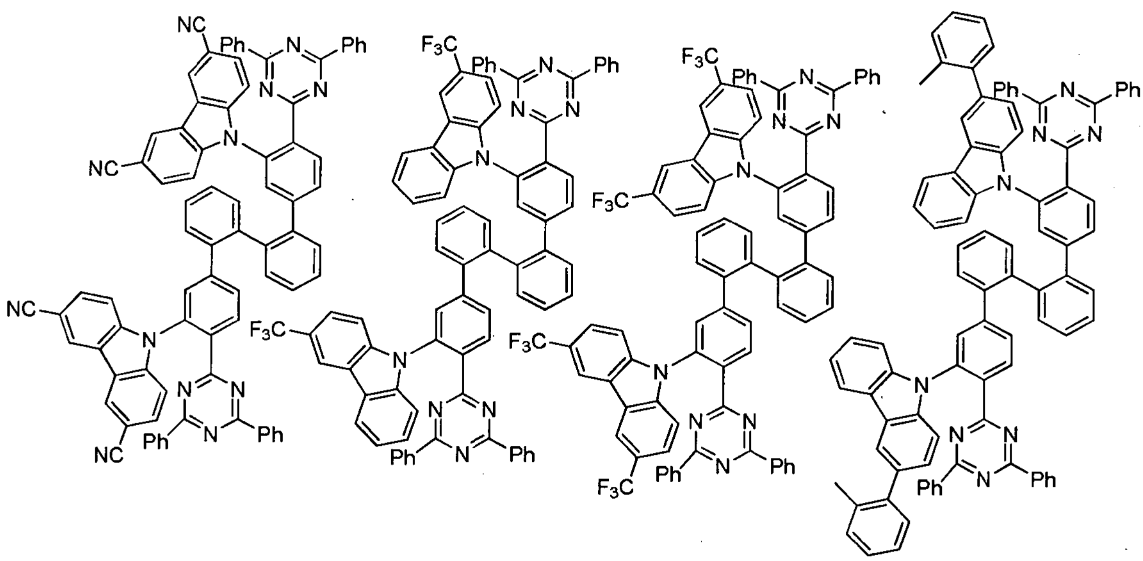

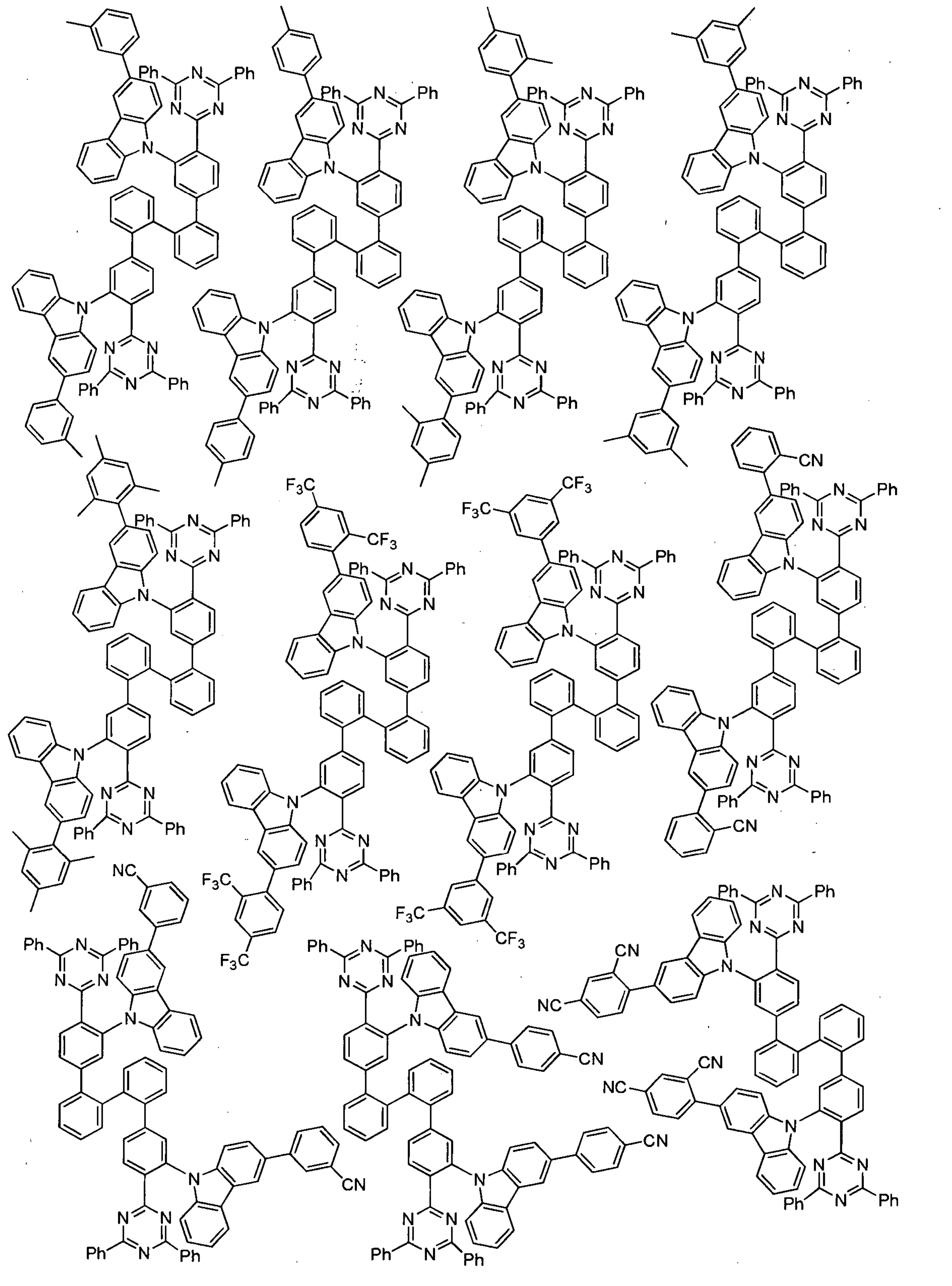

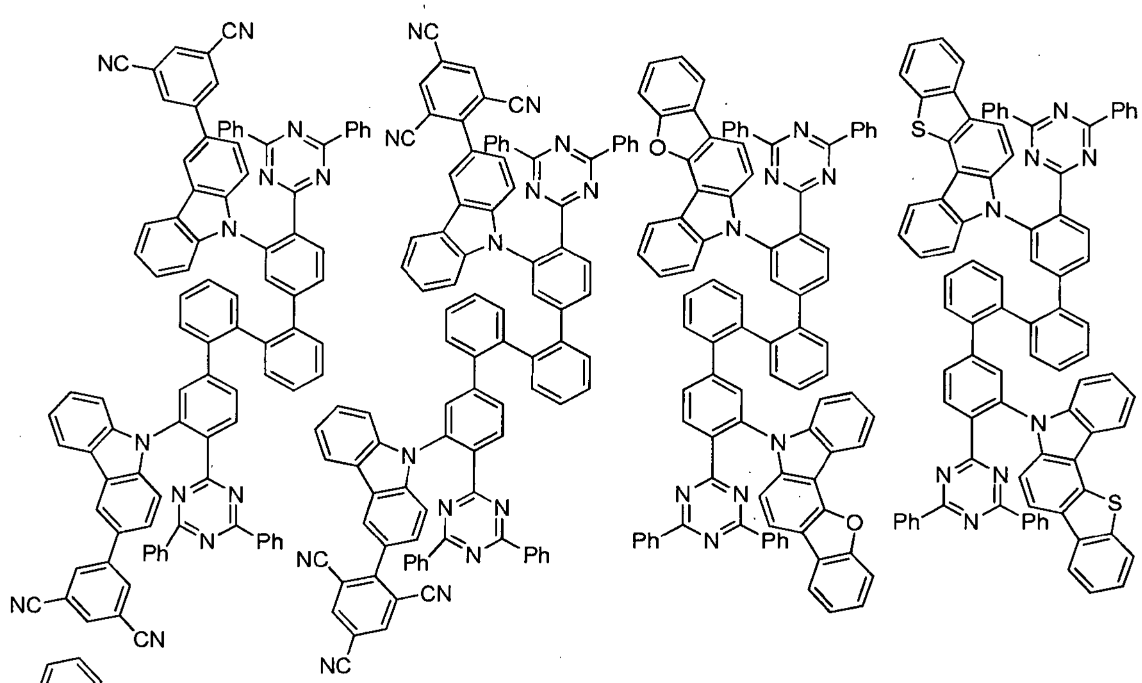

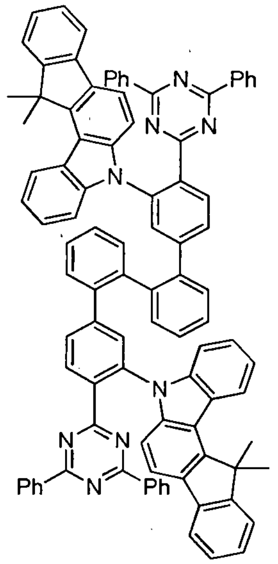

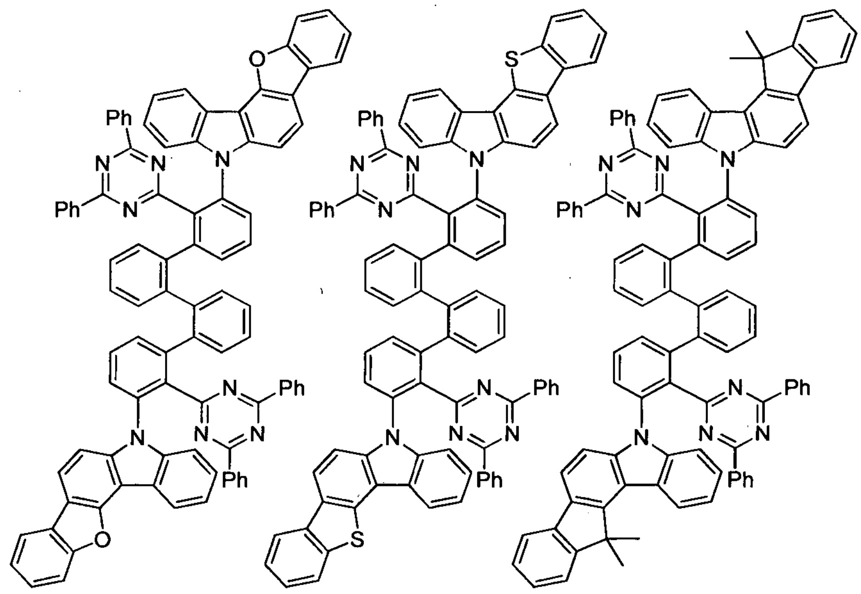

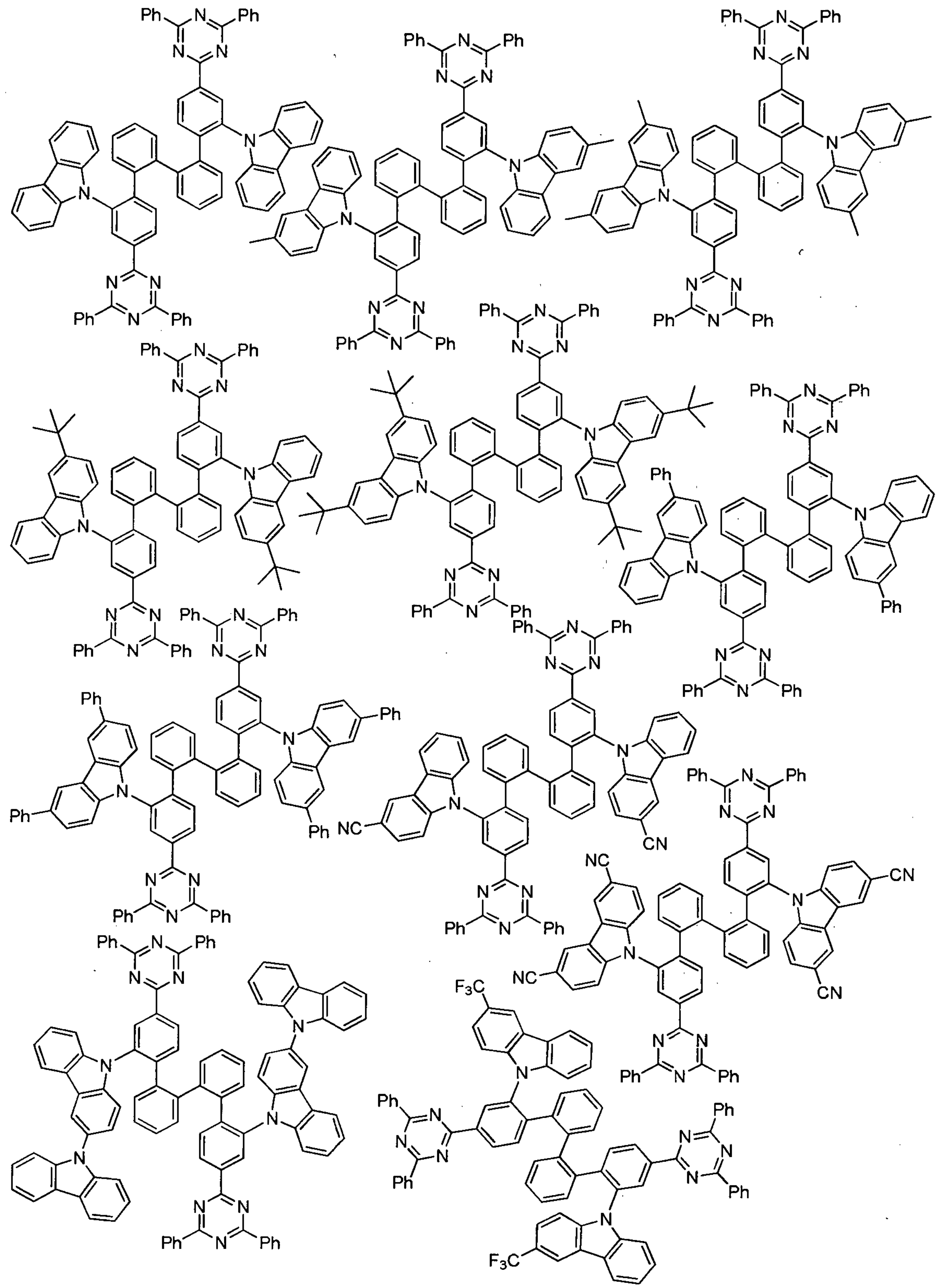

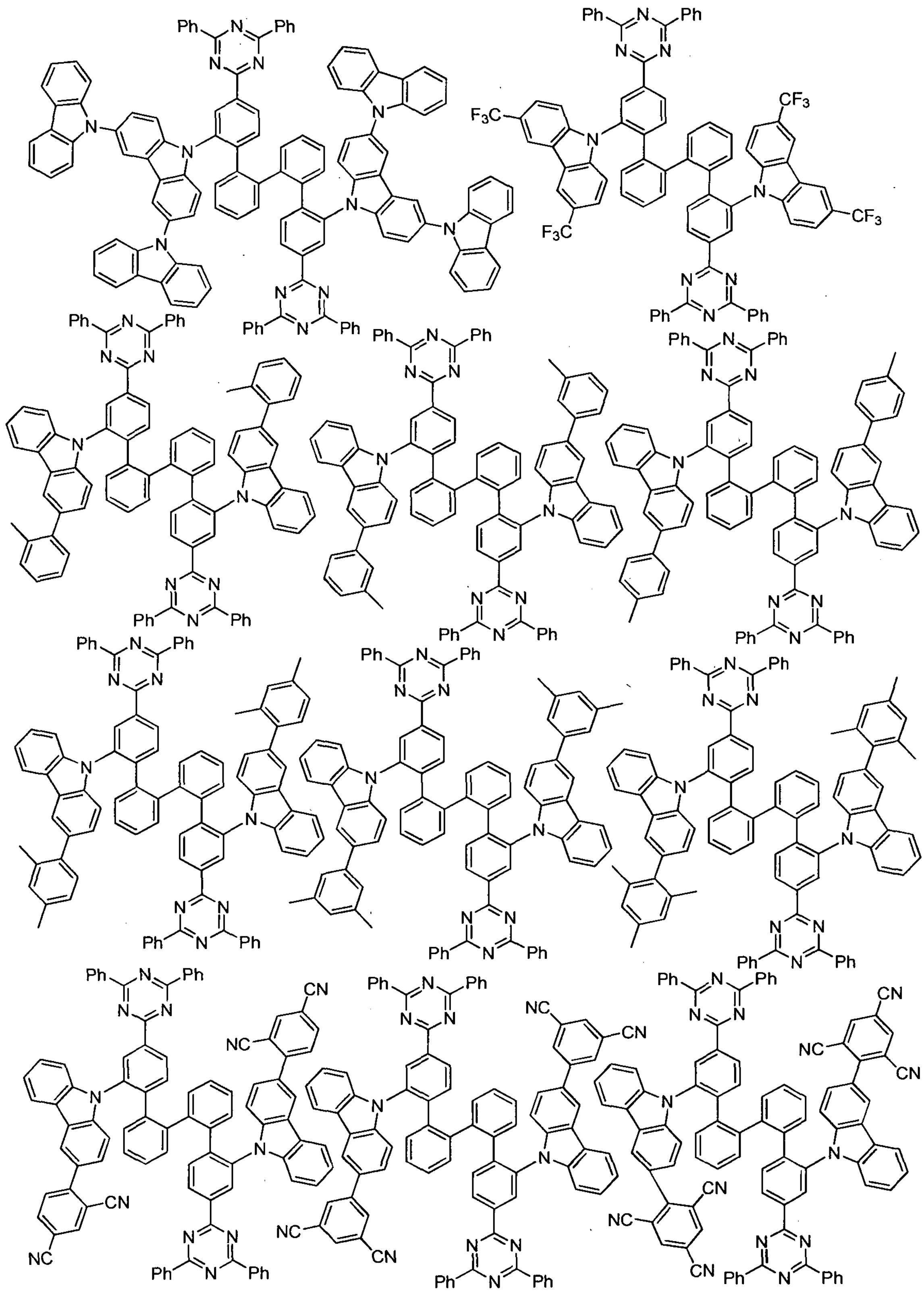

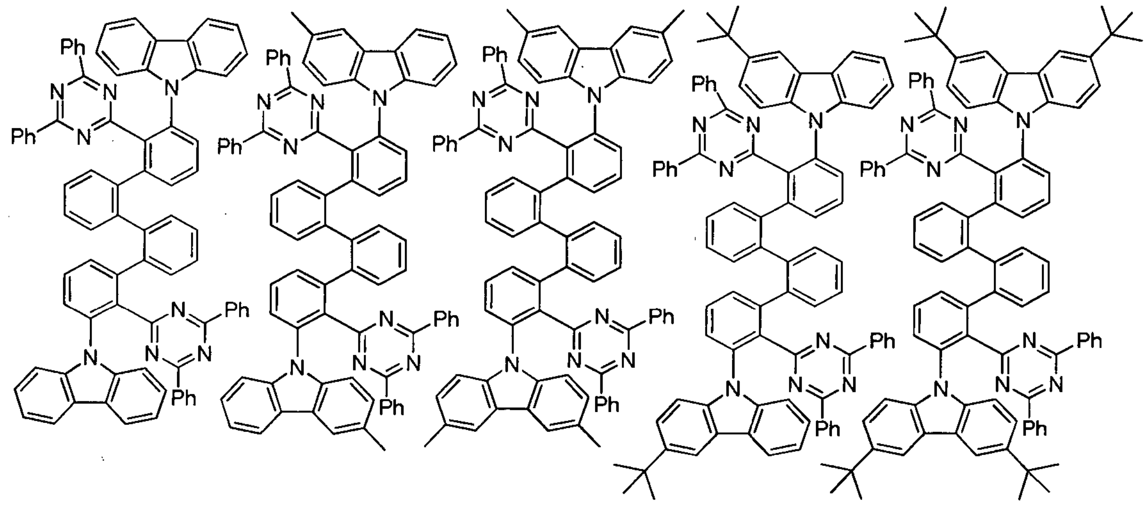

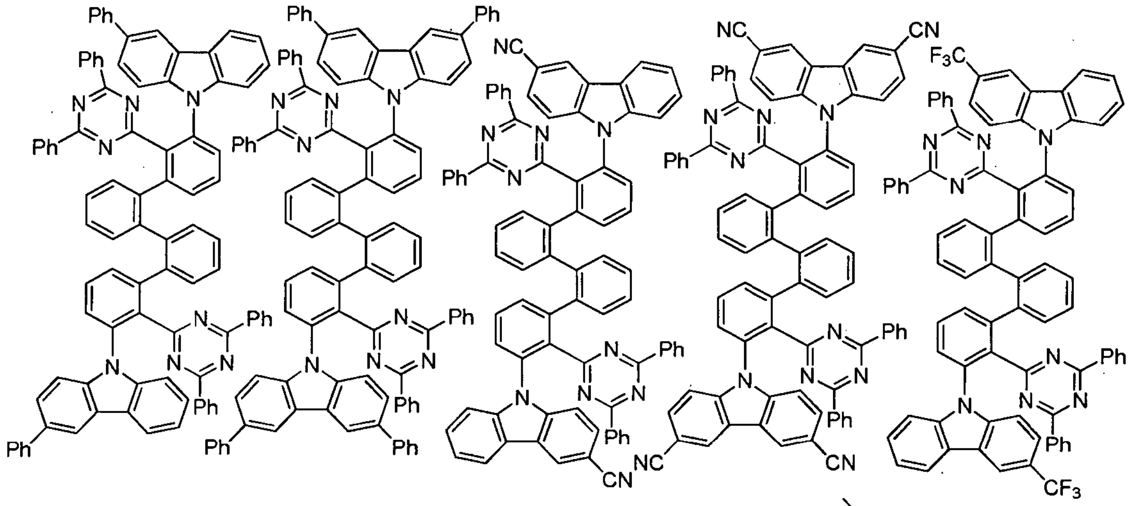

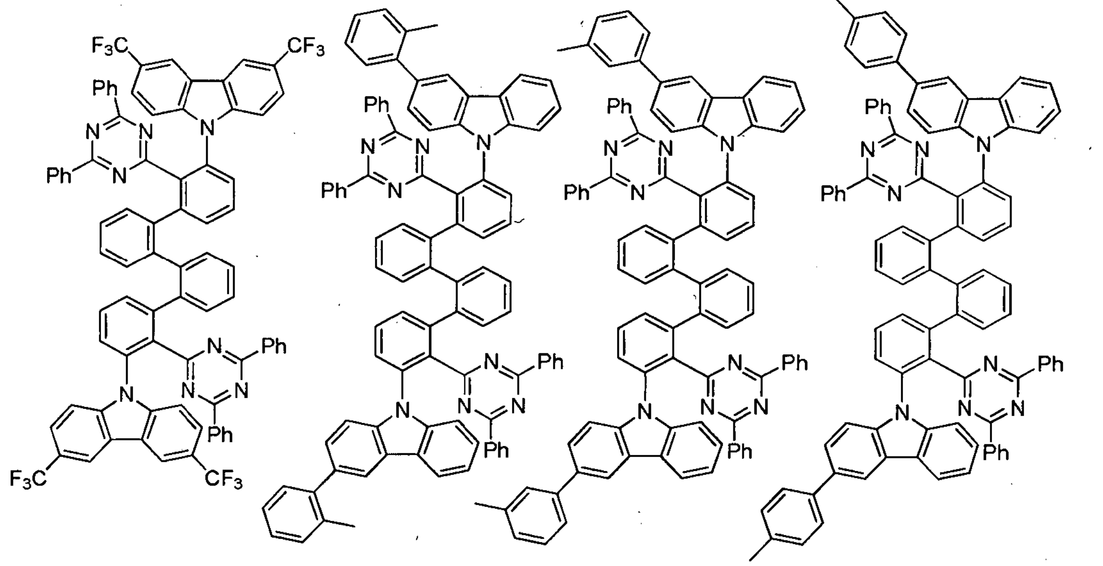

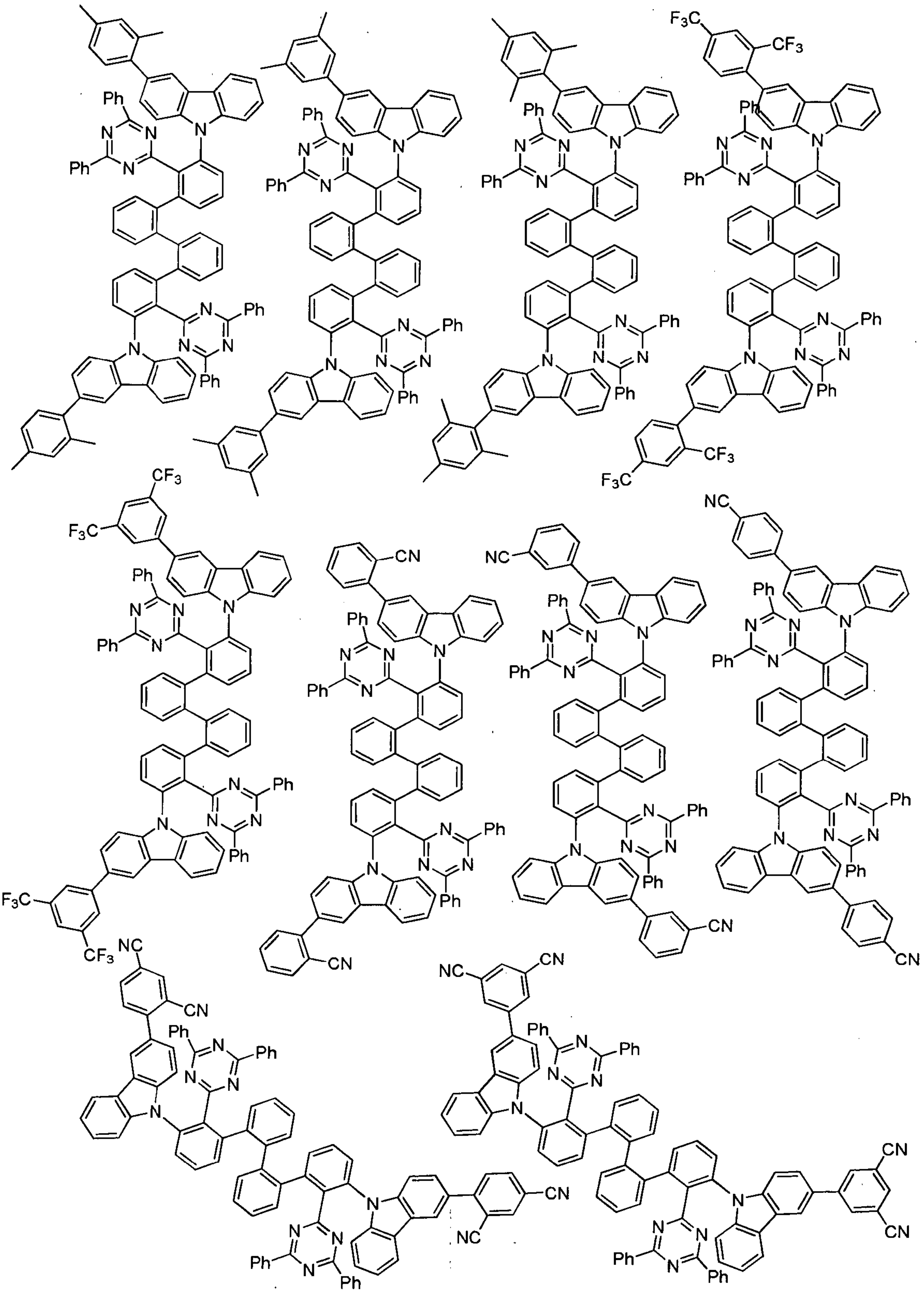

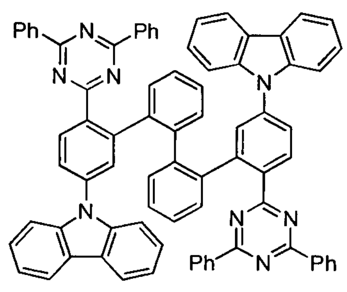
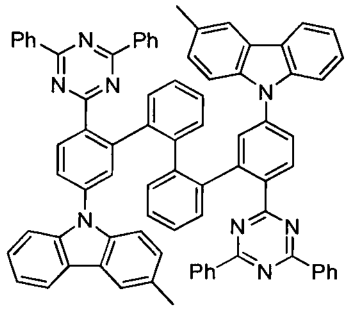
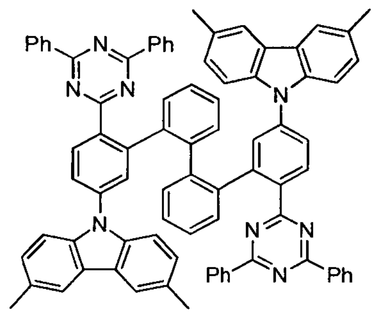

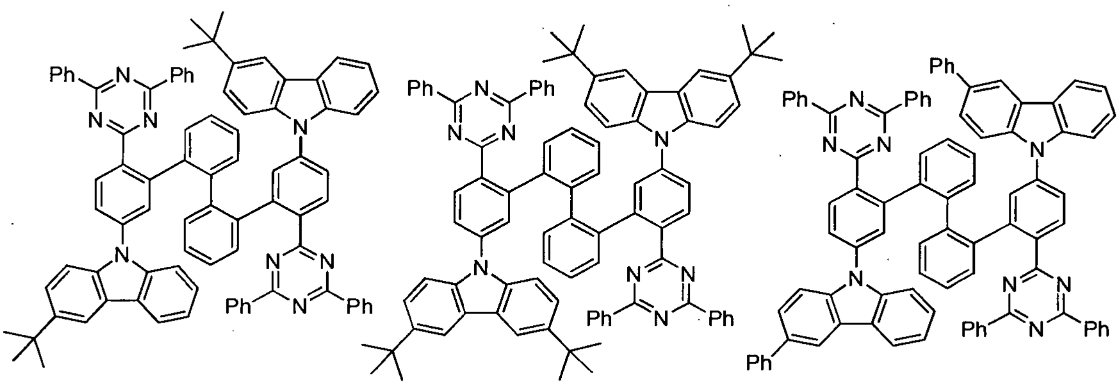

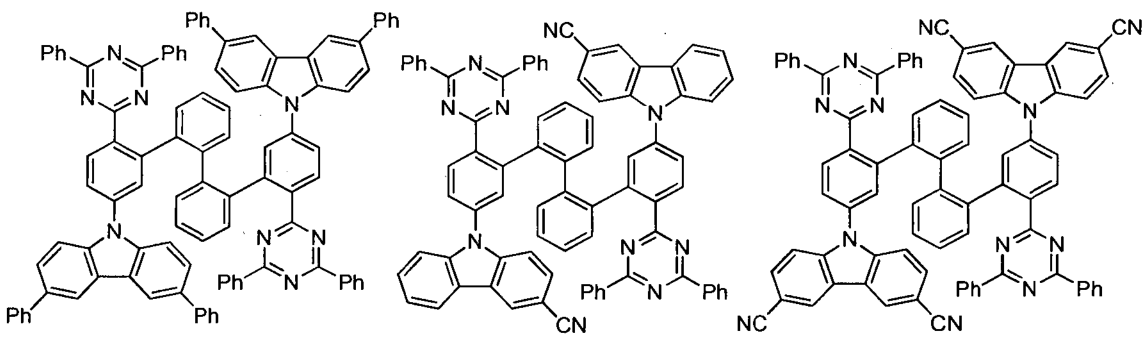

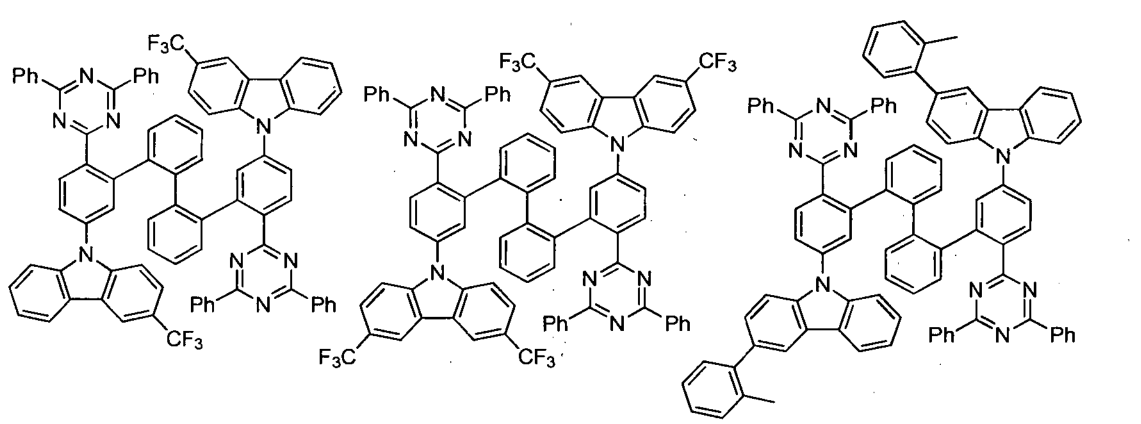

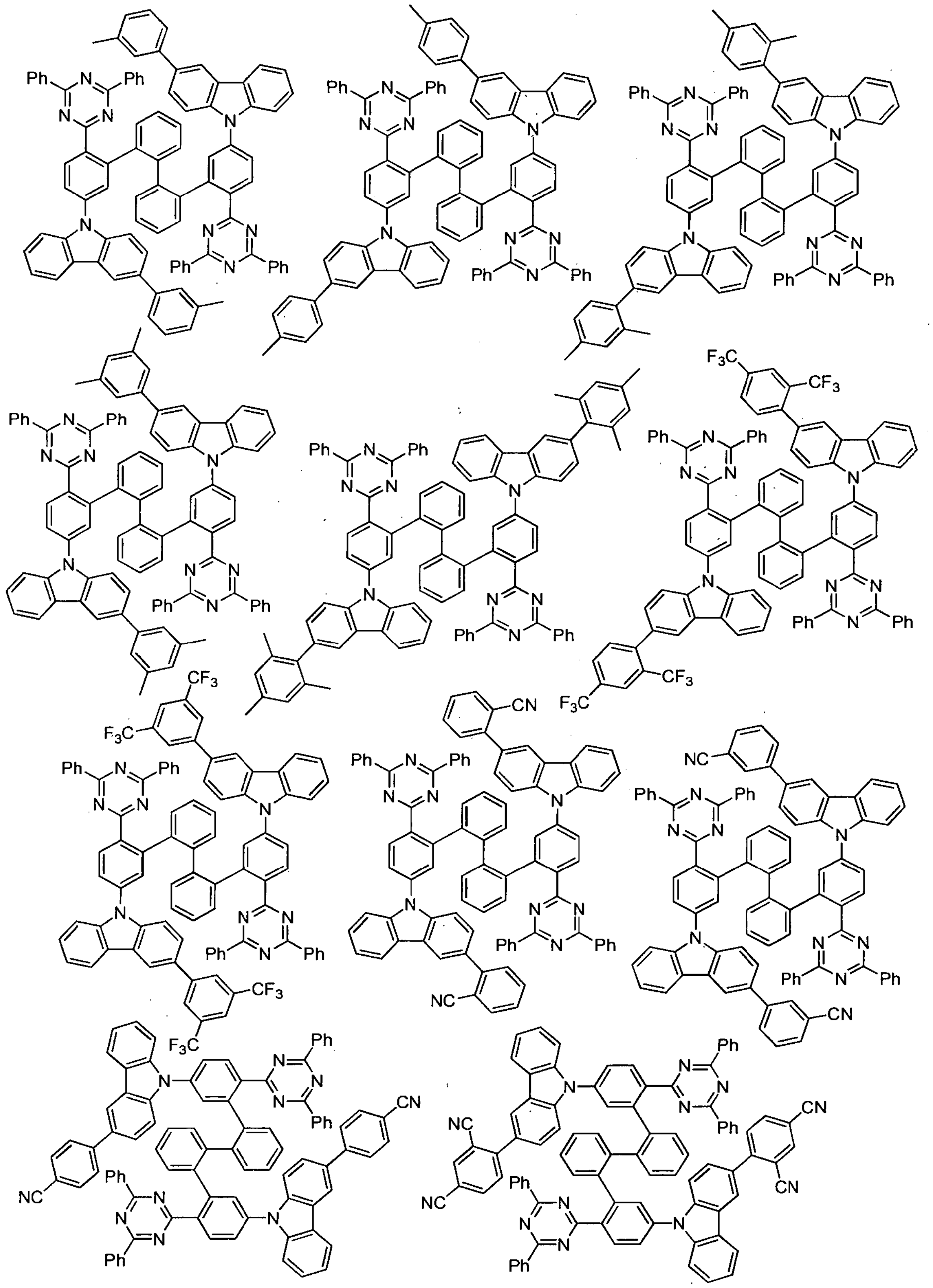

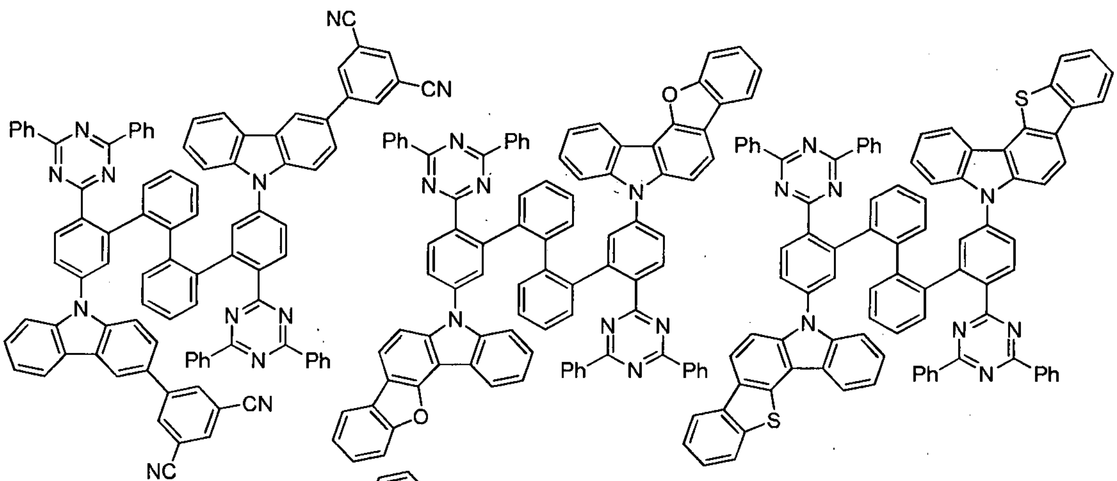

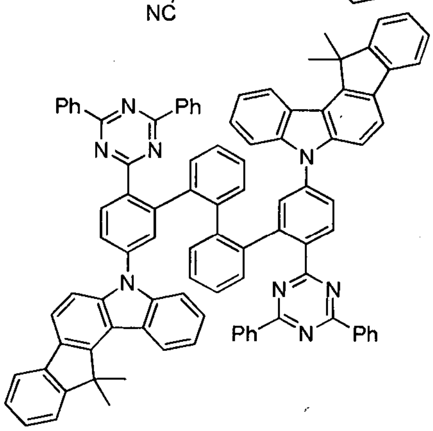

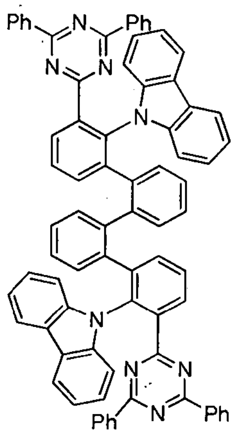

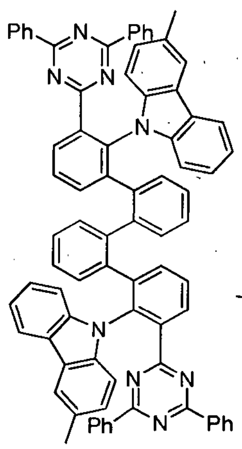

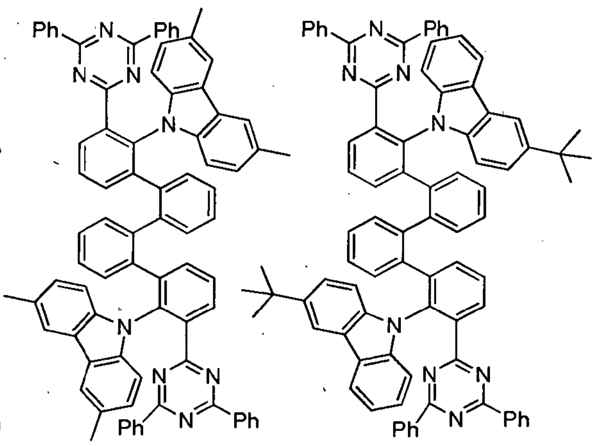

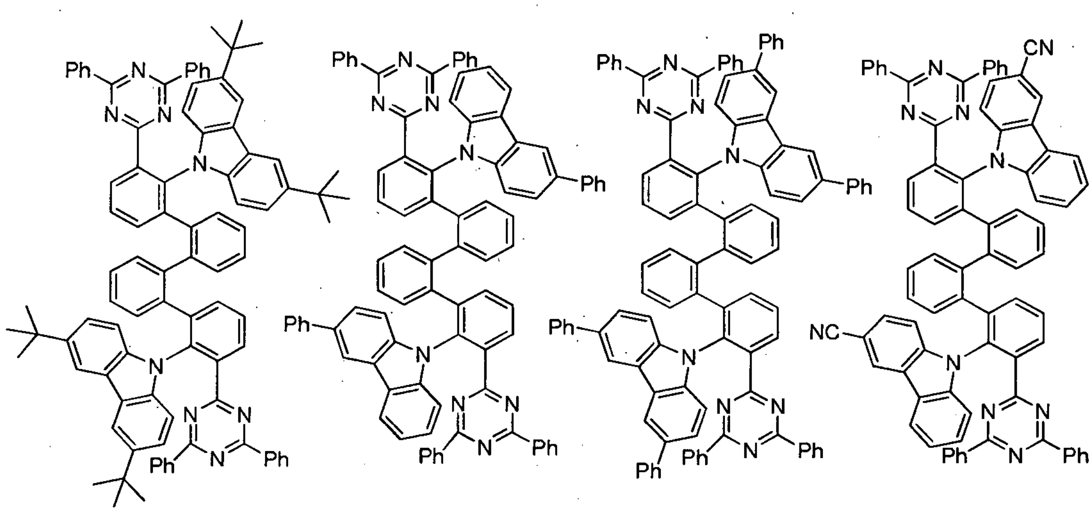

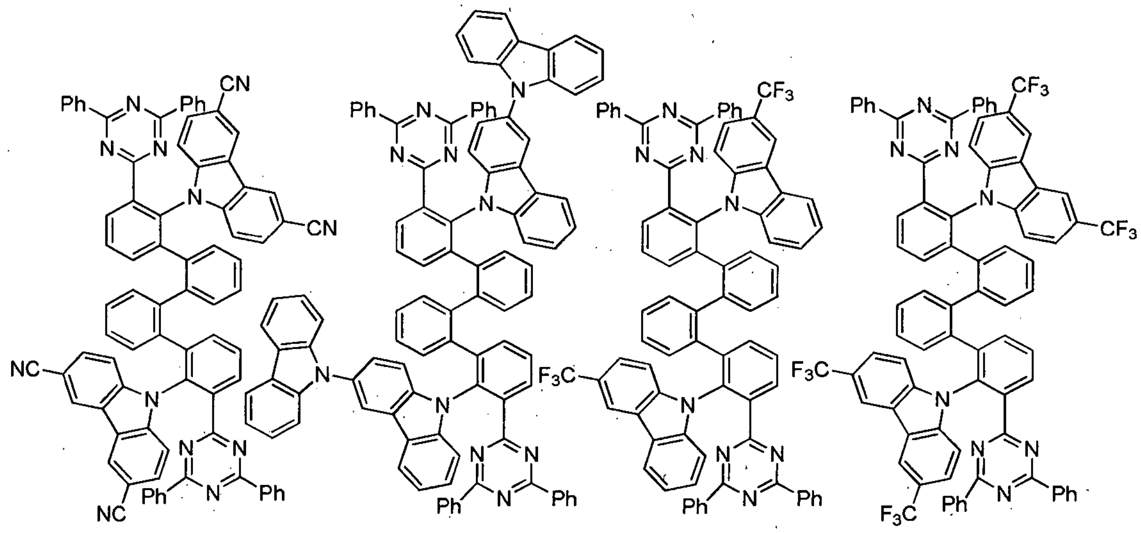

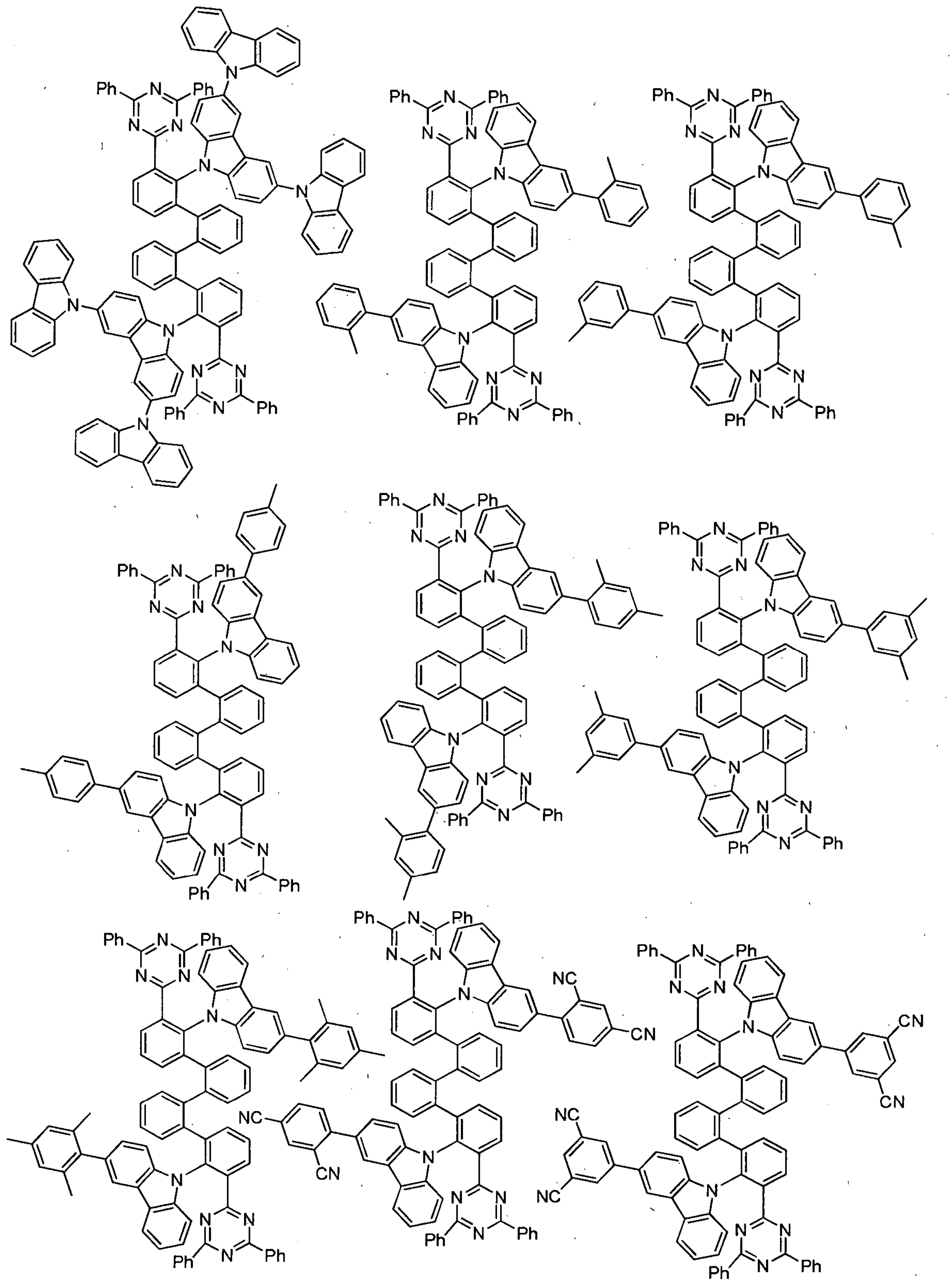

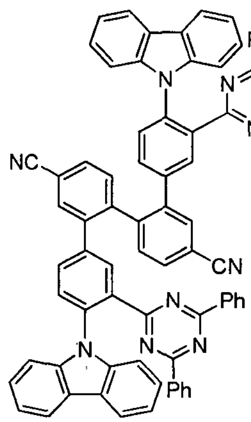
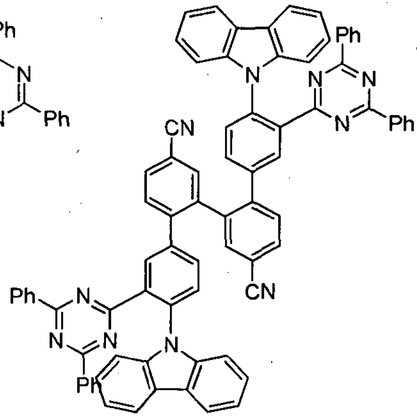
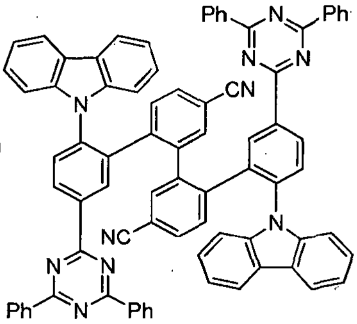
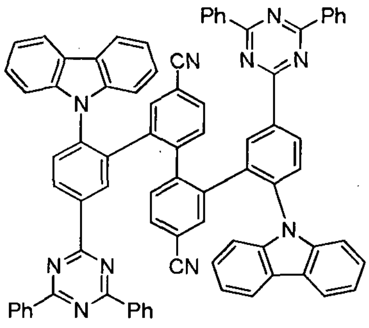
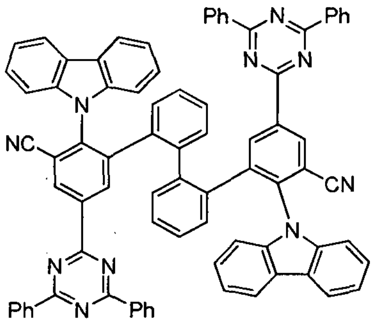
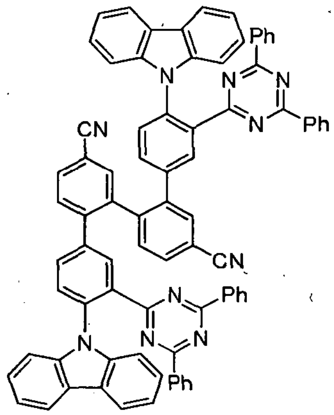
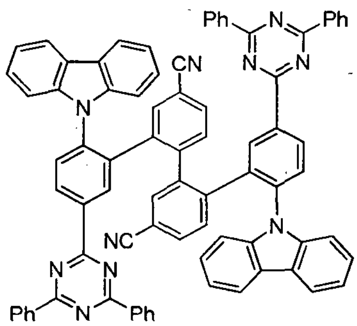

## Figures

The figures show:

Figure 1 Emission spectrum of example **1** (10% by weight) in PMMA.
Figure 2 Emission spectrum of example **2** (10% by weight) in PMMA.

## Claims

**1.** An organic molecule, consisting of

- one first chemical moiety formula I

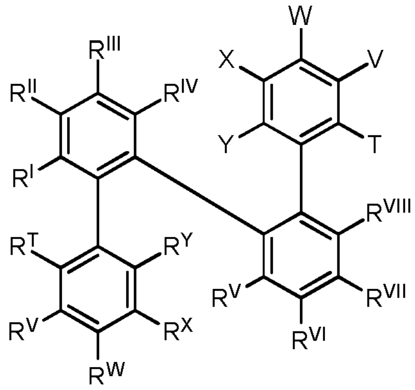

Formula I

and

- two second chemical moieties, each independently from another being of formula II,

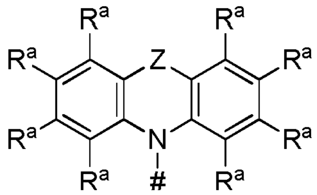

Formula II

wherein the first chemical moiety is linked to each of the two second chemical moieties via a single bond; wherein

$R^T$, $R^V$, $R^W$, $R^X$, and $R^Y$ is the binding site of a single bond linking the first chemical moiety to one of the two second chemical moieties or is selected from the group consisting of $R^1$ and $R^A$;

$R^A$ is a structure of formula A:

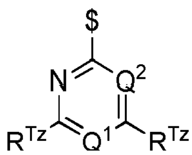

Formula A

wherein

$Q^1$ and $Q^2$ are selected from the group consisting of N and C-$R^{Py}$; and

$ represents the binding site of a single bond linking the structure of formula $R^A$ to the structure of formula 1;

T, V, W, X, and Y is the binding site of a single bond linking the first chemical moiety to one of the two second chemical moieties or is selected from the group consisting of $R^2$ and $R^B$;

$R^B$ is a structure of formula B:

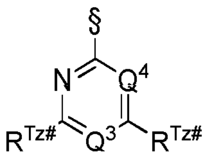

Formula B

wherein

$Q^3$ and $Q^4$ are selected from the group consisting of N and C-$R^{Py\#}$;
§ represents the binding site of a single bond linking the structure of formula $R^B$ to the structure of formula 1;
# represents the binding site of a single bond linking each of the second chemical moieties to the first chemical moiety;
Z is at each occurrence independently from another selected from the group consisting of a direct bond, $CR^3R^4$, $C=CR^3R^4$, C=O, $C=NR^3$, $NR^3$, O, $SiR^3R^4$, S, S(O) and $S(O)_2$;
$R^1$, $R^2$ is at each occurrence independently from another selected from the group consisting of:

hydrogen, deuterium,
F, Cl, Br, I,
CN,
$CF_3$,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$;

$R^I$, $R^{II}$, $R^{III}$, $R^{IV}$, $R^V$, $R^{VI}$, $R^{VII}$, $R^{VIII}$ is at each occurrence independently from another selected from the group consisting of:

hydrogen, deuterium,
F, Cl, Br, I,
CN,
$CF_3$,
$C_1$-$C_5$-alkyl,
wherein one or more hydrogen atoms are optionally substituted by deuterium;
$C_2$-$C_8$-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents $R^6$;

$R^{Tz}$ is at each occurrence independently from another selected from the group consisting of:

hydrogen, deuterium,
$C_1$-$C_{10}$-alkyl,
wherein one or more hydrogen atoms are optionally substituted by deuterium;
$C_6$-$C_{18}$-aryl,
which is optionally substituted with one or more substituents $R^5$, and
$C_3$-$C_{17}$-heteroaryl,
which is optionally substituted with one or more substituents $R^5$;

$R^{Tz\#}$ is at each occurrence independently from another selected from the group consisting of:

hydrogen, deuterium,
$C_1$-$C_{10}$-alkyl,
wherein one or more hydrogen atoms are optionally substituted by deuterium;
$C_6$-$C_{18}$-aryl,
which is optionally substituted with one or more substituents $R^5$, and
$C_3$-$C_{17}$-heteroaryl,
which is optionally substituted with one or more substituents $R^5$;

$R^{Py}$ is at each occurrence independently from another selected from the group consisting of:

hydrogen, deuterium,
$C_1$-$C_{10}$-alkyl,
wherein one or more hydrogen atoms are optionally substituted by deuterium;
$C_6$-$C_{18}$-aryl,
which is optionally substituted with one or more substituents $R^5$, and $C_3$-$C_{17}$-heteroaryl,
which is optionally substituted with one or more substituents $R^5$;

$R^{Py\#}$ is at each occurrence independently from another selected from the group consisting of:

hydrogen, deuterium,
$C_1$-$C_{10}$-alkyl,
wherein one or more hydrogen atoms are optionally substituted by deuterium;
$C_6$-$C_{18}$-aryl,
which is optionally substituted with one or more substituents $R^5$, and
$C_3$-$C_{17}$-heteroaryl,
which is optionally substituted with one or more substituents $R^5$;

$R^a$, $R^3$ and $R^4$ is at each occurrence independently from another selected from the group consisting of: hydrogen, deuterium, $N(R^5)_2$, $OR^5$, $SR^5$, $Si(R^5)_3$, $B(OR^5)_2$, $OSO_2R^5$, $CF_3$, CN, F, Br, I,

$C_1$-$C_{40}$-alkyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$;
$C_3$-$C_{57}$-heteroaryl,
which is optionally substituted with one or more substituents $R^5$; and
a mono- or polycyclic, aliphatic, aromatic and/or benzo-fused ring system formed by ring-closure with one or more of the other substituents selected from the group consisting of $R^a$, $R^3$, $R^4$ and $R^5$;

$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,

$C_1$-$C_{40}$-alkyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$;
$C_3$-$C_{57}$-heteroaryl,
which is optionally substituted with one or more substituents $R^6$; and
a mono- or polycyclic, aliphatic, aromatic and/or benzo-fused ring system formed by ring-closure with one or more of the other substituents selected from the group consisting of $R^a$, $R^3$, $R^4$ and $R^5$;

$R^6$ is at each occurrence independently from another selected from the group consisting of:

hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;

$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$, and
$N(C_3$-$C_{17}$-heteroaryl$)(C_6$-$C_{18}$-aryl$)$;

wherein the substituents $R^a$, $R^3$, $R^4$ or $R^5$ independently from each other optionally form a mono- or polycyclic, aliphatic, aromatic and/or benzo-fused ring system together with one or more of the other substituents $R^a$, $R^3$, $R^4$ and/or $R^5$;
wherein

exactly one substituent selected from the group consisting of $R^T$, $R^V$, $R^W$, $R^X$, and $R^Y$ is $R^A$,
exactly one substituent selected from the group consisting of T, V, W, X, and Y is $R^B$,
at least one of the group consisting of $Q^1$ and $Q^2$ is N;
at least one of the group consisting of $Q^3$ and $Q^4$ is N;
exactly one substituent selected from the group consisting of $R^T$, $R^V$, $R^W$, $R^X$, and $R^Y$ represents the binding site of a single bond linking the first chemical moiety and one of the two second chemical moieties, and
exactly one substituent selected from the group consisting of T, V, W, X and Y represents the binding site of a single bond linking the first chemical moiety and one of the two second chemical moieties.

**2.** The organic molecule according to claim 1, wherein

$R^{Tz}$ and $R^{Tz\#}$ is independently from each other selected from the group consisting of H, methyl and phenyl, which is optionally substituted with one or more substituents $R^6$, and
$R^{Py}$ and $R^{Py\#}$ is hydrogen.

**3.** The organic molecule according to claim 1 or 2, wherein $R^1$, $R^2$, $R^I$, $R^{II}$, $R^{III}$, $R^{IV}$, $R^V$, $R^{VI}$, $R^{VII}$, and $R^{VIII}$ is independently from each other at each occurrence independently selected from another selected from the group consisting of hydrogen, deuterium, and CN.

**4.** The organic molecule according to one or more of claims 1 to 3, wherein $Q^1$, $Q^2$, $Q^3$, and $Q^4$ is N.

**5.** The organic molecule according to one or more of claims 1 to 4, wherein the first chemical moiety is a structure of formula Iaaaa:

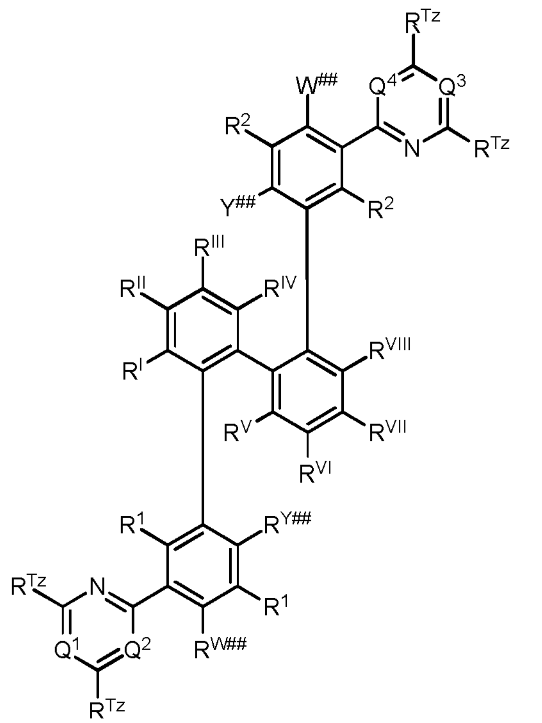

Formula Iaaaa

wherein

$R^{W\#\#}$ and $R^{Y\#\#}$ is selected from the group consisting of $R^1$ and the binding site of a single bond linking the first chemical moiety and one of the two second chemical moieties;
$W^{\#\#}$ and $Y^{\#\#}$ is selected from the group consisting of $R^2$ and the binding site of a single bond linking the first chemical moiety and one of the two second chemical moieties;
exactly one substituent selected from the group consisting of $R^{W\#\#}$ and $R^{Y\#\#}$ represents the binding site of a single bond linking the first chemical moiety and one of the two second chemical moieties,
exactly one substituent selected from the group consisting of $W^{\#\#}$ and $Y^{\#\#}$ represents the binding site of a single bond linking the first chemical moiety and one of the two second chemical moieties;
wherein apart from that the definitions of claim 1 apply.

**6.** The organic molecule according to one or more of claims 1 to 5, wherein the two second chemical moieties, each at each occurrence independently from another are a structure of formula IIa:

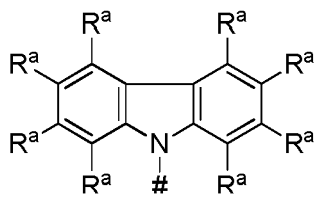

Formula IIa

wherein
$R^a$ is at each occurrence independently from another selected from the group consisting of

- H,
- Me, $^i$Pr, $^t$Bu, CN, $CF_3$,
- Ph, which is optionally substituted with one or more substituents independently from each other selected from the group consisting of Me, $^i$Pr, $^t$Bu, CN, $CF_3$ and Ph;
- pyridinyl, which is optionally substituted with one or more substituents independently from each other selected

from the group consisting of Me, $^{i}Pr$, $^{t}Bu$, CN, $CF_3$ and Ph;
- pyrimidinyl, which is optionally substituted with one or more substituents independently from each other selected from the group consisting of Me, $^{i}Pr$, $^{t}Bu$, CN, $CF_3$ and Ph;
- carbazolyl, which is optionally substituted with one or more substituents independently from each other selected from the group consisting of Me, $^{i}Pr$, $^{t}Bu$, CN, $CF_3$ and Ph;
- triazinyl, which is optionally substituted with one or more substituents independently from each other selected from the group consisting of Me, $^{i}Pr$, $^{t}Bu$, CN, $CF_3$, and Ph; and
- $N(Ph)_2$.

**7.** The organic molecule according to one or more of claims 1 to 6, wherein the two second chemical moieties, each at each occurrence independently from another are a structure of formula IIb:

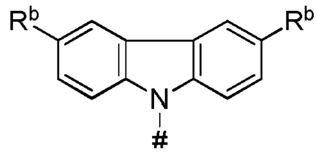

Formula IIb

wherein

$R^b$ is at each occurrence independently from another selected from the group consisting of

- H,
- Me, $^{i}Pr$, $^{t}Bu$, CN, $CF_3$,
- Ph, which is optionally substituted with one or more substituents independently from each other selected from the group consisting of Me, $^{i}Pr$, $^{t}Bu$, CN, $CF_3$ and Ph;
- pyridinyl, which is optionally substituted with one or more substituents independently from each other selected from the group consisting of Me, $^{i}Pr$, $^{t}Bu$, CN, $CF_3$ and Ph;
- pyrimidinyl, which is optionally substituted with one or more substituents independently from each other selected from the group consisting of Me, $^{i}Pr$, $^{t}Bu$, CN, $CF_3$ and Ph;
- carbazolyl, which is optionally substituted with one or more substituents independently from each other selected from the group consisting of Me, $^{i}Pr$, $^{t}Bu$, CN, $CF_3$ and Ph;
- triazinyl, which is optionally substituted with one or more substituents independently from each other selected from the group consisting of Me, $^{i}Pr$, $^{t}Bu$, CN, $CF_3$, and Ph; and
- $N(Ph)_2$.

and wherein apart from that the definitions of claim 1 apply.

**8.** The organic molecule according to one or more of claims 1 to 6, wherein the two second chemical moieties, each at each occurrence independently from another are a structure of formula IIc:

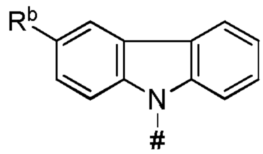

Formula IIc

wherein

$R^b$ is at each occurrence independently from another selected from the group consisting of

- H,
- Me, $^{i}Pr$, $^{t}Bu$, CN, $CF_3$,
- Ph, which is optionally substituted with one or more substituents independently from each other selected

from the group consisting of Me, $^{i}Pr$, $^{t}Bu$, CN, $CF_3$ and Ph;
- pyridinyl, which is optionally substituted with one or more substituents independently from each other selected from the group consisting of Me, $^{i}Pr$, $^{t}Bu$, CN, $CF_3$ and Ph;
- pyrimidinyl, which is optionally substituted with one or more substituents independently from each other selected from the group consisting of Me, $^{i}Pr$, $^{t}Bu$, CN, $CF_3$ and Ph;
- carbazolyl, which is optionally substituted with one or more substituents independently from each other selected from the group consisting of Me, $^{i}Pr$, $^{t}Bu$, CN, $CF_3$ and Ph;
- triazinyl, which is optionally substituted with one or more substituents independently from each other selected from the group consisting of Me, $^{i}Pr$, $^{t}Bu$, CN, $CF_3$, and Ph; and
- $N(Ph)_2$.

and wherein apart from that the definitions of claim 1 apply.

**9.** The organic molecule according to claim 7 or 8, wherein $R^b$ is at each occurrence independently from another selected from the group consisting of

- H,
- Me, $^{i}Pr$, $^{t}Bu$, CN, $CF_3$, and
- Ph.

**10.** Use of an organic molecule according to one or more of claims 1 to 9 as a luminescent emitter in an optoelectronic device.

**11.** The use according to claim 10, wherein the optoelectronic device is selected from the group consisting of:

• organic light-emitting diodes (OLEDs),
• light-emitting electrochemical cells,
• OLED-sensors,
• organic diodes,
• organic solar cells,
• organic transistors,
• organic field-effect transistors,
• organic lasers, and
• down-conversion elements.

**12.** A composition, comprising:

(a) an organic molecule according to one or more of claims 1 to 9 in the form of an emitter, and
(b) an emitter and/or host material, which differs from said organic molecule, and
(c) optionally, a dye and/or a solvent.

**13.** An optoelectronic device, comprising an organic molecule according to one or more of claims 1 to 9 or a composition according to claim 12, in particular in the form of a device selected from the group consisting of organic light-emitting diode (OLED), light-emitting electrochemical cell, OLED-sensor, organic diode, organic solar cell, organic transistor, organic field-effect transistor, organic laser, and down-conversion element.

**14.** The optoelectronic device according to claim 13, comprising

- a substrate,
- an anode, and
- a cathode, wherein the anode or the cathode are disposed on the substrate, and
- a light-emitting layer, which is arranged between the anode and the cathode and which comprises the organic molecule according to claims 1 to 9 or the composition according to claim 12.

**15.** A method for producing an optoelectronic device, wherein an organic molecule according to one or more of claims 1 to 9 or a composition according to claim 12 is used, in particular comprising the processing of the organic molecule by a vacuum evaporation method or from a solution.

**Patentansprüche**

**1.** Organisches Molekül, bestehend aus

- einer ersten chemischen Einheit mit einer Struktur der Formel I

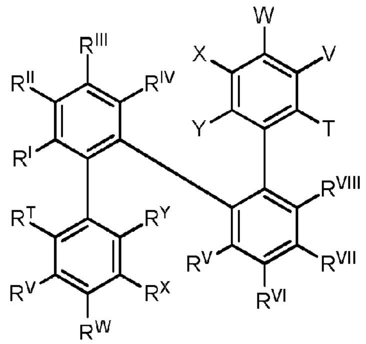

Formel I

und

zwei zweiten chemischen Einheiten, die unabhängig voneinander eine Struktur der Formel II

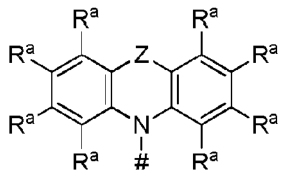

Formel II

aufweisen, wobei die erste chemische Einheit jeweils über eine Einfachbindung mit den zwei zweiten chemischen Einheiten verknüpft ist;

wobei

$R^T$, $R^V$, $R^W$, $R^X$ und $R^Y$ für die Bindungsstelle einer Einfachbindung, die die erste chemische Einheit mit einer der zwei zweiten chemischen Einheiten verknüpft, stehen oder aus der Gruppe bestehend aus $R^1$ und $R^A$ ausgewählt sind;

$R^A$ für eine Struktur der Formel A:

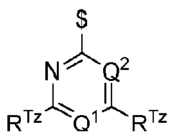

Formel A

steht, wobei

$Q^1$ und $Q^2$ aus der Gruppe bestehend aus N und C-$R^{Py}$ ausgewählt sind und

$ für die Bindungsstelle einer Einfachbindung, die die Struktur der Formel $R^A$ mit der Struktur der Formel 1 verknüpft, steht;

T, V, W, X und Y für die Bindungsstelle einer Einfachbindung, die die erste chemische Einheit mit einer der zwei zweiten chemischen Einheiten verknüpft, stehen oder aus der Gruppe bestehend aus $R^2$ und $R^B$ ausgewählt sind;

$R^B$ für eine Struktur der Formel B:

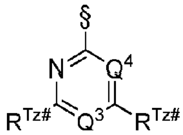

Formel B

steht, wobei

$Q^3$ und $Q^4$ aus der Gruppe bestehend aus N und C-$R^{Py\#}$ ausgewählt sind;
§ für die Bindungsstelle einer Einfachbindung, die die Struktur der Formel $R^B$ mit der Struktur der Formel 1 verknüpft, steht;
# für die Bindungsstelle einer Einfachbindung, die die zweiten chemischen Einheiten mit der ersten chemischen Einheit verknüpft, steht;
Z bei jedem Auftreten unabhängig voneinander aus der Gruppe bestehend aus einer direkten Bindung, $CR^3R^4$, $C=CR^3R^4$, C=O, $C=NR^3$, $NR^3$, O, $SiR^3R^4$, S, S(O) und $S(O)_2$ ausgewählt ist;
$R^1$ und $R^2$ bei jedem Auftreten unabhängig voneinander ausgewählt sind aus der Gruppe bestehend aus:

Wasserstoff, Deuterium,
F, Cl, Br, I,
CN,
$CF_3$,
$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkinyl,
wobei ein oder mehrere Wasserstoffatome optional durch Deuterium substituiert sind; und
$C_6$-$C_{18}$-Aryl,
das optional durch einen oder mehrere Substituenten $R^6$ substituiert ist;

$R^I$, $R^{II}$, $R^{III}$, $R^{IV}$, $R^V$, $R^{VI}$, $R^{VII}$ und $R^{VIII}$ bei jedem Auftreten unabhängig voneinander ausgewählt sind aus der Gruppe bestehend aus:

Wasserstoff, Deuterium,
F, Cl, Br, I,
CN,
$CF_3$,
$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkinyl,
wobei ein oder mehrere Wasserstoffatome optional durch Deuterium substituiert sind; und
$C_6$-$C_{18}$-Aryl,
das optional durch einen oder mehrere Substituenten $R^6$ substituiert ist;

$R^{Tz}$ bei jedem Auftreten unabhängig voneinander aus der Gruppe bestehend aus:

Wasserstoff, Deuterium,
$C_1$-$C_{10}$-Alkyl,
wobei ein oder mehrere Wasserstoffatome optional durch Deuterium substituiert sind;
$C_6$-$C_{18}$-Aryl,
das optional durch einen oder mehrere Substituenten $R^5$ substituiert ist; und
$C_3$-$C_{17}$-Heteroaryl,
das optional durch einen oder mehrere Substituenten $R^5$ substituiert ist;
ausgewählt ist;
$R^{Tz\#}$ bei jedem Auftreten unabhängig voneinander aus der Gruppe bestehend aus:

Wasserstoff, Deuterium,
$C_1$-$C_{10}$-Alkyl,
wobei ein oder mehrere Wasserstoffatome optional durch Deuterium substituiert sind;
$C_6$-$C_{18}$-Aryl,
das optional durch einen oder mehrere Substituenten $R^5$ substituiert ist; und
$C_3$-$C_{17}$-Heteroaryl,
das optional durch einen oder mehrere Substituenten $R^5$ substituiert ist;

ausgewählt ist;

$R^{Py}$ bei jedem Auftreten unabhängig voneinander aus der Gruppe bestehend aus:

Wasserstoff, Deuterium,
$C_1$-$C_{10}$-Alkyl,
wobei ein oder mehrere Wasserstoffatome optional durch Deuterium substituiert sind;
$C_6$-$C_{18}$-Aryl,
das optional durch einen oder mehrere Substituenten $R^5$ substituiert ist; und
$C_3$-$C_{17}$-Heteroaryl,
das optional durch einen oder mehrere Substituenten $R^5$ substituiert ist;

ausgewählt ist;

$R^{Py\#}$ bei jedem Auftreten unabhängig voneinander aus der Gruppe bestehend aus:

Wasserstoff, Deuterium,
$C_1$-$C_{10}$-Alkyl,
wobei ein oder mehrere Wasserstoffatome optional durch Deuterium substituiert sind;
$C_6$-$C_{18}$-Aryl,
das optional durch einen oder mehrere Substituenten $R^5$ substituiert ist; und
$C_3$-$C_{17}$-Heteroaryl,
das optional durch einen oder mehrere Substituenten $R^5$ substituiert ist;

ausgewählt ist;

$R^a$, $R^3$ und $R^4$ bei jedem Auftreten unabhängig voneinander aus der Gruppe bestehend aus:

Wasserstoff, Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,
das optional durch einen oder mehrere Substituenten $R^5$ substituiert ist und
wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Alkoxy,

das optional durch einen oder mehrere Substituenten $R^5$ substituiert ist und
wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Thioalkoxy,

das optional durch einen oder mehrere Substituenten $R^5$ substituiert ist und
wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkenyl,

das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkinyl,

das optional durch einen oder mehrere Substituenten $R^5$ substituiert ist und wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_6$-$C_{60}$-Aryl,
das optional durch einen oder mehrere Substituenten $R^5$ substituiert ist; und
$C_3$-$C_{57}$-Heteroaryl,
das optional durch einen oder mehrere Substituenten $R^5$ substituiert ist; und
einem durch Ringschluss mit einem oder mehreren der anderen Substituenten aus der Gruppe bestehend aus $R^a$, $R^3$, $R^4$ und $R^5$ gebildeten mono- oder polycyclischen, aliphatischen, aromatischen und/oder benzoannelierten Ringsystem

ausgewählt sind;
$R^5$ bei jedem Auftreten unabhängig voneinander ausgewählt ist aus der Gruppe bestehend aus Wasserstoff, Deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-Alkyl,

das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_1$-$C_{40}$-Alkoxy,

das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_1$-$C_{40}$-Thioalkoxy,

das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_2$-$C_{40}$-Alkenyl,

das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_2$-$C_{40}$-Alkinyl,

das optional durch einen oder mehrere Substituenten $R^6$ substituiert ist und wobei eine oder mehrere nicht benachbarte $CH_2$-Gruppen optional durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_6$-$C_{60}$-Aryl,
das optional durch einen oder mehrere Substituenten $R^6$ substituiert ist; und
$C_3$-$C_{57}$-Heteroaryl,
das optional durch einen oder mehrere Substituenten $R^6$ substituiert ist; und
einem durch Ringschluss mit einem oder mehreren der anderen Substituenten aus der Gruppe bestehend

aus $R^a$, $R^3$, $R^4$ und $R^5$ gebildeten mono- oder polycyclischen, aliphatischen, aromatischen und/oder benzoannelierten Ringsystem
$R^6$ bei jedem Auftreten unabhängig voneinander ausgewählt ist aus der Gruppe bestehend aus:

Wasserstoff, Deuterium, OPh, $CF_3$, CN, F,
$C_1$-$C_5$-Alkyl,
wobei ein oder mehrere Wasserstoffatome optional unabhängig voneinander durch Deuterium, CN, $CF_3$ oder F ersetzt sind;
$C_1$-$C_5$-Alkoxy,
wobei ein oder mehrere Wasserstoffatome optional unabhängig voneinander durch Deuterium, CN, $CF_3$ oder F ersetzt sind;
$C_1$-$C_5$-Thioalkoxy,
wobei ein oder mehrere Wasserstoffatome optional unabhängig voneinander durch Deuterium, CN, $CF_3$ oder F ersetzt sind;
$C_2$-$C_5$-Alkenyl,
wobei ein oder mehrere Wasserstoffatome optional unabhängig voneinander durch Deuterium, CN, $CF_3$ oder F ersetzt sind;
$C_2$-$C_5$-Alkinyl,
wobei ein oder mehrere Wasserstoffatome optional unabhängig voneinander durch Deuterium, CN, $CF_3$ oder F ersetzt sind;
$C_6$-$C_{18}$-Aryl,
das optional durch einen oder mehrere $C_1$-$C_5$-Alkylsubstituenten substituiert ist;
$C_3$-$C_{17}$-Heteroaryl,
das optional durch einen oder mehrere $C_1$-$C_5$-Alkylsubstituenten substituiert ist;
$N(C_6$-$C_{18}$-$Aryl)_2$;
$N(C_3$-$C_{17}$-$Heteroaryl)_2$,
und $N(C_3$-$C_{17}$-Heteroaryl)($C_6$-$C_{18}$-aryl)

wobei die Substituenten $R^a$, $R^3$, $R^4$ oder $R^5$ unabhängig voneinander optional mit einem oder mehreren Substituenten $R^a$, $R^3$, $R^4$ und/oder $R^5$ ein mono- oder polycyclisches, aliphatisches, aromatisches und/oder benzoanneliertes Ringsystem bilden;
wobei

genau ein Substituent aus der Gruppe bestehend aus $R^T$, $R^V$, $R^W$, $R^X$ und $R^Y$ für $R^A$ steht;
genau ein Substituent aus der Gruppe bestehend aus T, V, W, X und Y für $R^B$ steht;
mindestens eines der Gruppe bestehend aus $Q^1$ und $Q^2$ für N steht;
mindestens eines der Gruppe bestehend aus $Q^3$ und $Q^4$ für N steht;
genau ein Substituent aus der Gruppe bestehend aus $R^T$, $R^V$, $R^W$, $R^X$ und $R^Y$ für die Bindungsstelle einer Einfachbindung, die die erste chemische Einheit und eine der zwei zweiten chemischen Einheiten verknüpft, steht und
genau ein Substituent aus der Gruppe bestehend aus T, V, W, X und Y für die Bindungsstelle einer Einfachbindung steht, die die erste chemische Einheit und eine der zwei zweiten chemischen Einheiten verknüpft.

**2.** Organisches Molekül nach Anspruch 1, wobei

$R^{Tz}$ und $R^{Tz\#}$ unabhängig voneinander ausgewählt sind aus der Gruppe bestehend aus H, Methyl und Phenyl, das optional durch einen oder mehrere Substituenten $R^6$ substituiert ist, und
$R^{Py}$ und $R^{Py\#}$ für Wasserstoff stehen.

**3.** Organisches Molekül nach Anspruch 1 oder 2, wobei $R^1$, $R^2$, $R^I$, $R^{II}$, $R^{III}$, $R^{IV}$, $R^V$, $R^{VI}$, $R^{VII}$ und $R^{VIII}$ unabhängig voneinander bei jedem Auftreten unabhängig voneinander ausgewählt sind aus der Gruppe bestehend aus Wasserstoff, Deuterium und CN.

**4.** Organisches Molekül nach einem oder mehreren der Ansprüche 1 bis 3, wobei $Q^1$, $Q^2$, $Q^3$ und $Q^4$ für N stehen.

**5.** Organisches Molekül nach einem oder mehreren der Ansprüche 1 bis 4, wobei die erste chemischenEinheit um eine Struktur der Formel Iaaaa aufweist:

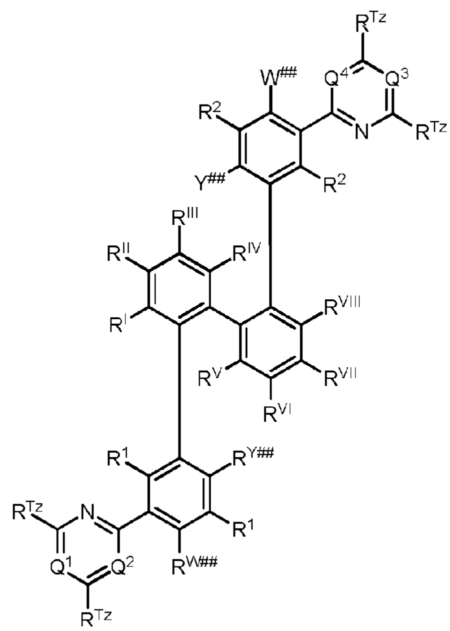

Formel Iaaaa

wobei

$R^{W\#\#}$ und $R^{Y\#\#}$ aus der Gruppe bestehend aus $R^1$ und der Bindungsstelle einer Einfachbindung, die die erste chemische Einheit und eine der zwei zweiten chemischen Einheiten verknüpft, steht;
$W^{\#\#}$ und $Y^{\#\#}$ aus der Gruppe bestehend aus $R^2$ und der Bindungsstelle einer Einfachbindung, die die erste chemische Einheit und eine der zwei zweiten chemischen Einheiten verknüpft, steht;
genau ein Substituent aus der Gruppe bestehend aus $R^{W\#\#}$ und $R^{Y\#\#}$ für die Bindungsstelle einer Einfachbindung, die die erste chemische Einheit und eine der zwei zweiten chemischen Einheiten verknüpft, steht;
genau ein Substituent aus der Gruppe bestehend aus $W^{\#\#}$ und $Y^{\#\#}$ für die Bindungsstelle einer Einfachbindung, die die erste chemische Einheit und eine der zwei zweiten chemischen Einheiten verknüpft, steht;
wobei davon abgesehen die Definitionen in Anspruch 1 gelten.

**6.** Organisches Molekül nach einem oder mehreren der Ansprüche 1 bis 5, wobei die zwei zweiten chemischen Einheiten bei jedem Auftreten unabhängig voneinander eine Struktur der Formel IIa:

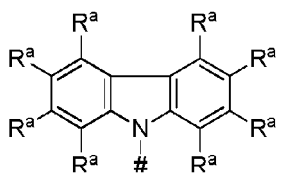

Formel IIa

aufweisen, wobei

$R^a$ bei jedem Auftreten unabhängig voneinander ausgewählt ist aus der Gruppe bestehend aus

- H,
- Me, $^iPr$, $^tBu$, CN, $CF_3$,
- Ph, das optional durch einen oder mehrere Substituenten, die unabhängig voneinander aus der Gruppe bestehend aus Me, $^iPr$, $^tBu$, CN, $CF_3$ und Ph ausgewählt sind, substituiert ist;
- Pyridinyl, das optional durch einen oder mehrere Substituenten, die unabhängig voneinander aus der Gruppe bestehend aus Me, $^iPr$, $^tBu$, CN, $CF_3$ und Ph ausgewählt sind, substituiert ist;
- Pyrimidinyl, das optional durch einen oder mehrere Substituenten, die unabhängig voneinander aus der Gruppe bestehend aus Me, $^iPr$, $^tBu$, CN, $CF_3$ und Ph ausgewählt sind, substituiert ist;

- Carbazolyl, das optional durch einen oder mehrere Substituenten, die unabhängig voneinander aus der Gruppe bestehend aus Me, $^{i}$Pr, $^{t}$Bu, CN, $CF_3$ und Ph ausgewählt sind, substituiert ist;
- Triazinyl, das optional durch einen oder mehrere Substituenten, die unabhängig voneinander aus der Gruppe bestehend aus Me, $^{i}$Pr, $^{t}$Bu, CN, $CF_3$ und Ph ausgewählt sind, substituiert ist;

und

- $N(Ph)_2$.

**7.** Organisches Molekül nach einem oder mehreren der Ansprüche 1 bis 6, wobei die zwei zweiten chemischen Einheiten bei jedem Auftreten unabhängig voneinander eine Struktur der Formel IIb:

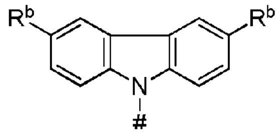

Formel IIb

aufweisen, wobei

$R^b$ bei jedem Auftreten unabhängig voneinander aus der Gruppe bestehend aus

- H,
- Me, $^{i}$Pr, $^{t}$Bu, CN, $CF_3$,
- Ph, das optional durch einen oder mehrere Substituenten, die unabhängig voneinander aus der Gruppe bestehend aus Me, $^{i}$Pr, $^{t}$Bu, CN, $CF_3$ und Ph ausgewählt sind, substituiert ist;
- Pyridinyl, das optional durch einen oder mehrere Substituenten, die unabhängig voneinander aus der Gruppe bestehend aus Me, $^{i}$Pr, $^{t}$Bu, CN, $CF_3$ und Ph ausgewählt sind, substituiert ist;
- Pyrimidinyl, das optional durch einen oder mehrere Substituenten, die unabhängig voneinander aus der Gruppe bestehend aus Me, $^{i}$Pr, $^{t}$Bu, CN, $CF_3$ und Ph ausgewählt sind, substituiert ist;
- Carbazolyl, das optional durch einen oder mehrere Substituenten, die unabhängig voneinander aus der Gruppe bestehend aus Me, $^{i}$Pr, $^{t}$Bu, CN, $CF_3$ und Ph ausgewählt sind, substituiert ist;
- Triazinyl, das optional durch einen oder mehrere Substituenten, die unabhängig voneinander aus der Gruppe bestehend aus Me, $^{i}$Pr, $^{t}$Bu, CN, $CF_3$ und Ph ausgewählt sind, substituiert ist;

und

- $N(Ph)_2$

ausgewählt ist
und wobei davon abgesehen die Definitionen von Anspruch 1 gelten.

**8.** Organisches Molekül nach einem oder mehreren der Ansprüche 1 bis 6, wobei die zwei zweiten chemischen Einheiten bei jedem Auftreten unabhängig voneinander eine Struktur der Formel IIc:

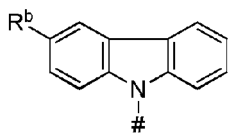

Formel IIc

aufweisen, wobei

$R^b$ bei jedem Auftreten unabhängig voneinander aus der Gruppe bestehend aus

- H,

- Me, $^{i}Pr$, $^{t}Bu$, CN, $CF_3$,
- Ph, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
- Pyridinyl, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
- Pyrimidinyl, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
- Carbazolyl, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;
- Triazinyl, das optional durch einen oder mehrere Substituenten, die unabhängig voneinander aus der Gruppe bestehend aus Me, $^{i}Pr$, $^{t}Bu$, CN, $CF_3$ und Ph ausgewählt sind, substituiert ist;

und

- $N(Ph)_2$

ausgewählt ist
und wobei davon abgesehen die Definitionen von Anspruch 1 gelten.

**9.** Organisches Molekül nach Anspruch 7 oder 8, wobei $R^b$ bei jedem Auftreten unabhängig voneinander ausgewählt ist aus der Gruppe bestehend aus

H,
Me, $^{i}Pr$, $^{t}Bu$, CN, $CF_3$ und
Ph.

**10.** Verwendung eines organischen Moleküls nach einem oder mehreren der Ansprüche 1 bis 9 als Lumineszenzemitter in einer optoelektronischen Vorrichtung.

**11.** Verwendung nach Anspruch 10, wobei die optoelektronische Vorrichtung ausgewählt ist aus der Gruppe bestehend aus:

• organischen lichtemittierenden Dioden (OLED),
• lichtemittierenden elektrochemischen Zellen,
• OLED-Sensoren,
• organischen Dioden,
• organischen Solarzellen,
• organischen Transistoren,
• organischen Feldeffekttransistoren,
• organischen Lasern und
• Abwärtswandlungselementen.

**12.** Zusammensetzung, aufweisend:

(a) ein organisches Molekül nach einem oder mehreren der Ansprüche 1 bis 9, insbesondere in der Form eines Emitters und/oder eines Wirts und
(b) ein Emitter- und/oder Wirtmaterial, das sich von dem organischen Molekül unterscheidet und
(c) optional einen Farbstoff und/oder ein Lösungsmittel.

**13.** Optoelektronische Vorrichtung, aufweisend ein organisches Molekül nach einem oder mehreren der Ansprüche 1 bis 9 oder eine Zusammensetzung nach Anspruch 12, insbesondere in der Form einer Vorrichtung ausgewählt aus der Gruppe bestehend aus organischer lichtemittierender Diode (OLED), lichtemittierender elektrochemischer Zelle, OLED-Sensor, organischer Diode, organischer Solarzelle, organischem Transistor, organischem Feldeffekttransistor, organischem Laser und Abwärtswand lungselement.

**14.** Optoelektronische Vorrichtung nach Anspruch 13, aufweisend

- ein Substrat,

- eine Anode und
- eine Kathode, wobei die Anode oder die Kathode auf dem Substrat angeordnet ist, und
- mindestens eine lichtemittierende Schicht, die zwischen der Anode und der Kathode angeordnet ist und die das organische Molekül gemäß einem der Ansprüche 1 bis 9 oder eine Zusammensetzung nach Anspruch 12 aufweist.

**15.** Verfahren zur Herstellung einer optoelektronischen Vorrichtung, wobei ein organisches Molekül nach einem der Ansprüche 1 bis 9 oder eine Zusammensetzung nach Anspruch 12 verwendet wird, insbesondere aufweisend das Verarbeiten des organischen Moleküls mittels eines Vakuumverdampfungsverfahrens oder aus einer Lösung.

**Revendications**

**1.** Molécule organique, constituée

- d'un premier fragment chimique de formule I,

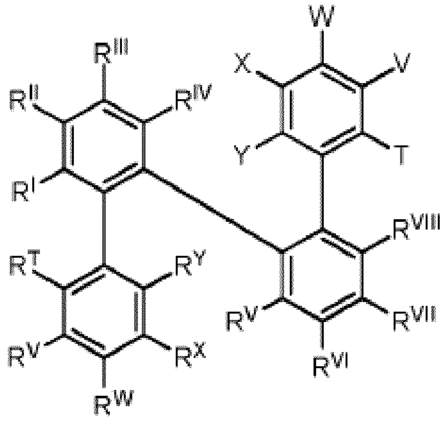

Formule I

et
- de deux deuxièmes fragments chimiques, chacun indépendamment l'un de l'autre étant de formule II,

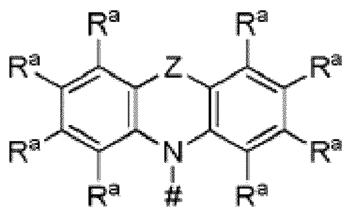

Formule II

le premier fragment chimique étant lié à chacun des deux deuxièmes fragments chimiques via une simple liaison ;

$R^T$, $R^V$, $R^W$, $R^X$, et $R^Y$ étant le site de liaison d'une simple liaison liant le premier fragment chimique à l'un des deux deuxièmes fragments chimiques ou étant choisi dans le groupe constitué par $R^1$ et $R^A$ ;
$R^A$ étant une structure de formule A :

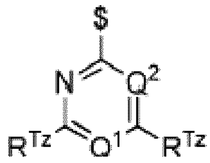

Formule A

$Q^1$ et $Q^2$ étant choisis dans le groupe constitué par N et C-$R^{Py}$; et
$ représentant le site de liaison d'une simple liaison liant la structure de formule $R^A$ à la structure de formule 1 ;
T, V, W, X, et Y étant le site de liaison d'une simple liaison liant le premier fragment chimique à l'un des deux deuxièmes fragments chimiques ou étant choisi dans le groupe constitué par $R^2$ et $R^B$ ;
$R^B$ étant la structure de formule B :

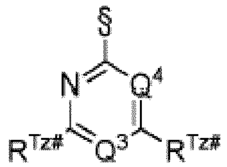

Formule B

$Q^3$ et $Q^4$ étant choisis dans le groupe constitué par N et C-$R^{Py\#}$ ;
$ représentant le site de liaison d'une simple liaison liant la structure de formule $R^B$ à la structure de formule 1 ;
# représentant le site de liaison d'une simple liaison liant chacun des deuxièmes fragments chimiques au premier fragment chimique ;
Z étant en chaque occurrence indépendamment l'un de l'autre choisi dans le groupe constitué par une liaison directe, $CR^3R^4$, $C{=}CR^3R^4$, C=O, $C{=}NR^3$, $NR^3$, O, $SiR^3R^4$, S, S(O) et $S(O)_2$ ;
$R^1$, $R^2$ étant en chaque occurrence indépendamment l'un de l'autre choisis dans le groupe constitué par :

hydrogène, deutérium,
F, Cl, Br, I,
CN,
$CF_3$,
$C_{1-5}$-alkyle,
un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcynyle,
un ou plusieurs atomes d'hydrogène étant éventuellement substitués par deutérium ; et
$C_{6-18}$-aryle,
qui est éventuellement substitué par un ou plusieurs substituants $R^6$ ;

$R^I$, $R^{II}$, $R^{III}$, $R^{IV}$, $R^V$, $R^{VI}$, $R^{VII}$, $R^{VIII}$ étant en chaque occurrence indépendamment les uns des autres choisis dans le groupe constitué par :

hydrogène, deutérium,
F, Cl, Br, I,
CN,
$CF_3$,
$C_{1-5}$-alkyle,
un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcynyle,
un ou plusieurs atomes d'hydrogène étant éventuellement substitués par deutérium ; et

$C_{6-18}$-aryle,
qui est éventuellement substitué par un ou plusieurs substituants $R^6$ ;

$R^{Tz}$ étant en chaque occurrence indépendamment l'un de l'autre choisi dans le groupe constitué par :

hydrogène, deutérium,
$C_{1-10}$-alkyle,
un ou plusieurs atomes d'hydrogène étant éventuellement substitués par deutérium ;
$C_{6-18}$-aryle,

qui est éventuellement substitué par un ou plusieurs substituants $R^5$, et $C_{3-17}$-hétéroaryle,
qui est éventuellement substitué par un ou plusieurs substituants $R^5$ ;

$R^{Tz\#}$ étant en chaque occurrence indépendamment l'un de l'autre choisi dans le groupe constitué par :

hydrogène, deutérium,
$C_{1-10}$-alkyle,
un ou plusieurs atomes d'hydrogène étant éventuellement substitués par deutérium ;
$C_{6-18}$-aryle,
qui est éventuellement substitué par un ou plusieurs substituants $R^5$, et
$C_{3-17}$-hétéroaryle,
qui est éventuellement substitué par un ou plusieurs substituants $R^5$ ;

$R^{Py}$ étant en chaque occurrence indépendamment l'un de l'autre choisi dans le groupe constitué par :

hydrogène, deutérium,
$C_{1-10}$-alkyle,
un ou plusieurs atomes d'hydrogène étant éventuellement substitués par deutérium ;
$C_{6-18}$-aryle,

qui est éventuellement substitué par un ou plusieurs substituants $R^5$, et $C_{3-17}$-hétéroaryle,
qui est éventuellement substitué par un ou plusieurs substituants $R^5$ ;

$R^{Py\#}$ étant en chaque occurrence indépendamment l'un de l'autre choisi dans le groupe constitué par :

hydrogène, deutérium,
$C_{1-10}$-alkyle,
un ou plusieurs atomes d'hydrogène étant éventuellement substitués par deutérium ;
$C_{6-18}$-aryle,

qui est éventuellement substitué par un ou plusieurs substituants $R^5$, et $C_{3-17}$-hétéroaryle,
qui est éventuellement substitué par un ou plusieurs substituants $R^5$ ;

$R^a$, $R^3$ et $R^4$ étant en chaque occurrence indépendamment les uns des autres choisis dans le groupe constitué par : hydrogène, deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,
qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{1-40}$-alcoxy,
qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{1-40}$-thioalcoxy,
qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{2-40}$-alcényle,

qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{2-40}$-alcynyle,
qui est éventuellement substitué par un ou plusieurs substituants $R^5$ et un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S, ou $CONR^5$;
$C_{6-60}$-aryle,
qui est éventuellement substitué par un ou plusieurs substituants $R^5$ ;
$C_{3-57}$-hétéroaryle,
qui est éventuellement substitué par un ou plusieurs substituants $R^5$ ; et un système cyclique monocyclique ou polycyclique, aliphatique, aromatique et/ou benzo-condensé formé par fermeture de cycle avec un ou plusieurs des autres substituants choisis dans le groupe constitué par $R^a$, $R^3$, $R^4$ et $R^5$ ;

$R^5$ étant en chaque occurrence indépendamment l'un de l'autre choisi dans le groupe constitué par hydrogène, deutérium, $N(R^6)_2$, $OR^6$, $Si(R^6)_3$, $B(OR^6)_2$, $OSO_2R^6$, $CF_3$, CN, F, Br, I,
$C_{1-40}$-alkyle,

qui est éventuellement substitué par un ou plusieurs substituants $R^6$ et
un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;

$C_{1-40}$-alcoxy,
qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;
$C_{1-40}$-thioalcoxy,
qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;
$C_{2-40}$-alcényle,
qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;
$C_{2-40}$-alcynyle,
qui est éventuellement substitué par un ou plusieurs substituants $R^6$ et un ou plusieurs groupes $CH_2$ non adjacents étant éventuellement substitués par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S, ou $CONR^6$;
$C_{6-60}$-aryle,
qui est éventuellement substitué par un ou plusieurs substituants $R^6$ ;
$C_{3-57}$-hétéroaryle,
qui est éventuellement substitué par un ou plusieurs substituants $R^6$ ; et un système cyclique monocyclique ou polycyclique, aliphatique, aromatique et/ou benzo-condensé formé par fermeture de cycle avec un ou plusieurs des autres substituants choisis dans le groupe constitué par $R^a$, $R^3$, $R^4$ et $R^5$ ;
$R^6$ étant en chaque occurrence indépendamment l'un de l'autre choisi dans le groupe constitué par hydrogène, deutérium, OPh, $CF_3$, CN, F,

$C_{1-5}$-alkyle,
un ou plusieurs atomes d'hydrogène étant éventuellement, indépendamment les uns des autres, substitués par deutérium, CN, $CF_3$, ou F ;
$C_{1-5}$-alcoxy,
un ou plusieurs atomes d'hydrogène étant éventuellement, indépendamment les uns des autres, substitués par deutérium, CN, $CF_3$, ou F ;
$C_{1-5}$-thioalcoxy,
un ou plusieurs atomes d'hydrogène étant éventuellement, indépendamment les uns des autres, substitués par deutérium, CN, $CF_3$, ou F ;
$C_{2-5}$-alcényle,
un ou plusieurs atomes d'hydrogène étant éventuellement, indépendamment les uns des autres, subs-

titués par deutérium, CN, $CF_3$, ou F ;
$C_{2-5}$-alcynyle,
un ou plusieurs atomes d'hydrogène étant éventuellement, indépendamment les uns des autres, substitués par deutérium, CN, $CF_3$, ou F ;
$C_{6-18}$-aryle,
qui est éventuellement substitué par un ou plusieurs substituants $C_{1-5}$-alkyle ;
$C_{3-17}$-hétéroaryle,
qui est éventuellement substitué par un ou plusieurs substituants $C_{1-5}$-alkyle ;
$N(C_{6-18}\text{-aryle})_2$ ;
$N(C_{3-17}\text{-hétéroaryle})_2$, et
et $N(C_{3-17}\text{-hétéroaryle})(C_{6-18}\text{-aryle})$ ;

les substituants $R^a$, $R^3$, $R^4$ ou $R^5$ formant éventuellement, indépendamment les uns des autres, un système cyclique monocyclique ou polycyclique, aliphatique, aromatique et/ou benzo-condensé ensemble avec un ou plusieurs des autres substituants $R^a$, $R^3$, $R^4$ et/ou $R^5$;
exactement un substituant choisi dans le groupe constitué par $R^T$, $R^V$, $R^W$, $R^X$, et $R^Y$ étant $R^A$,
exactement un substituant choisi dans le groupe constitué par T, V, W, X, et Y étant $R^B$,
au moins l'un du groupe constitué par $Q^1$ et $Q^2$ étant N ;
au moins l'un du groupe constitué par $Q^3$ et $Q^4$ étant N ;
exactement un substituant choisi dans le groupe constitué par $R^T$, $R^V$, $R^W$, $R^X$, et $R^Y$ représentant le site de liaison d'une simple liaison liant le premier fragment chimique et l'un des deux deuxièmes fragments chimiques, et exactement un substituant choisi dans le groupe constitué par T, V, W, X, et Y représentant le site de liaison d'une simple liaison liant le premier fragment chimique et l'un des deux deuxièmes fragments chimiques.

**2.** Molécule organique selon la revendication 1,

$R^{Tz}$ et $R^{Tz\#}$ étant indépendamment l'un de l'autre choisis dans le groupe constitué par H, méthyle et phényle, lequel est éventuellement substitué par un ou plusieurs substituants $R^6$, et
$R^{Py}$ et $R^{Py\#}$ étant hydrogène.

**3.** Molécule organique selon la revendication 1 ou 2, $R^1$, $R^2$, $R^I$, $R^{II}$, $R^{III}$, $R^{IV}$, $R^V$, $R^{VI}$, $R^{VII}$, et $R^{VIII}$ étant indépendamment les uns des autres en chaque occurrence indépendamment les uns des autres choisis dans le groupe constitué par hydrogène, deutérium, et CN.

**4.** Molécule organique selon l'une ou plusieurs des revendications 1 à 3, $Q^1$, $Q^2$, $Q^3$ et $Q^4$ étant N.

**5.** Molécule organique selon l'une ou plusieurs des revendications 1 à 4, le premier fragment chimique étant une structure de formule Iaaaa :

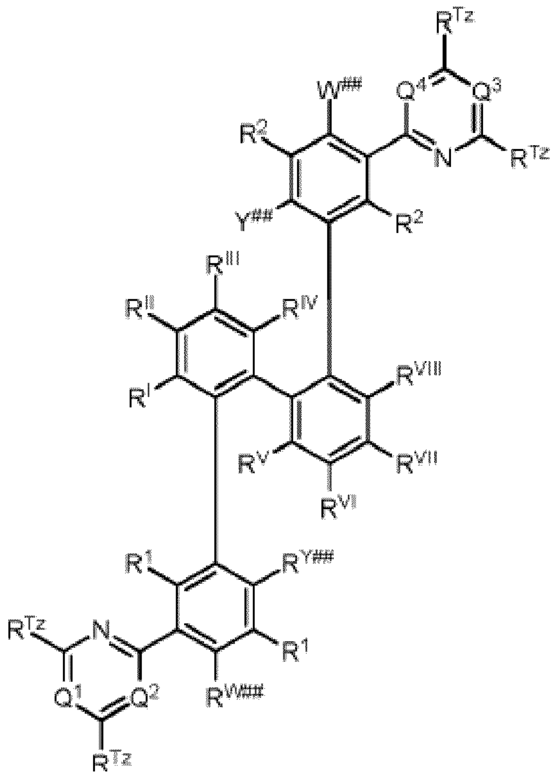

Formule Iaaaa

$R^{W\#\#}$ et $R^{Y\#\#}$ étant choisis dans le groupe constitué par $R^1$ et le site de liaison d'une simple liaison liant le premier fragment chimique et l'un des deux deuxièmes fragments chimiques ;
$W^{\#\#}$ et $Y^{\#\#}$ étant choisis dans le groupe constitué par $R^2$ et le site de liaison d'une simple liaison liant le premier fragment chimique et l'un des deux deuxièmes fragments chimiques ;
exactement un substituant choisi dans le groupe constitué par $R^{W\#\#}$ et $R^{Y\#\#}$ représentant le site de liaison d'une simple liaison liant le premier fragment chimique et l'un des deux deuxièmes fragments chimiques ;
exactement un substituant choisi dans le groupe constitué par $W^{\#\#}$ et $Y^{\#\#}$ représentant le site de liaison d'une simple liaison liant le premier fragment chimique et l'un des deux deuxièmes fragments chimiques ;
mis à part cela, les définitions de la revendication 1 s'appliquant.

**6.** Molécule organique selon l'une ou plusieurs des revendications 1 à 5, les deux deuxièmes fragments chimiques, chacun en chaque occurrence indépendamment l'un de l'autre étant une structure de formule IIa :

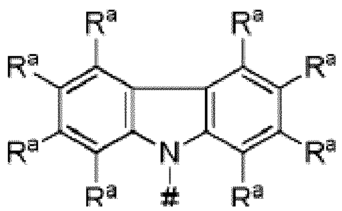

Formule IIa

$R^a$ étant chaque occurrence indépendamment l'un de l'autre choisi dans le groupe constitué par

- H,
- Me, $^i$Pr, $^t$Bu, CN, $CF_3$,
- Ph, qui est éventuellement substitué par un ou plusieurs substituants choisis indépendamment les uns des autres dans le groupe constitué par Me, $^i$Pr, $^t$Bu, CN, $CF_3$ et Ph ;
- pyridinyle, qui est éventuellement substitué par un ou plusieurs substituants choisis indépendamment les uns

des autres dans le groupe constitué par Me, $^{i}Pr$, $^{t}Bu$, CN, $CF_3$ et Ph ;
- pyrimidinyle, qui est éventuellement substitué par un ou plusieurs substituants choisis indépendamment les uns des autres dans le groupe constitué par Me, $^{i}Pr$, $^{t}Bu$, CN, $CF_3$ et Ph ;
- carbazolyle, qui est éventuellement substitué par un ou plusieurs substituants choisis indépendamment les uns des autres dans le groupe constitué par Me, $^{i}Pr$, $^{t}Bu$, CN, $CF_3$ et Ph ;
- triazinyle, qui est éventuellement substitué par un ou plusieurs substituants choisis indépendamment les uns des autres dans le groupe constitué par Me, $^{i}Pr$, $^{t}Bu$, CN, $CF_3$ et Ph ; et
- $N(Ph)_2$.

**7.** Molécule organique selon l'une ou plusieurs des revendications 1 à 6, les deux deuxièmes fragments chimiques, chacun en chaque occurrence indépendamment l'un de l'autre étant une structure de formule IIb :

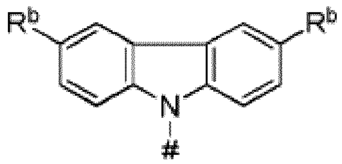

Formule IIb

$R^b$ étant chaque occurrence indépendamment l'un de l'autre choisi dans le groupe constitué par :

- H,
- Me, $^{i}Pr$, $^{t}Bu$, CN, $CF_3$,
- Ph, qui est éventuellement substitué par un ou plusieurs substituants choisis indépendamment les uns des autres dans le groupe constitué par Me, $^{i}Pr$, $^{t}Bu$, CN, $CF_3$ et Ph ;
- pyridinyle, qui est éventuellement substitué par un ou plusieurs substituants choisis indépendamment les uns des autres dans le groupe constitué par Me, $^{i}Pr$, $^{t}Bu$, CN, $CF_3$ et Ph ;
- pyrimidinyle, qui est éventuellement substitué par un ou plusieurs substituants choisis indépendamment les uns des autres dans le groupe constitué par Me, $^{i}Pr$, $^{t}Bu$, CN, $CF_3$ et Ph ;
- carbazolyle, qui est éventuellement substitué par un ou plusieurs substituants choisis indépendamment les uns des autres dans le groupe constitué par Me, $^{i}Pr$, $^{t}Bu$, CN, $CF_3$ et Ph ;
- triazinyle, qui est éventuellement substitué par un ou plusieurs substituants choisis indépendamment les uns des autres dans le groupe constitué par Me, $^{i}Pr$, $^{t}Bu$, CN, $CF_3$ et Ph ; et
- $N(Ph)_2$ ;

et mis à part cela, les définitions de la revendication 1 s'appliquant.

**8.** Molécule organique selon l'une ou plusieurs des revendications 1 à 6, les deux deuxièmes fragments chimiques, chacun en chaque occurrence indépendamment l'un de l'autre étant une structure de formule IIc :

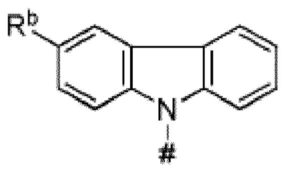

Formule IIc

$R^b$ étant chaque occurrence indépendamment l'un de l'autre choisi dans le groupe constitué par :

- H,
- Me, $^{i}Pr$, $^{t}Bu$, CN, $CF_3$,
- Ph, qui est éventuellement substitué par un ou plusieurs substituants choisis indépendamment les uns des autres dans le groupe constitué par Me, $^{i}Pr$, $^{t}Bu$, CN, $CF_3$ et Ph ;
- pyridinyle, qui est éventuellement substitué par un ou plusieurs substituants choisis indépendamment les uns des autres dans le groupe constitué par Me, $^{i}Pr$, $^{t}Bu$, CN, $CF_3$ et Ph ;
- pyrimidinyle, qui est éventuellement substitué par un ou plusieurs substituants choisis indépendamment

les uns des autres dans le groupe constitué par Me, $^{i}$Pr, $^{t}$Bu, CN, $CF_3$ et Ph ;
- carbazolyle, qui est éventuellement substitué par un ou plusieurs substituants choisis indépendamment les uns des autres dans le groupe constitué par Me, $^{i}$Pr, $^{t}$Bu, CN, $CF_3$ et Ph ;
- triazinyle, qui est éventuellement substitué par un ou plusieurs substituants choisis indépendamment les uns des autres dans le groupe constitué par Me, $^{i}$Pr, $^{t}$Bu, CN, $CF_3$ et Ph ; et
- $N(Ph)_2$ ;

et mis à part cela, les définitions de la revendication 1 s'appliquant.

**9.** Molécule organique selon la revendication 7 ou 8, $R^b$ étant chaque occurrence indépendamment l'un de l'autre choisi le groupe constitué par

- H,
- Me, $^{i}$Pr, $^{t}$Bu, CN, $CF_3$, et
- Ph.

**10.** Utilisation d'une molécule organique selon l'une ou plusieurs des revendications 1 à 9 en tant qu'émetteur luminescent dans un dispositif optoélectronique.

**11.** Utilisation selon la revendication 10, le dispositif optoélectronique étant choisi dans le groupe constitué par :

- des diodes luminescentes organiques (OLED),
- des cellules électrochimiques luminescentes,
- des capteurs OLED,
- des diodes organiques,
- des cellules solaires organiques,
- des transistors organiques,
- des transistors organiques à effet de champ,
- des lasers organiques, et
- des éléments de conversion par abaissement.

**12.** Composition, comprenant :

(a) une molécule organique selon l'une ou plusieurs des revendications 1 à 9 sous la forme d'un émetteur, et
(b) un matériau émetteur et/ou hôte, qui diffère de ladite molécule organique, et
(c) éventuellement, un colorant et/ou un solvant.

**13.** Dispositif optoélectronique, comprenant une molécule organique selon l'une ou plusieurs des revendications 1 à 9 ou une composition selon la revendication 12, en particulier sous la forme d'un dispositif choisi dans le groupe constitué par une diode luminescente organique (OLED), une cellule électrochimique luminescente, un capteur OLED, une diode organique, une cellule solaire organique, un transistor organique, un transistor organique à effet de champ, un laser organique, et un élément de conversion par abaissement.

**14.** Dispositif optoélectronique selon la revendication 13, comprenant

- un substrat,
- une anode, et
- une cathode, l'anode ou la cathode étant disposée sur le substrat, et
- une couche luminescente, qui est agencée entre l'anode et la cathode et qui comprend la molécule organique selon les revendications 1 à 9 ou la composition selon la revendication 12.

**15.** Procédé pour la production d'un dispositif optoélectronique, une molécule organique selon l'une ou plusieurs des revendications 1 à 9 ou une composition selon la revendication 12 étant utilisée, en particulier comprenant le traitement de la molécule organique par un procédé d'évaporation sous vide ou à partir d'une solution.

Figure 1

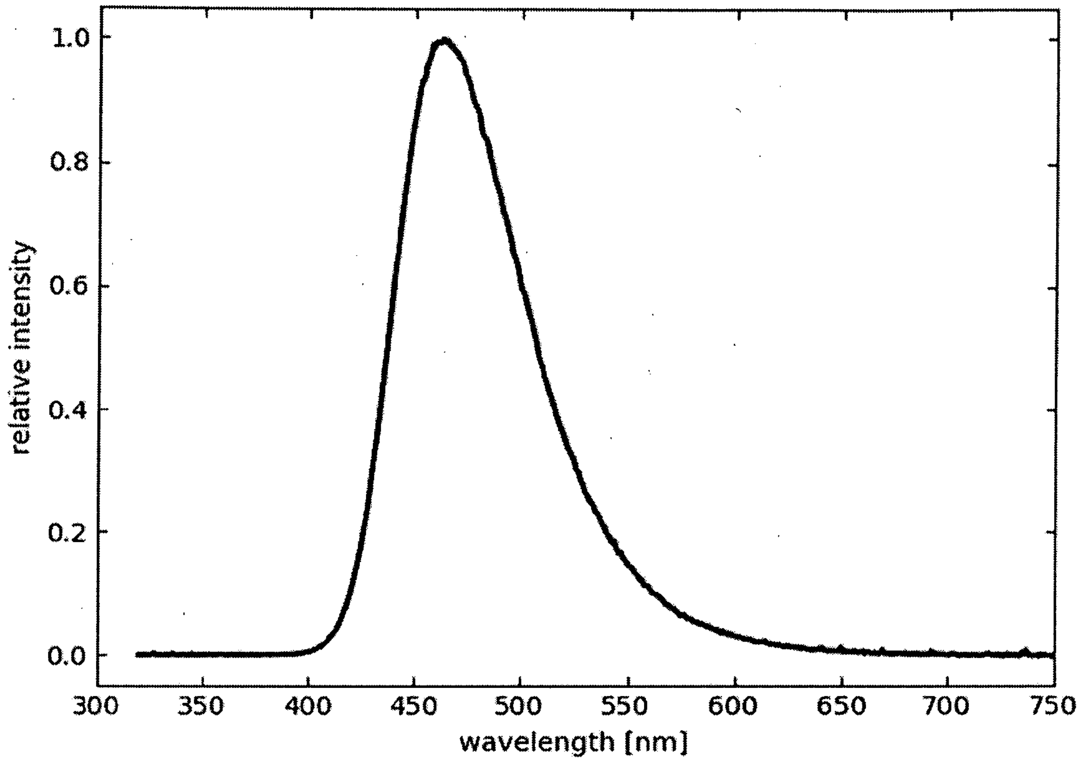


Figure 2

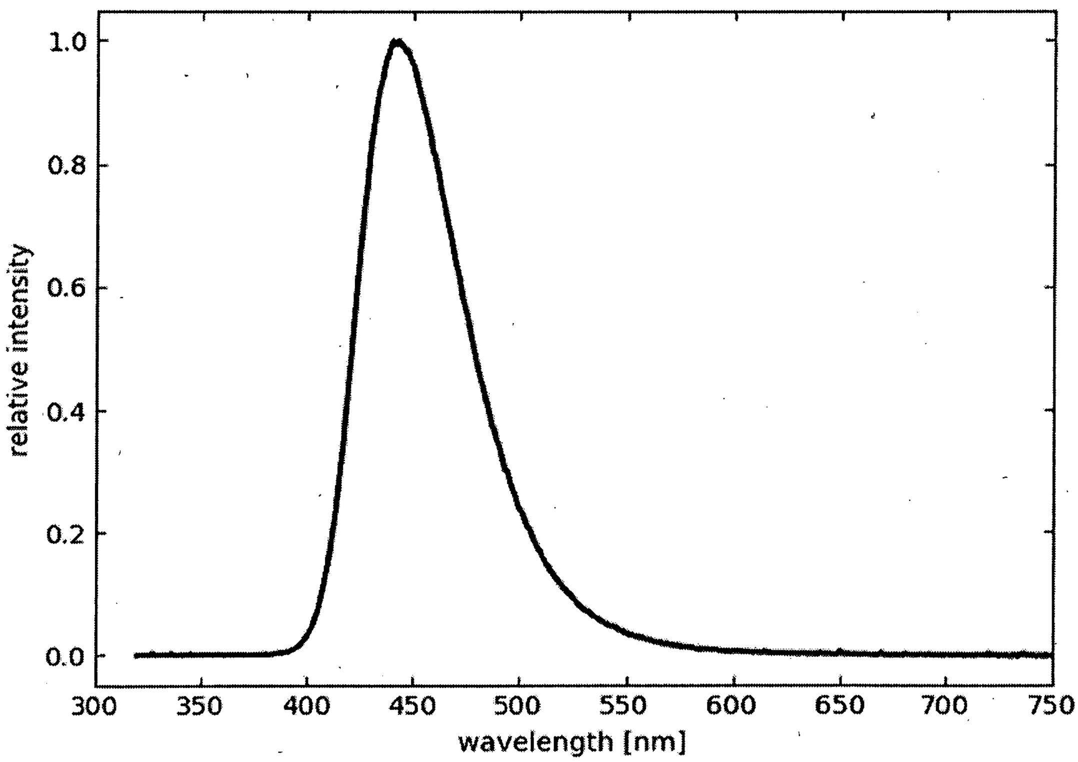

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- EP 2272828 A1
- US 20070196692 A1

### Non-patent literature cited in the description

- *CHEMICAL ABSTRACTS,* 73183-34-3